(19) Europäisches Patentamt
European Patent Office
Office européen des brevets

(11) **EP 2 221 317 A2**

(12) **EUROPEAN PATENT APPLICATION**

(43) Date of publication:
**25.08.2010 Bulletin 2010/34**

(21) Application number: **08166692.7**

(22) Date of filing: **30.06.2004**

(51) Int Cl.:
***C07K 16/46*** *(2006.01)* ***C12N 15/13*** *(2006.01)*
***C12N 15/79*** *(2006.01)* ***C12N 5/10*** *(2006.01)*
***G01N 33/68*** *(2006.01)* ***G01N 33/577*** *(2006.01)*
***G01N 33/542*** *(2006.01)*

(84) Designated Contracting States:
**AT BE BG CH CY CZ DE DK EE ES FI FR GB GR HU IE IT LI LU MC NL PL PT RO SE SI SK TR**

(30) Priority: **08.10.2003 US 509613 P**
**08.01.2004 US 535076 P**
**30.06.2003 PCT/GB03/02804**

(62) Document number(s) of the earlier application(s) in accordance with Art. 76 EPC:
**04737302.2 / 1 639 011**

(27) Previously filed application:
**30.06.2004 PCT/GB2004/002829**

(71) Applicant: **Domantis Limited**
**Brentford, Middlesex TW8 9GS (GB)**

(72) Inventor: **Basran, Amrik**
**Domantis Limited**
**Cambridge, Cambridgeshire CB4 0WG (GB)**

(74) Representative: **Reddish, Anna et al**
**GlaxoSmithKline**
**980 Great West Road**
**Brentford, Middlesex TW8 9GS (GB)**

Remarks:
This application was filed on 15-10-2008 as a divisional application to the application mentioned under INID code 62.

(54) **PEGylated single domain antibodies (dAb)**

(57) Disclosed is a PEG-linked polypeptide comprising an antibody single variable domain, wherein the variable domain comprises a framework region and has an antigen binding site, and said variable domain binds antigen as a single antibody variable domain in the polypeptide, and further wherein said PEG is present as one or more molecules linked to the polypeptide at a cysteine or lysine residue in the framework regions of said antibody single variable domain.

Figure 6

Monomer PEGylation of a VH or VL dAb

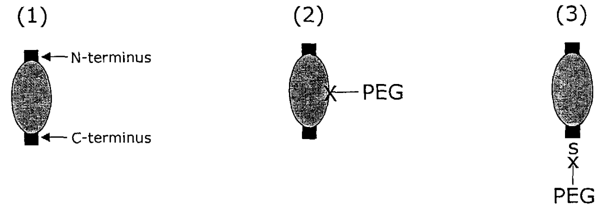


(1) Unmodified VH or VL dAb
(2) VH or VL dAb with surface PEGylation event
(3) VH or VL dAb with a C-terminal cysteine which can be PEGylated

X represents the chemical modification of an amino acid with a chemically activated PEG e.g NHS, SPA, MAL, VS, thiol etc. The amino acid residue that is modified maybe located anywhere on the surface of the dAb. PEG represents a linear/branched/forked or multi arm PEG. S represents the amino acid cysteine

EP 2 221 317 A2

## Description

BACKGROUND

Conventional antibodies are large multi-subunit protein molecules comprising at least four polypeptide chains. For example, human IgG has two heavy chains and two light chains that are disulfide bonded to form the functional antibody. The size of a conventional IgG is about 150 kD. Because of their relatively large size, complete antibodies (e.g., IgG, IgA, IgM, etc.) are limited in their therapeutic usefulness due to problems in, for example, tissue penetration. Considerable efforts have focused on identifying and producing smaller antibody fragments that retain antigen binding function and solubility.

The heavy and light polypeptide chains of antibodies comprise variable (V) regions that directly participate in antigen interactions, and constant (C) regions that provide structural support and function in non-antigen-specific interactions with immune effectors. The antigen binding domain of a conventional antibody is comprised of two separate domains: a heavy chain variable domain ($V_H$) and a light chain variable domain ($V_L$: which can be either $V_\kappa$ or $V_\lambda$). The antigen binding site itself is formed by six polypeptide loops: three from the $V_H$ domain (H1, H2 and H3) and three from the $V_L$ domain (L1, L2 and L3). *In vivo,* a diverse primary repertoire of V genes that encode the $V_H$ and $V_L$ domains is produced by the combinatorial rearrangement of gene segments. C regions include the light chain C regions (referred to as $C_L$ regions) and the heavy chain C regions (referred to as $C_H1$, $C_H2$ and $C_H3$ regions).

A number of smaller antigen binding fragments of naturally occurring antibodies have been identified following protease digestion. These include, for example, the "Fab fragment" ($V_L$-$C_L$-$C_H1$-$V_H$), "Fab' fragment" (a Fab with the heavy chain hinge region) and "F(ab')$_2$ fragment" (a dimer of Fab' fragments joined by the heavy chain hinge region). Recombinant methods have been used to generate even smaller antigen-binding fragments, referred to as "single chain Fv" (variable fragment) or "scFv," consisting of $V_L$ and $V_H$ joined by a synthetic peptide linker.

While the antigen binding unit of a naturally-occurring antibody (e.g., in humans and most other mammals) is generally known to be comprised of a pair of V regions ($V_L$/$V_H$), camelid species express a large proportion of fully functional, highly specific antibodies that are devoid of light chain sequences. The camelid heavy chain antibodies are found as homodimers of a single heavy chain, dimerized via their constant regions. The variable domains of these camelid heavy chain antibodies are referred to as $V_H$H domains and retain the ability, when isolated as fragments of the $V_H$ chain, to bind antigen with high specificity ((Hamers-Casterman et al., 1993, Nature 363: 446-448; Gahroudi et al., 1997, FEBS Lett. 414: 521-526). Antigen binding single $V_H$ domains have also been identified from, for example, a library of murine $V_H$ genes amplified from genomic DNA from the spleens of immunized mice and expressed in *E. coli* (Ward et al., 1989, Nature 341: 544-546). Ward et al. named the isolated single $V_H$ domains "dAbs," for "domain antibodies." The term "dAb" will refer herein to an antibody single variable domain ($V_H$ or $V_L$) polypeptide that specifically binds antigen. A "dAb" binds antigen independently of other V domains; however, as the term is used herein, a "dAb" can be present in a homo- or heteromultimer with other $V_H$ or $V_L$ domains where the other domains are not required for antigen binding by the dAb, i.e., where the dAb binds antigen independently of the additional $V_H$ or $V_L$ domains.

Antibody single variable domains, for example, $V_H$H, are the smallest antigen-binding antibody unit known. For use in therapy, human antibodies are preferred, primarily because they are not as likely to provoke an immune response when administered to a patient. As noted above, isolated non-camelid $V_H$ domains tend to be relatively insoluble and are often poorly expressed. Comparisons of camelid $V_{HH}$ with the $V_H$ domains of human antibodies reveals several key differences in the framework regions of the camelid $V_{HH}$ domain corresponding to the $V_H$/$V_L$ interface of the human $V_H$ domains. Mutation of these residues of human $V_H3$ to more closely resemble the $V_{HH}$ sequence (specifically Gly 44→Glu, Leu 45→Arg and Trp 47→Gly) has been performed to produce "camelized" human $V_H$ domains that retain antigen binding activity (Davies & Riechmann, 1994, FEBS Lett. 339: 285-290) yet have improved expression and solubility. (Variable domain amino acid numbering used herein is consistent with the Kabat numbering convention (Kabat et al., 1991, Sequences of Immunological Interest, 5th ed. U.S. Dept. Health & Human Services, Washington, D.C.)) WO 03/035694 (Muyldermans) reports that the Trp 103→Arg mutation improves the solubility of non-camelid $V_H$ domains. Davies & Riechmann (1995, Biotechnology N.Y. 13: 475-479) also report production of a phage-displayed repertoire of camelized human $V_H$ domains and selection of clones that bind hapten with affinities in the range of 100-400 nM, but clones selected for binding to protein antigen had weaker affinities.

WO 00/29004 (Plaskin et al.) and Reiter et al. (1999, J. Mol. Biol. 290: 685-698) describe isolated $V_H$ domains of mouse antibodies expressed in *E. coli* that are very stable and bind protein antigens with affinity in the nanomolar range. WO 90/05144 (Winter et al.) describes a mouse $V_H$ domain antibody fragment that binds the experimental antigen lysozyme with a dissociation constant of 19 nM.

WO 02/051870 (Entwistle et al.) describes human $V_H$ single domain antibody fragments that bind experimental antigens, including a $V_H$ domain that binds an scFv specific for a *Brucella* antigen with an affinity of 117 nM, and a $V_H$ domain that binds an anti-FLAG IgG.

Tanha et al. (2001, J. Biol. Chem. 276: 24774-24780) describe the selection of camelized human $V_H$ domains

that bind two monoclonal antibodies used as experimental antigens and have dissociation constants in the micromolar range.

U.S. 6,090,382 (Salfeld et al.) describe human antibodies that bind human TNF-$\alpha$ with affinities of $10^{-8}$ M or less, have an off-rate ($K_{off}$) for dissociation of human TNF-$\alpha$ of $10^{-3}$ $sec^{-1}$ or less and neutralize human TNF-$\alpha$ activity in a standard L929 cell assay.

While many antibodies and their derivatives are useful for diagnosis and therapy, the ideal pharmacokinetics of antibodies are often not achieved for a particular application. In order to provide improvement in the pharmacokinetics of antibody molecules, the present invention provides single domain variable region polypeptides that are linked to polymers which provide increased stability and half-life. The attachment of polymer molecules (e.g., polyethylene glycol; PEG) to proteins is well established and has been shown to modulate the pharmacokinetic properties of the modified proteins. For example, PEG modification of proteins has been shown to alter the *in vivo* circulating half-life, antigenicity, solubility, and resistance to proteolysis of the protein (Abuchowski et al., J. Biol. Chem. 1977, 252:3578; Nucci et al., Adv. Drug Delivery Reviews 1991, 6:133; Francis et al., Pharmaceutical Biotechnology Vol. 3 (Borchardt, R. T. ed.); and Stability of Protein Pharmaceuticals: in vivo Pathways of Degradation and Strategies for Protein Stabilization 1991 pp235-263, Plenum, NY).

Both site-specific and random PEGylation of protein molecules is known in the art (See, for example, Zalipsky and Lee, Poly(ethylene glycol) Chemistry: Biotechnical and Biomedical Applications 1992, pp 347-370, Plenum, NY; Goodson and Katre, 1990, Bio/Technology, 8:343; Hershfield et al., 1991, PNAS 88:7185). More specifically, random PEGylation of antibody molecules has been described at lysine residues and thiolated derivatives (Ling and Mattiasson, 1983, Immunol, Methods 59: 327; Wilkinson et al., 1987, Immunol. Letters, 15: 17; Kitamura et al., 1991, Cancer Res. 51:4310; Delgado et al., 1996 Br.J. Cancer, 73: 175; Pedley et al., 1994, Br. J. Cancer, 70:1126).

SUMMARY OF THE INVENTION

The present invention is based on the discovery that attachment of polymer moieties such as PEG to antibody single variable domain polypeptides (domain antibodies; dAb) provides a longer *in vivo* half-life and increased resistance to proteolysis without a loss in dAb activity or target binding affinity. The invention also provides dAb molecules in various formats including dimers, trimers, and tetramers, which are linked to one or more polymer molecules such as PEG.

In one embodiment the present invention encompasses a PEG-linked polypeptide comprising one or two antibody single variable domain polypeptides, wherein the polypeptide has a hydrodynamic size of at least 24 kDa and a half life of at least 1.3 hours, and wherein each variable domain has an antigen binding site, and each variable domain binds antigen as a single antibody variable domain in the polypeptide.

In a further embodiment the invention encompasses a PEG-linked polypeptide comprising one or two antibody single variable domains, wherein the polypeptide has a hydrodynamic size of at least 200 kDa and a total PEG size of from 20 to 60 kDa, and wherein each variable domain has an antigen binding site, and each variable domain binds antigen as a single antibody variable domain in the polypeptide.

The invention also encompasses a PEG-linked multimer of antibody single variable domains having a hydrodynamic size of at least 24 kDa, and wherein the total PEG size is from 20 to 60 kDa, and wherein each variable domain has an antigen binding site and each variable domain binds antigen as a single antibody variable domain in the polypeptide.

In one embodiment, the PEG-linked polypeptide retains at least 90% activity relative to the same polypeptide not linked to PEG, wherein activity is measured by affinity of the PEG-linked or non-PEG-linked polypeptide to a target ligand.

In one embodiment, each variable domain comprises a universal framework.

In a further embodiment, the universal framework comprises a $V_H$ framework selected from the group consisting of DP47, DP45 and DP38; and/or the $V_L$ framework is DPK9.

The present invention also encompasses a PEG-linked polypeptide comprising an antigen binding site specific for TNF$\alpha$, the polypeptide having one or two antibody variable domains, each variable domain having a TNF$\alpha$ binding site, wherein the polypeptide has a hydrodynamic size of at least 200 kDa and a total PEG size of from 20 to 60 kDa.

In one embodiment, the polypeptide has specificity for TNF$\alpha$.

In one embodiment, the polypeptide dissociates from human TNF$\alpha$ with a dissociation constant ($K_d$) of 50nM to 20pM, and a $K_{off}$ rate constant of 5x $10^{-7}$ to 1x$10^{-7}$ $s^{-1}$, as determined by surface plasmon resonance.

In one embodiment, the polypeptide neutralizes human TNF$\alpha$ in a standard cell assay with an ND50 of 500nM to 50pM.

In one embodiment, the PEG-linked polypeptide comprises a universal framework, wherein the universal framework comprises a $V_H$ framework selected from the group consisting of DP47, DP45 and DP38; and/or the $V_L$ framework is DPK9.

The invention also encompasses a polymer-linked antibody single variable domain having a half life of at least

1.3 hours, and wherein the polymer is directly or indirectly linked to the antibody single variable domain at a cysteine or lysine residue of the single antibody variable domain.

In one embodiment, the polymer linked antibody single variable domain has a hydrodynamic size of at least 24 kDa.

In one embodiment, the cysteine or lysine residue is at a predetermined location in the antibody single variable domain.

In one embodiment, the cysteine or lysine residue is present at the C-terminus of the antibody single variable domain.

In one embodiment the polymer is linked to the antibody single variable domain at a cysteine or lysine residue not present at either the C-terminus or N-terminus of said antibody single variable domain.

In one embodiment, the polymer is linked to the antibody single variable domain at a cysteine or lysine residue spaced at least two residues away from the C- and/or N-terminus.

In one embodiment, the polymer is linked to a heavy chain variable domain comprising a cysteine or lysine residue substituted at a position selected from the group consisting of Gln13, Pro41, or Leu115.

In one embodiment, the antibody single variable domain comprises a C-terminal hinge region and wherein the polymer is attached to the hinge region.

In one embodiment, the polymer is selected from the group consisting of straight or branched chain poly (ethylene glycol) (PEG), poly(propylene glycol), poly(vinyl alcohol), methoxy(polyethylene glycol), lactose, amylose, dextran, and glycogen

In one embodiment, the polymer is PEG

In one embodiment, one or more predetermined residues of said antibody single variable domain are mutated to a cysteine or lysine residue, and wherein said PEG is linked to said mutated residue

In one embodiment, the antibody single variable domain is a heavy chain variable domain.

In one embodiment, the antibody single variable domain is a light chain variable domain ($V_L$).

In one embodiment, the half life is between 1.3 and 170 hours.

In one embodiment, the polymer-linked antibody single variable domain has a t½ alpha of between 0.25 and 6 hours.

In one embodiment, the polymer-linked antibody single variable domain has a t½ beta of between 2 and 40 hours.

The invention also encompasses a PEG-linked multimer of antibody single variable domains having a half life of at least 1.3 hours, and wherein said PEG is linked to said multimer at a cysteine or lysine residue of said multimer, and wherein each variable domain has an antigen binding site, and each variable domain binds antigen as a single antibody variable domain in the polypeptide.

In one embodiment, the multimer is a dimer of antibody single variable domains.

In one embodiment, the multimer is a trimer of antibody single variable domains.

In one embodiment, the multimer is a tetramer of antibody single variable domains.

In one embodiment, the cysteine or lysine residue is present at the C-terminus or N-terminus of a antibody single variable domain comprised by said multimer.

In one embodiment, one or more predetermined residues of at least one of said antibody single variable domains are mutated to a cysteine or lysine residue, and wherein said PEG is linked to said mutated residue.

In one embodiment, the mutated residue is not at the C-terminus or N-terminus of said antibody single variable domains.

In one embodiment, the antibody single variable domain polypeptide is a heavy chain variable domain, and said mutated residue is selected from the group consisting of Gln13, Pro41 or Leu115.

In one embodiment, the PEG is linked to said antibody single variable domains at a cysteine or lysine residue spaced at least two residues away from the C- and/or N-terminus.

In one embodiment, the half life is between 1.3 and 170 hours.

In one embodiment, the PEG-linked antibody single variable domain has a t½ alpha of between 0.25 and 5.8 hours.

In one embodiment, the PEG-linked antibody single variable domain has a t½ beta of between 2 and 40 hours.

The invention also encompasses a PEG-linked multimer antibody single variable domains comprising three or more antibody single variable domains wherein the variable domain has an antigen binding site, and each variable domain binds antigen as a single antibody variable domain.

In one embodiment, the multimer has a hydrodynamic size of at least 24 kDa.

In one embodiment, the multimer has a hydrodynamic size of at least 200 kDa.

In one embodiment, the multimer has 3, 4, 5, 6, 7, or 8 antibody single variable domains..

In one embodiment, the PEG-linked multimer of claim 40 has a half life of at least 1.3 hours.

In one embodiment, the half life is between 1.3 and 170 hours.

In one embodiment, the PEG-linked antibody single variable domain has a t½ alpha of between 0.55 and 6 hours.

In one embodiment, the PEG-linked antibody single variable domain has a t½ beta of between 2 and 40 hours.
In one embodiment, the PEG is linked to said antibody single variable domain trimer or tetramer at a predetermined cysteine or lysine residue provided by a variable domain of the multimer.
In one embodiment, the cysteine or lysine residue is present at the C-terminus or N-terminus of an antibody single variable domain of said multimer.
In one embodiment, one or more predetermined residues of said antibody single variable domain are mutated to a cysteine or lysine residue, and wherein said PEG is linked to said mutated residue.
In one embodiment, the mutated residue is not at the C-terminus or N-terminus of said antibody single variable domains.
In one embodiment, the antibody single variable domain is a heavy chain variable domain and said mutated residue is selected from the group consisting of Gln13, Pro41 or Leu115.
In one embodiment, the PEG is linked to said antibody single variable domains at a cysteine or lysine residue which is spaced at least two residues away from the C- or N-terminus.
The invention still further encompasses a polypeptide comprising an antigen binding site, the polypeptide comprising one or two antibody variable domains, wherein the polypeptide has a hydrodynamic size of at least 24 kDa and a half life of at least 1.3 hours, wherein each variable domain has an antigen binding site, and each variable domain binds antigen as a antibody single variable domain in the polypeptide.
The invention also encompasses a polypeptide comprising a binding site specific for TNF-$\alpha$, said polypeptide comprising one or two antibody variable domains, wherein the polypeptide has a hydrodynamic size of at least 24 kDa and a half life of at least 1.3 hours.
In one embodiment, each variable domain has an antigen binding site and each variable domain binds antigen as an antibody single variable domain in the polypeptide.
In one embodiment, the polypeptide is linked to a PEG polymer having a size of between 20 and 60 kDa.
In one embodiment, the polypeptide has a hydrodynamic size of at least 200 kDa.
In one embodiment, the half life is between 1.3 and 170 hours.
In one embodiment, the polypeptide has a t½ alpha of between 0.25 and 6 hours.
In one embodiment, the polypeptide has a t½ beta of between 2 and 40 hours.
In one embodiment, the polypeptide comprises a variable domain that is linked to a PEG moiety at a cysteine or lysine residue of said variable domain.
In one embodiment, the cysteine or lysine residue is present at the C-terminus or N-terminus of said antibody single variable domain.
In one embodiment, one or more predetermined residues of said variable domain are mutated to a cysteine or lysine residue, and wherein said PEG is linked to said mutated residue.
In one embodiment, the mutated residue is not at the C-terminus or N-terminus of said antibody single variable domains.
In one embodiment, the variable domain is a heavy chain variable domain and said mutated residue is selected from the group consisting of Gln13, Pro41 or Leu115.
The invention encompasses a homomultimer of antibody single variable domains, wherein said homomultimer has a hydrodynamic size of at least 24 kDa and a half life of at least 1.3 hours.
In one embodiment, each variable domain has an antigen binding site, and each variable domain binds antigen as a single antibody variable domain in the homomultimer.
In one embodiment, the homomultimer is linked to at least one PEG polymer.
In one embodiment, the half life is between 1.3 and 170 hours.
In one embodiment, the homomultimer has a t½ alpha of between 0.25 and 6 hours.
In one embodiment, the homomultimer has a t½ beta of between 1 and 40 hours.
In one embodiment, each antibody single variable domain of said homomultimer comprises either heavy chain variable domain or $V_L$.
In one embodiment, each antibody single variable domain of said homomultimer is engineered to contain an additional cysteine residue at the C-terminus of said antibody single variable domain.
In one embodiment, the antibody single variable domains of said homomultimer are linked to each other by a peptide linker.
In one embodiment, the homomultimer comprises only a first and second antibody single variable domain, wherein said first antibody single variable domain of said homodimer comprises an antibody single variable domain and a heavy chain (CH1) constant region, and wherein said second antibody single variable domain of said homodimer comprises an antibody single variable domain and a light chain (CL) constant region.
In one embodiment, the homomultimer has specificity for TNF$\alpha$.
In one embodiment, the homomultimer dissociates from human TNF$\alpha$ with a dissociation constant ($K_d$) of 50nM to 20pM, and a $K_{off}$ rate constant of $5x10^{-1}$ to $1x10^{-7}s^{-1}$, as determined by surface plasmon resonance.

In one embodiment, the homomultimer neutralizes human TNFα in a standard cell assay with an ND50 of 500nM to 50pM.

In one embodiment, the antibody single variable domain of said homomultimer binds TNFα.

In one embodiment, each antibody single variable domain of the homomultimer dissociates from human TNFα with a dissociation constant ($K_d$) of 50nM to 20pM, and a $K_{off}$ rate constant of $5x10^{-1}$ to $1x10^{-7}$ $s^{-1}$, as determined by surface plasmon resonance. In one embodiment, the homomultimer dissociates from human TNFα with a dissociation constant ($K_d$) of 50nM to 20pM, and a $K_{off}$ rate constant of $5x10^{-1}$ to $1x10^{-7}$ $s^{-1}$.

In one embodiment, the antibody single variable domain of said homomultimer neutralizes human TNFα in a standard cell assay with an ND50 of 500nM to 50pM.

The invention further encompasses a heteromultimer of antibody single variable domains, and wherein said heteromultimer has a hydrodynamic size of at least 24 kDa and a half life of at least 1.3 hours, and wherein each variable domain has an antigen binding site, and each antibody single variable domain binds antigen as a single antibody variable domain in the heteromultimer.

In one embodiment, the heteromultimer is linked to at least one PEG polymer.

In one embodiment, the half life of the homomultimer is between 1.3 and 170 hours.

In one embodiment, the heteromultimer has a t½ alpha of between 0.25 and 6 hours.

In one embodiment, the heteromultimer has a t½ beta of between 2 and 40 hours.

In one embodiment, each antibody single variable domain of said heteromultimer comprises either heavy chain variable domain or $V_L$.

In one embodiment, each antibody single variable domain of said heteromultimer is engineered to contain an additional cysteine residue at the C-terminus or N-terminus of said antibody single variable domain.

In one embodiment, the antibody single variable domains of said heteromultimer are linked to each other by a peptide linker.

In one embodiment, the heteromultimer comprises only a first and second antibody single variable domain, wherein said first antibody single variable domain of said heteromultimer comprises an antibody single variable domain and a heavy chain (CH1) constant region, and wherein said second antibody single variable domain of said heteromultimer comprises an antibody single variable domain and a light chain (CL) constant region.

In one embodiment, the heteromultimer has specificity for TNFα.

In one embodiment, the heteromultimer dissociates from human TNFα with a dissociation constant ($K_d$) of 50nM to 20pM, and a $K_{off}$ rate constant of $5x10^{-1}$ to $1x10^{-7}$ $s^{-1}$, as determined by surface plasmon resonance.

In one embodiment, the heteromultimer neutralizes human TNFα in a standard cell assay with an ND50 of 500nM to 50pM.

In one embodiment, each antibody single variable domain of said heteromultimer has specificity for TNFα.

In one embodiment, each antibody single variable domain of said heteromultimer dissociates from human TNFα with a dissociation constant ($K_d$) of 50nM to 20pM, and a $K_{off}$ rate constant of $5x10^{-1}$ to $1x10^{-7}s^{-1}$, as determined by surface plasmon resonance.

In one embodiment, each antibody single variable domain of said heteromultimer neutralizes human TNFα in a standard cell assay with an ND50 of 500nM to 50pM.

The invention also encompasses a PEG-linked antibody single variable domain specific for a target ligand which retains activity relative to a non-PEG-linked antibody single variable domain having the same antibody single variable domain as said PEG-linked antibody single variable domain, wherein activity is measured by affinity of said PEG-linked or non-PEG-linked antibody single variable domain to the target ligand.

In one embodiment, the PEG-linked antibody single variable domain retains at least 90% of the activity of the same antibody single variable domain not linked to PEG.

In one embodiment, the activity is measured by surface plasmon resonance as the binding of said PEG-linked antibody single variable domain to TNFα.

In one embodiment, the PEG-linked antibody single variable domain dissociates from human TNFα with a dissociation constant ($K_d$) of 50nM to 20pM, and a $K_{off}$ rate constant of $5x10^{-1}$ to $1x10^{-7}$ $s^{-1}$, as determined by surface plasmon resonance.

In one embodiment, the activity is measured as the ability of said PEG-linked antibody single variable domain to neutralize human TNFα or TNF receptor 1 in a standard cell assay.

In one embodiment, the PEG-linked antibody single variable domain neutralizes human TNFα or TNF receptor 1 in a standard cell assay with an ND50 of 500nM to 50pM

In one embodiment, the PEG-linked antibody single variable domain has an IC50 or ND50 which is no more than 10% greater than the IC50 or ND50 respectively of a non-PEG-linked antibody variable domain having the same antibody single variable domain as said PEG-linked antibody single variable domain.

The invention also includes a PEG-linked antibody single variable domain specific for a target antigen which specifically binds to the target antigen with a $K_d$ of 80 nM to 30 pM.

The invention also includes a PEG-linked antibody single variable domain which specifically binds to a target antigen with a $K_d$ of 3 nM to 30 pM.

The invention also includes a PEG-linked antibody single variable domain which specifically binds to a target antigen with a $K_d$ of 100 pM to 30 pM.

In one embodiment, the PEG-linked antibody single variable domain of claim 105, wherein said PEG-linked antibody single variable domain binds to TNFα with a dissociation constant ($K_d$) of 50nM to 20pM, and a $K_{off}$ rate constant of $5x10^{-1}$ to $1x10^{-7}$ $s^{-1}$, as determined by surface plasmon resonance.

In one embodiment, the binding is measured as the ability of said PEG-linked antibody single variable domain to neutralize human TNFα or TNF receptor 1 in a standard cell assay.

In one embodiment, the PEG-linked antibody single variable domain neutralizes human TNFα or TNF receptor 1 in a standard cell assay with an ND50 of 500nM to 50pM

The present invention still further includes a PEG-linked antibody single variable domain homomultimer which retains activity relative to a non-PEG-linked antibody single variable domain homomultimer having the same antibody single variable domain as said PEG-linked antibody single variable domain, wherein activity is measured by affinity of said PEG-linked or non-PEG-linked antibody single variable domain homomultimer to a target ligand.

In one embodiment, the PEG-linked antibody single variable domain retains 90% of the activity of the same antibody single variable domain homomultimer not linked to PEG.

In one embodiment, the activity is measured as the binding of said PEG-linked antibody single variable domain homomultimer to TNFα.

In one embodiment, the activity is measured as the ability of said PEG-linked antibody single variable domain homomultimer to inhibit cell cytotoxicity in response to TNFα.

In one embodiment, the PEG-linked antibody single variable domain has an IC50 which is no more than 10% greater than the IC50 of a non-PEG-linked antibody variable domain homomultimer.

In one embodiment, each member of said homomultimer comprises either heavy chain variable domain or $V_L$.

In one embodiment, the homomultimer comprises an antibody single variable domain that is engineered to contain an additional cysteine residue at the C-terminus or N-terminus of said antibody single variable domain.

In one embodiment, the members of said homomultimer are linked to each other by a peptide linker.

In one embodiment, where said multimer comprises only a first and second member, said first member of said homodimer comprises an antibody single variable domain and a heavy chain (CH1) constant region, and said second member of said homodimer comprises an antibody single variable domain and a light chain (CL) constant region.

The invention still further encompasses a PEG-linked antibody single variable domain heteromultimer which retains activity relative to the same antibody single variable domain heteromultimer not linked to PEG, wherein activity is measured by affinity of said PEG-linked antibody single variable domain heteromultimer or antibody single variable domain heteromultimer not linked to PEG to a target ligand.

In one embodiment, the PEG-linked antibody single variable domain retains 90% of the activity of the same antibody single variable domain heteromultimer not linked to PEG.

In one embodiment, the activity is measured as the binding of said PEG-linked antibody single variable domain heteromultimer to TNFα.

In one embodiment, the activity is measured as the ability of said PEG-linked antibody single variable domain heteromultimer to inhibit cell cytotoxicity in response to TNFα.

In one embodiment, the PEG-linked antibody single variable domain has an IC50 which is no more than 10% greater than the IC50 of a non-PEG-linked antibody variable domain heteromultimer having the same antibody single variable domain as the PEG-linked antibody single variable domain.

In one embodiment, each member of said heteromultimer comprises either heavy chain variable domain or $V_L$.

In one embodiment, each of said antibody single variable domain is engineered to contain an additional cysteine residue at the C-terminus or N-terminus of said antibody single variable domain.

In one embodiment, the members of said heteromultimer are linked to each other by a peptide linker.

In one embodiment, the multimer comprises only a first and second member, said first member of said heteromultimer comprises an antibody single variable domain and a heavy chain (CH1) constant region, and said second member of said homodimer comprises an antibody single variable domain and a light chain (CL) constant region.

In one embodiment, the above homo- or heteromultimer is selected from the group consisting of a dimer, trimer, and tetramer.

In one embodiment, the PEG moiety of the above homo- or heteromultimer is a branched PEG.

The invention also encompasses a PEG-linked homomultimer of antibody single variable domains which specifically binds to a target antigen with a $K_d$ of 80 nM to 30 pM.

In one embodiment, the PEG-linked homomultimer binds to TNFα with a dissociation constant ($K_d$) of 50nM to 20pM, and a $K_{off}$ rate constant of $5x10^{-1}$ to $1x10^{-7}$ $s^{-1}$, as determined by surface plasmon resonance.

In one embodiment, the binding is measured as the ability of said PEG-linked homomultimer to neutralize

human TNFα or TNF receptor 1 in a standard cell assay.

In one embodiment, the PEG-linked homomultimer neutralizes human TNFα or TNF receptor 1 in a standard cell assay with an ND50 of 500nM to 50pM

The invention encompasses a PEG-linked homomultimer of antibody single variable domains which specifically binds to a target antigen with a $K_d$ of 3 nM to 30 pM.

The invention also encompasses a PEG-linked homomultimer of antibody single variable domains which specifically binds to a target antigen with a $K_d$ of 100 pM to 30 pM.

The invention further encompasses a PEG-linked heteromultimer of antibody single variable domains which specifically binds to a target antigen with a $K_d$ of 80 nM to 30 pM.

The invention still further encompasses a PEG-linked heteromultimer of antibody single variable domains which specifically binds to a target antigen with a $K_d$ of 3 nM to 30 pM.

The invention also encompasses a PEG-linked heteromultimer of antibody single variable domains which specifically binds to a target antigen with a $K_d$ of 100 pM to 30 pM.

The present invention encompasses an antibody single variable domain comprising at least one solvent-accessible lysine residue at a predetermined location in said antibody single variable domain which is linked to a PEG molecule.

In one embodiment, the PEG is linked to said solvent-accessible lysine in the form of a PEG linked N-hydroxylsuccinimide active ester.

In one embodiment, the N-hydroxylsuccinimide active ester is selected from the group consisting of PEG-O-$CH_2CH_2CH_2$-$CO_2$-NHS; PEG-O-$CH_2$-NHS; PEG-O-$CH_2CH_2$-$CO_2$-NHS; PEG-S-$CH_2CH_2$-CO-NHS; PEG-$O_2$CNH-CH(R)-$CO_2$-NHS; PEG-NHCO-$CH_2CH_2$-CO-NHS; and PEG-O-$CH_2$-$CO_2$-NHS; where R is $(CH_2)_4)NHCO_2$(mPEG).

In one embodiment, the PEG is a branched PEG

The invention encompasses an antibody single variable domain multimer, each member of said multimer comprising at least one solvent accessible lysine residue which is linked to a PEG molecule.

In one embodiment, the solvent accessible lysine residue results from a mutation at one or more residues selected from the group consisting of Gln13, Pro41 or Leu 115.

In one embodiment, the multimer is a homomultimer.

In one embodiment, the multimer is a heteromultimer.

In one embodiment, the multimer is a hetero- or homotrimer.

In one embodiment, the multimer is a hetero- or homotetramer.

The invention also encompasses an antibody single variable domain homo- or hetero-trimer or tetramer comprising at least one solvent-accessible cysteine residue which is linked to a PEG molecule.

In one embodiment, the PEG is linked to said solvent-accessible cysteine by a sulfhydryl-selective reagent selected from the group consisting of maleimide, vinyl sulfone, and thiol.

In one embodiment, the antibody single variable domain is a heavy chain variable domain and said solvent accessible cysteine residue results from a mutation at one or more residues selected from the group consisting of Gln13, Pro41 or Leu115.

The invention also encompasses a PEG-linked antibody variable region polypeptide having a half life which is at least seven times greater than the half life of the same antibody variable region polypeptide not linked to PEG.

In one embodiment, the PEG-linked antibody variable region has a hydrodynamic size of at least 24 kDa.

In one embodiment, the PEG-linked antibody variable region has a hydrodynamic size of between 24 kDa and 500 kDa.

The present invention encompasses a pharmaceutical formulation comprising a PEG-linked antibody single variable domain having a half life of at least 1.3 hours; and a carrier.

The present invention also encompasses a pharmaceutical formulation comprising a PEG-linked antibody single variable domain dimer having a half life of at least 1.3 hours and having a hydrodynamic size of at least 24 kDa; and a carrier.

The present invention still further encompasses a pharmaceutical formulation comprising a PEG-linked antibody single variable domain heterotrimer or homotrimer or heterotetramer or homotetramer, wherein each variable domain has an antigen binding site, and each variable domain binds antigen as a single variable domain..

The present invention also encompasses a pharmaceutical formulation comprising a PEG-linked antibody single variable domain, wherein said PEG-linked antibody single variable domain is degraded by no more than 10% after administration of said pharmaceutical formulation to the stomach of an animal.

The present invention includes a pharmaceutical formulation comprising a PEG-linked antibody single variable domain, wherein said PEG-linked antibody single variable domain is degraded by no more than 10% in vitro by exposure to a protease selected from the group consisting of pepsin, trypsin, elastase, chymotrypsin, and carboxypeptidase, wherein if said protease is pepsin, then said PEG-linked antibody single variable domain is degraded by no more than 10% in the presence of pepsin at pH 2.0 for 30 minutes, and wherein if said protease is trypsin, elastase, chymotrypsin,

or carboxypeptidase, then said PEG-linked antibody single variable domain is degraded by no more than 10% in the presence of trypsin, elastase, chymotrypsin, and carboxypeptidase at pH 8.0 for 30 minutes.

In one embodiment, the pharmaceutical formulation is suitable for oral administration or is suitable for parenteral administration via a route selected from the group consisting of intravenous, intramuscular or intraperitoneal injection, implantation, rectal and transdermal administration.

In one embodiment, the pharmaceutical formulation is an extended release parenteral or oral dosage formulation.

The present invention encompasses a method for reducing the degradation of an antibody single variable monomer or multimer domain by a protease selected from the group consisting of pepsin, trypsin, elastase, chymotrypsin, and carboxypeptidase comprising linking said single variable domain to at least one PEG polymer.

In one embodiment, the degradation is reduced in the stomach of an animal.

In one embodiment, the degradation is reduced *in vitro* by at least 10% when said antibody single variable domain is exposed to pepsin at pH 2.0 for 30 minutes, and wherein said degradation is reduced *in vitro* by at least 10% when said antibody variable domain is exposed to trypsin, elastase, chymotrypsin, and carboxypeptidase at pH 8.0 for 30 minutes.

In one embodiment, the polymer is selected from the group consisting of straight or branched chain poly (ethylene glycol) (PEG), poly(propylene glycol), poly(vinyl alcohol), methoxy(polyethylene glycol), lactose, amylose, dextran, and glycogen

In one embodiment, the polymer is PEG.

In one embodiment, one or more predetermined residues of the antibody single variable domain are mutated to a cysteine or lysine residue, and wherein the PEG is linked to the mutated residue

In one embodiment, the antibody single variable domain is a heavy chain variable domain ($V_H$).

In one embodiment, the antibody single variable domain is a light chain variable domain ($V_L$).

In one embodiment, the half life is between 0.25 and 170 hours.

In one embodiment, the polymer-linked antibody single variable domain has a t½ alpha of between 0.25 and 6 hours.

In one embodiment, the polymer-linked antibody single variable domain has a t½ beta of between 2 and 40 hours.

The present invention also encompasses a PEG-linked multimer of antibody single variable domains having a half life of at least 0.25 hours, and wherein the PEG is linked to the multimer at a cysteine or lysine residue of the multimer, and wherein each variable domain has an antigen binding site and each variable domain binds antigen as a single antibody variable domain in the multimer.

In one embodiment, the multimer is a dimer of antibody single variable domains.

In one embodiment, the multimer is a trimer of antibody single variable domains.

In a further embodiment, the multimer is a tetramer of antibody single variable domains.

In one embodiment, the cysteine or lysine residue is present at the C-terminus of a antibody single variable domain comprised by the multimer.

In one embodiment, one or more predetermined residues of at least one of the antibody single variable domains are mutated to a cysteine or lysine residue, and wherein the PEG is linked to the mutated residue.

In one embodiment, the half life is between 0.25 and 170 hours.

In one embodiment, the PEG-linked antibody single variable domain has a t½ alpha of between 0.25 and 5.8 hours.

In one embodiment, the PEG-linked antibody single variable domain has a t½ beta of between 2 and 40 hours.

The present invention also encompasses a PEG-linked multimer antibody single variable domains comprising three or more antibody single variable domains wherein the variable domain has an antigen binding site, and each variable domain binds antigen as a single antibody variable domain.

In one embodiment the PEG linked multimer has a hydrodynamic size of at least 24 kDa.

In a further embodiment, the PEG-linked multimer has a hydrodynamic size of at least 200 kDa.

In one embodiment, the multimer has 3, 4, 5, 6, 7, or 8 antibody single variable domains.

In one embodiment, the PEG-linked multimer has a half life of at least 0.25 hours.

In one embodiment, the half life is between 0.25 and 170 hours.

In one embodiment, the PEG-linked antibody single variable domain has a t½ alpha of between 0.55 and 6 hours.

In one embodiment, the PEG-linked antibody single variable domain has a t½ beta of between 2 and 40 hours.

In one embodiment, the PEG is linked to the antibody single variable domain trimer or tetramer at a predetermined cysteine or lysine residue provided by a variable domain of the multimer.

In one embodiment, the cysteine or lysine residue is present at the C-terminus of an antibody single variable domain of the multimer.

In one embodiment, one or more predetermined residues of the antibody single variable domain are mutated to a cysteine or lysine residue, and wherein the PEG is linked to the mutated residue.

The invention further encompasses a polypeptide comprising an antigen binding site, the polypeptide comprising one or two antibody variable domains, wherein the polypeptide has a hydrodynamic size of at least 24 kDa and a half life of at least 0.25 hours, wherein each variable domain has an antigen binding site, and each variable domain binds antigen as a antibody single variable domain in the polypeptide.
The invention still further encompasses a polypeptide comprising a binding site specific for TNF-α, the polypeptide comprising one or two antibody variable domains, wherein the polypeptide has a hydrodynamic size of at least 24 kDa and a half life of at least 0.25 hours.
In one embodiment each variable domain has an antigen binding site and each variable domain binds antigen as an antibody variable domain in the polypeptide.
In one embodiment, the polypeptide is linked to a PEG polymer having a size of between 20 and 60 kDa.
In one embodiment, the polypeptide has a hydrodynamic size of at least 200 kDa.
In one embodiment, the half life is between 0.25 and 170 hours.
In one embodiment, the polypeptide has a t½ alpha of between 0.25 and 6 hours.
In one embodiment, the polypeptide domain has a t½ beta of between 2 and 40 hours.
In one embodiment, the polypeptide comprises a variable domain that is linked to a PEG moiety at a cysteine or lysine residue of the variable domain.
In one embodiment, the cysteine or lysine residue is present at the C-terminus of the antibody single variable domain.
In one embodiment, one or more predetermined residues of the variable domain are mutated to a cysteine or lysine residue, and wherein the PEG is linked to the mutated residue.
The invention also encompasses a homomultimer of antibody single variable domains, wherein the homomultimer has a hydrodynamic size of at least 24 kDa and a half life of at least 0.25 hours.
In one embodiment, each variable domain has an antigen binding site, and each variable domain binds antigen as a single antibody variable domain in the homomultimer.
In one embodiment, the homomultimer is linked to at least one PEG polymer.
In one embodiment, the half life is between 0.25 and 170 hours.
In one embodiment, the homomultimer has a t½ alpha of between 0.25 and 6 hours.
In one embodiment, the homomultimer has a t½ beta of between 2 and 40 hours.
In one embodiment, each antibody single variable domain of the homomultimer comprises either $V_H$ or $V_L$.
In one embodiment, each antibody single variable domain of the homomultimer is engineered to contain an additional cysteine residue at the C-terminus of the antibody single variable domain.
In one embodiment, the antibody single variable domains of the homomultimer are linked to each other by a peptide linker.
In one embodiment, the homomultimer comprises only a first and second antibody single variable domain, wherein the first antibody single variable domain of the homodimer comprises an antibody single variable domain and a heavy chain (CH1) constant region, and wherein the second antibody single variable domain of the homodimer comprises an antibody single variable domain and a light chain (CL) constant region.
In one embodiment, the homomultimer has specificity for TNFα.
In one embodiment, the homomultimer dissociates from human TNFα with a dissociation constant ($K_d$) of 50nM to 20pM, and a $K_{off}$ rate constant of $5x10^{-1}$ to $1x10^{-7}s^{-1}$, as determined by surface plasmon resonance.
In one embodiment, the homomultimer neutralizes human TNFα in a standard cell assay with an ND50 of 500nM to 50pM.
In one embodiment, each antibody single variable domain of the homomultimer binds TNFα.
In one embodiment, each antibody single variable domain of the homomultimer dissociates from human TNFα with a dissociation constant ($K_d$) of 50nM to 20pM, and a $K_{off}$ rate constant of $5x10^{-1}$ to $1X10^{-7}$ $s^{-1}$, as determined by surface plasmon resonance. In one embodiment, the homomultimer dissociates from human TNFα with a dissociation constant ($K_d$) of 50nM to 20pM, and a $K_{off}$ rate constant of $5x10^{-1}$ to $1x10^{-7}$ $s^{-1}$.
In one embodiment, each antibody single variable domain of the homomultimer neutralizes human TNFα in a standard cell assay with an ND50 of 500nM to 50pM.
The invention further encompasses a heteromultimer of antibody single variable domains, and wherein the heteromultimer has a hydrodynamic size of at least 24 kDa and a half life of at least 0.25 hours, and wherein each variable domain has an antigen binding site, and each antibody single variable domain binds antigen as a single antibody variable domain in the heteromultimer.
In one embodiment, the heteromultimer is linked to at least one PEG polymer.
In one embodiment, the half life is between 0.25 and 170 hours.
In one embodiment, the heteromultimer has a t½ alpha of between 0.25 and 6 hours.
In one embodiment, the heteromultimer has a t½ beta of between 2 and 40 hours.
In one embodiment, each antibody single variable domain of the heteromultimer comprises either $V_H$ or $V_L$.

In one embodiment, the antibody single variable domain of the heteromultimer is engineered to contain an additional cysteine residue at the C-terminus of the antibody single variable domain.

In one embodiment, the antibody single variable domains of the heteromultimer are linked to each other by a peptide linker.

In one embodiment, the heteromultimer comprises only a first and second antibody single variable domain, wherein the first antibody single variable domain of the heteromultimer comprises an antibody single variable domain and a heavy chain (CH1) constant region, and wherein the second antibody single variable domain of the heteromultimer comprises an antibody single variable domain and a light chain (CL) constant region.

In one embodiment, the heteromultimer has specificity for TNFα.

In one embodiment, the heteromultimer dissociates from human TNFα with a dissociation constant ($K_d$) of 50nM to 20pM, and a $K_{off}$ rate constant of $5x10^{-1}$ to $1x10^{-7}$ $s^{-1}$, as determined by surface plasmon resonance.

In one embodiment, the heteromultimer neutralizes human TNFα in a standard cell assay with an ND50 of 500nM to 50pM.

In one embodiment, each antibody single variable domain of the heteromultimer has specificity for TNFα.

In one embodiment, each antibody single variable domain of the heteromultimer dissociates from human TNFα with a dissociation constant ($K_d$) of 50nM to 20pM, and a $K_{off}$ rate constant of $5x10^{-1}$ to $1X10^{-7}$ $s^{-1}$, as determined by surface plasmon resonance.

In one embodiment, each antibody single variable domain of the heteromultimer neutralizes human TNFα in a standard cell assay with an ND50 of 500nM to 50pM.

The invention also encompasses a PEG-linked antibody single variable domain specific for a target ligand which retains activity relative to a non-PEG-linked antibody single variable domain having the same antibody single variable domain as the PEG-linked antibody single variable domain, wherein activity is measured by affinity of the PEG-linked or non-PEG-linked antibody single variable domain to the target ligand.

In one embodiment, the PEG-linked antibody single variable domain retains at least 90% of the activity of a non-PEG-linked antibody single variable domain.

In one embodiment, the activity is measured by surface plasmon resonance as the binding of the PEG-linked antibody single variable domain to TNFα.

In one embodiment, the PEG-linked antibody single variable domain dissociates from human TNFα with a dissociation constant ($K_d$) of 50nM to 20pM, and a $K_{off}$ rate constant of $5x10^{-1}$ to $1x10^{-7}s^{-1}$, as determined by surface plasmon resonance.

In one embodiment, the activity is measured as the ability of the PEG-linked antibody single variable domain to neutralize human TNFα or TNF receptor 1 in a standard cell assay.

In one embodiment, the PEG-linked antibody single variable domain neutralizes human TNFα or TNF receptor 1 in a standard cell assay with an ND50 of 500nM to 50pM.

In one embodiment, the PEG-linked antibody single variable domain has an IC50 or ND50 which is no more than 10% greater than the IC50 or ND50 respectively of a non-PEG-linked antibody variable domain having the same antibody single variable domain as the PEG-linked antibody single variable domain.

The invention also encompasses a PEG-linked antibody single variable domain specific for a target antigen which specifically binds to the target antigen with a $K_d$ of 80 nM to 30pM.

The invention further encompasses a PEG-linked antibody single variable domain which specifically binds to a target antigen with a $K_d$ of 3 nM to 30 pM.

The invention still further encompasses a PEG-linked antibody single variable domain which specifically binds to a target antigen with a $K_d$ of 100 pM to 30 pM.

In one embodiment, the PEG-linked antibody single variable domain binds to TNFα with a dissociation constant ($K_d$) of 50nM to 20pM, and a $K_{off}$ rate constant of $5x10^{-1}$ to $1x10^{-7}s^{-1}$, as determined by surface plasmon resonance.

In one embodiment, the binding is measured as the ability of the PEG-linked antibody single variable domain to neutralize human TNFα or TNF receptor 1 in a standard cell assay.

In one embodiment, the PEG-linked antibody single variable domain neutralizes human TNFα or TNF receptor 1 in a standard cell assay with an ND50 of 500nM to 50pM.

The present invention also encompasses a PEG-linked antibody single variable domain homomultimer which retains activity relative to a non-PEG-linked antibody single variable domain homomultimer having the same antibody single variable domain as the PEG-linked antibody single variable domain, wherein activity is measured by affinity of the PEG-linked or non-PEG-linked antibody single variable domain homomultimer to a target ligand.

In one embodiment, the PEG-linked antibody single variable domain retains 90% of the activity of a non-PEG-linked antibody single variable domain homomultimer.

In one embodiment, the activity is measured as the binding of the PEG-linked antibody single variable domain homomultimer to TNFα.

In one embodiment, the activity is measured as the ability of the PEG-linked antibody single variable domain

homomultimer to inhibit cell cytotoxicity in response to TNFα.

In one embodiment, the PEG-linked antibody single variable domain has an IC50 which is no more than 10% greater than the IC50 of a non-PEG-linked antibody variable domain homomultimer.

In one embodiment, each member of the homomultimer comprises either $V_H$ or $V_L$.

In one embodiment, the homomultimer comprises an antibody single variable domain that is engineered to contain an additional cysteine residue at the C-terminus of the antibody single variable domain.

In one embodiment, the members of the homomultimer are linked to each other by a peptide linker.

In one embodiment, the multimer comprises only a first and second member, the first member of the homodimer comprises an antibody single variable domain and a heavy chain (CH1) constant region, and the second member of the homodimer comprises an antibody single variable domain and a light chain (CL) constant region.

The invention still further encompasses a PEG-linked antibody single variable domain heteromultimer which retains activity relative to a non-PEG-linked antibody single variable domain heteromultimer having the same antibody single variable domain as the PEG-linked antibody single variable domain, wherein activity is measured by affinity of the PEG-linked or non-PEG-linked antibody single variable domain heteromultimer to a target ligand.

In one embodiment, the PEG-linked antibody single variable domain retains 90% of the activity of a non-PEG-linked antibody single variable domain heteromultimer.

In one embodiment, the activity is measured as the binding of the PEG-linked antibody single variable domain heteromultimer to TNFα.

In one embodiment, the activity is measured as the ability of the PEG-linked antibody single variable domain heteromultimer to inhibit cell cytotoxicity in response to TNFα.

In one embodiment, the PEG-linked antibody single variable domain has an IC50 which is no more than 10% greater than the IC50 of a non-PEG-linked antibody variable domain heteromultimer having the same antibody single variable domain as the PEG-linked antibody single variable domain.

In one embodiment, each member of the heteromultimer comprises either $V_H$ or $V_L$.

In one embodiment, each of the antibody single variable domain is engineered to contain an additional cysteine residue at the C-terminus of the antibody single variable domain.

In one embodiment, the members of the heteromultimer are linked to each other by a peptide linker.

In one embodiment, the multimer comprises only a first and second member, the first member of the heteromultimer comprises an antibody single variable domain and a heavy chain (CH1) constant region, and the second member of the homodimer comprises an antibody single variable domain and a light chain (CL) constant region.

The invention still further encompasses a PEG-linked homomultimer of antibody single variable domains which specifically binds to a target antigen with a $K_d$ of 80 nM to 30 pM.

In one embodiment, the PEG-linked homomultimer binds to TNFα with a dissociation constant ($K_d$) of 50nM to 20pM, and a $K_{off}$ rate constant of $5x10^{-1}$ to $1x10^{-7}$ $s^{-1}$, as determined by surface plasmon resonance.

In one embodiment, the binding is measured as the ability of the PEG-linked homomultimer to neutralize human TNFα or TNF receptor 1 in a standard cell assay.

In one embodiment, the PEG-linked homomultimer neutralizes human TNFα or TNF receptor 1 in a standard cell assay with an ND50 of 500nM to 50pM.

The present invention also encompasses a PEG-linked homomultimer of antibody single variable domains which specifically binds to a target antigen with a $K_d$ of 3 nM to 30 pM.

The present invention also encompasses a PEG-linked homomultimer of antibody single variable domains which specifically binds to a target antigen with a $K_d$ of 100 pM to 30 pM.

The present invention also encompasses a PEG-linked heteromultimer of antibody single variable domains which specifically binds to a target antigen with a $K_d$ of 80 nM to 30 pM.

The present invention also encompasses a PEG-linked heteromultimer of antibody single variable domains which specifically binds to a target antigen with a $K_d$ of 3 nM to 30 pM.

The present invention also encompasses a PEG-linked heteromultimer of antibody single variable domains which specifically binds to a target antigen with a $K_d$ of 100 pM to 30 pM.

The present invention also encompasses an antibody single variable domain comprising at least one solvent-accessible lysine residue at a predetermined location in the antibody single variable domain which is linked to a PEG molecule.

In one embodiment, the PEG is linked to the solvent-accessible lysine in the form of a PEG linked N-hydroxylsuccinimide active ester.

In one embodiment, the N-hydroxylsuccinimide active ester is selected from the group consisting of PEG-O-$CH_2CH_2CH_2$-$CO_2$-NHS; PEG-O-$CH_2$-NHS; PEG-O-$CH_2CH_2$-$CO_2$-NHS; PEG-S-$CH_2CH_2$-CO-NHS; PEG-$O_2$CNH-CH(R)-$CO_2$-NHS; PEG-NHCO-$CH_2CH_2$-CO-NHS; and PEG-O-$CH_2$-$CO_2$-NHS; where R is $(CH_2)_4)NHCO_2$(mPEG).

In one embodiment, the PEG is a branched PEG.

The present invention also encompasses an antibody single variable domain multimer, each member of the

multimer comprising at least one solvent accessible lysine residue which is linked to a PEG molecule.

In one embodiment, the multimer is a homomultimer.

In one embodiment, the multimer is a heteromultimer.

In one embodiment, the multimer is a hetero- or homotrimer.

In one embodiment, the multimer is a hetero- or homotetramer.

The invention further encompasses an antibody single variable domain homo- or hetero-trimer or tetramer comprising at least one solvent-accessible cysteine residue which is linked to a PEG molecule.

In one embodiment, the PEG is linked to the solvent-accessible cysteine by a sulfhydryl-selective reagent selected from the group consisting of maleimide, vinyl sulfone, and thiol.

The invention also encompasses a PEG-linked antibody variable region polypeptide having a half life which is at least seven times greater than the half life of the same antibody variable region polypeptide not linked to PEG.

In one embodiment, the PEG-linked antibody variable region has a hydrodynamic size of at least 24 kDa.

In one embodiment, the PEG-linked antibody variable region has a hydrodynamic size of between 24 kDa and 500 kDa.

The invention still further encompasses a pharmaceutical formulation comprising a PEG-linked antibody single variable domain having a half life of at least 0.25 hours; and a carrier.

The invention also encompasses a pharmaceutical formulation comprising a PEG-linked antibody single variable domain dimer having a half life of at least 0.25 hours and having a hydrodynamic size of at least 24 kDa; and a carrier.

The invention also encompasses a pharmaceutical formulation comprising a PEG-linked antibody single variable domain heterotrimer or homotrimer or heterotetramer or homotetramer, wherein each variable domain has an antigen binding site, and each variable domain binds antigen as a single variable domain.

The invention further encompasses a pharmaceutical formulation comprising a PEG-linked antibody single variable domain, wherein the PEG-linked antibody single variable domain is degraded by no more than 10% after administration of the pharmaceutical formulation to the stomach of an animal.

The invention still further encompasses a pharmaceutical formulation comprising a PEG-linked antibody single variable domain, wherein the PEG-linked antibody single variable domain is degraded by no more than 10% in vitro by exposure to pepsin at pH 2.0 for 30 minutes.

In one embodiment, the pharmaceutical formulation is suitable for oral administration or is suitable for parenteral administration via a route selected from the group consisting of intravenous, intramuscular or intraperitoneal injection, orally, sublingually, topically, by inhalation, implantation, rectal, vaginal, subcutaneous, and transdermal administration. A still further aspect of the invention is to provide a method and molecules for delivery of therapeutic polypeptides and/or agents across natural barriers such as the blood-brain barrier, lung-blood barrier.

The invention also encompasses a method for reducing the degradation of an antibody single variable domain monomer or multimer by pepsin comprising linking the single variable domain to at least one PEG polymer.

In one embodiment, degradation is reduced in the stomach of an animal.

In one embodiment, degradation is reduced *in vitro* by at least 10% when the antibody single variable domain is exposed to pepsin at pH 2.0 for 30 minutes.

Definitions

As used herein, the term "domain" refers to a folded protein structure which retains its tertiary structure independently of the rest of the protein. Generally, domains are responsible for discrete functional properties of proteins, and in many cases may be added, removed or transferred to other proteins without loss of function of the remainder of the protein and/or of the domain.

By "antibody single variable domain" is meant a folded polypeptide domain which comprises sequences characteristic of immunoglobulin variable domains and which specifically binds an antigen (i.e., dissociation constant of 1 μM or less), and which binds antigen as a single variable domain; that is, without any complementary variable domain. A "antibody single variable domain" therefore includes complete antibody variable domains as well as modified variable domains, for example in which one or more loops have been replaced by sequences which are not characteristic of antibody variable domains or antibody variable domains which have been truncated or comprise N- or C-terminal extensions, as well as folded fragments of variable domains which retain a dissociation constant of 500 nM or less (e.g., 450 nM or less, 400 nM or less, 350 nM or less, 300 nM or less, 250 nM or less, 200 nM or less, 150 nM or less, 100 nM or less) and the target antigen specificity of the full-length domain. Preferably an antibody single variable domain useful in the invention is selected from the group of $V_H$ and $V_L$, including Vkappa and Vlambda. According to the present invention, methods and compositions described herein that utilize $V_H$ domains can also utilize camelid $V_{HH}$ domains Antibody single variable domains are known in the art, and are described in e.g., Ward et al., Nature. 1989 Oct 12;341 (6242):544-6, the entirety of which is incorporated herein by reference.

The phrase "antibody single variable domain" encompasses not only an isolated antibody single variable domain

polypeptide, but also larger polypeptides that comprise one or more monomers of an antibody single variable domain polypeptide sequence. A "domain antibody" or "dAb" is equivalent to a "antibody single variable domain" polypeptide as the term is used herein. An antibody single variable domain polypeptide, as used herein refers to a mammalian single immunoglobulin variable domain polypeptide, preferably human, but also includes rodent (for example, as disclosed in WO00/29004, the contents of which are incorporated herein in their entirety) or camelid $V_{HH}$ dAbs. Camelid dAbs are antibody single variable domain polypeptides which are derived from species including camel, llama, alpaca, dromedary, and guanaco, and comprise heavy chain antibodies naturally devoid of light chain: $V_{HH}$. $V_{HH}$ molecules are about 10x smaller than IgG molecules, and as single polypeptides, they are very stable, resisting extreme pH and temperature conditions. Moreover, camelid antibody single variable domain polypeptides are resistant to the action of proteases. Camelid antibodies are described in, for example, U.S. Pat. Nos. 5,759,808; 5,800,988; 5,840,526; 5,874,541; 6,005,079; and 6,015,695, the contents of each of which are incorporated herein in their entirety. Camelid $V_{HH}$ antibody single variable domain polypeptides useful according to the invention include a class of camelid antibody single variable domain polypeptides having human-like sequences, wherein the class is characterized in that the $V_{HH}$ domains carry an amino acid from the group consisting of glycine, alanine, valine, leucine, isoleucine, proline, phenylalanine, tyrosine, tryptophan, methionine, serine, threonine, asparagine, or glutamine at position 45, such as for example L45, and further comprise a tryptophan at position 103 according to the Kabat numbering. Humanized camelid $V_{HH}$ polypeptides are taught, for example in WO04/041862, the teachings of which are incorporated herein in their entirety. It will be understood by one of skill in the art that naturally occurring camelid antibody single variable domain polypeptides may be modified according to the teachings of WO04/041862 (e.g., amino acid substitutions at positions 45 and 103) to generate humanized camelid $V_{HH}$ polypeptides.

According to the invention, the terms "antibody single variable domain polypeptide", "antibody single variable domain", "single antibody variable domain", and "immunoglobulin single variable domain" are understood to be equivalent.

As used herein, the phrase "sequence characteristic of immunoglobulin variable domains" refers to an amino acid sequence that is homologous, over 20 or more, 25 or more, 30 or more, 35 or more, 40 or more, 45 or more, or even 50 or more contiguous amino acids, to a sequence comprised by an immunoglobulin variable domain sequence.

As used herein, "linked" refers to the attachment of a polymer moiety, such as PEG to an amino acid residue of an antibody single variable domain or polypeptide of the invention. Attachment of a PEG polymer to an amino acid residue of a dAb or polypeptide is referred to as "PEGylation" and may be achieved using several PEG attachment moieties including, but not limited to N-hydroxylsuccinimide (NHS) active ester, succinimidyl propionate (SPA), maleimide (MAL), vinyl sulfone (VS), or thiol. A PEG polymer, or other polymer, can be linked to a dAb polypeptide at either a predetermined position, or may be randomly linked to the dAb molecule. It is preferred, however, that the PEG polymer be linked to a dAb or polypeptide at a predetermined position. A PEG polymer may be linked to any residue in the dAb or polypeptide, however, it is preferable that the polymer is linked to either a lysine or cysteine, which is either naturally occurring in the dAb or polypeptide, or which has been engineered into the dAb or polypeptide, for example, by mutagenesis of a naturally occurring residue in the dAb to either a cysteine or lysine. As used herein, "linked" can also refer to the association of two or more antibody single variable domain polypeptide monomers to form a dimer, trimer, tetramer, or other multimer, dAb monomers can be linked to form a multimer by several methods known in the art including, but not limited to expression of the dAb monomers as a fusion protein, linkage of two or more monomers via a peptide linker between monomers, or by chemically joining monomers after translation either to each other directly or through a linker by disulfide bonds, or by linkage to a di-, tri- or multivalent linking moiety (e.g., a multi-arm PEG).

As used herein, the phrase "directly linked" with respect to a polymer "directly linked" to an antibody single variable domain polypeptide refers to a situation in which the polymer is attached to a residue (naturally occurring or engineered) which is part of the variable domain, e.g., not contained within a constant region, hinge region, or linker peptide. Conversely, as used herein, the phrase "indirectly linked" to an antibody single variable domain refers to a linkage of a polymer molecule to an antibody single variable domain wherein the polymer is not attached to an amino acid residue which is part of the variable region (e.g., can be attached to a hinge region). A polymer is "indirectly linked" if it is linked to the single variable domain via a linking peptide, that is the polymer is not attached to an amino acid residue which is a part of the antibody single variable domain itself Alternatively a polymer is "indirectly linked" to an antibody single variable domain if it is linked to a C-terminal hinge region of the single variable domain, or attached to any residues of a constant region which may be present as part of the antibody single variable domain polypeptide.

As used herein, the terms "homology" or "similarity" or "identity" refer to the degree with which two nucleotide or amino acid sequences structurally resemble each other. A homologous sequence according to the invention may be a polypeptide modified by the addition, deletion or substitution of amino acids, said modification not substantially altering the functional characteristics compared with the unmodified polypeptide. Where an antibody single variable domain polypeptide of the invention is a camelid polypeptide, a homologous sequence according to the invention may be a sequence which exists in other *Camelidae* species such as camel, dromedary, llama, alpaca, and guanaco. As used herein, sequence "similarity" is a measure of the degree to which amino acid sequences share similar amino acid

residues at corresponding positions in an alignment of the sequences. Amino acids are similar to each other where their side chains are similar. Specifically, "similarity" encompasses amino acids that are conservative substitutes for each other. A "conservative" substitution is any substitution that has a positive score in the blosum62 substitution matrix (Hentikoff and Hentikoff, 1992, Proc. Natl. Acad. Sci. USA 89: 10915-10919). By the statement "sequence A is n% similar to sequence B" is meant that n% of the positions of an optimal global alignment between sequences A and B consists of identical amino acids or conservative substitutions. Optimal global alignments can be performed using the following parameters in the Needleman-Wunsch alignment algorithm:

For polypeptides:

Substitution matrix: blosum62.

Gap scoring function: -A -B*LG, where A=11 (the gap penalty), B=1 (the gap length penalty) and LG is the length of the gap.

For nucleotide sequences:

Substitution matrix: 10 for matches, 0 for mismatches.

Gap scoring function: -A -B*LG where A=50 (the gap penalty), B=3 (the gap length penalty) and LG is the length of the gap.

Typical conservative substitutions are among Met, Val, Leu and Ile; among Ser and Thr; among the residues Asp, Glu and Asn; among the residues Gln, Lys and Arg; or aromatic residues Phe and Tyr.

As used herein, two sequences are "homologous" or "similar" to each other where they have at least 85% sequence similarity to each other when aligned using either the Needleman-Wunsch algorithm or the "BLAST 2 sequences" algorithm described by Tatusova & Madden, 1999, FEMS Microbiol Lett. 174:247-250. Where amino acid sequences are aligned using the "BLAST 2 sequences algorithm," the Blosum 62 matrix is the default matrix.

As used herein, the terms "low stringency," "medium stringency," "high stringency," or "very high stringency conditions" describe conditions for nucleic acid hybridization and washing. Guidance for performing hybridization reactions can be found in Current Protocols in Molecular Biology, John Wiley & Sons, N.Y. (1989), 6.3.1-6.3.6, which is incorporated herein by reference in its entirety. Aqueous and nonaqueous methods are described in that reference and either can be used. Specific hybridization conditions referred to herein are as follows: (1) low stringency hybridization conditions in 6X sodium chloride/sodium citrate (SSC) at about 45°C, followed by two washes in 0.2X SSC, 0.1% SDS at least at 50°C (the temperature of the washes can be increased to 55°C for low stringency conditions); (2) medium stringency hybridization conditions in 6X SSC at about 45°C, followed by one or more washes in 0.2X SSC, 0.1% SDS at 60°C; (3) high stringency hybridization conditions in 6X SSC at about 45°C, followed by one or more washes in 0.2X SSC, 0.1% SDS at 65°C; and preferably (4) very high stringency hybridization conditions are 0.5M sodium phosphate, 7% SDS at 65°C, followed by one or more washes at 0.2X SSC, 1% SDS at 65°C.

As used herein, the phrase "specifically binds" refers to the binding of an antigen by an antibody single variable domain with a dissociation constant ($K_d$) of 1 $\mu$M or lower as measured by surface plasmon resonance analysis using, for example, a BIAcore™ surface plasmon resonance system and BIAcore™ kinetic evaluation software (e.g., version 2.1). The affinity or $K_d$ for a specific binding interaction is preferably about 500 nM or lower, preferably about 300 nM, preferably about 100 nM or lower, more preferably about 80 nM or lower, and preferably as low as 10 pM.

As used herein, the term "high affinity binding" refers to binding with a $K_d$ of less than or equal to 100 nM.

As used herein, the phrase "at a concentration of" means that a given polypeptide is dissolved in solution (preferably aqueous solution) at the recited mass or molar amount per unit volume and thus includes molar concentration and weight/volume percent. A polypeptide that is present "at a concentration of X" or "at a concentration of at least X" is therefore exclusive of both dried and crystallized preparations of a polypeptide.

As used herein, the term "repertoire" refers to a collection of diverse variants, for example nucleic acid variants which differ in nucleotide sequence or polypeptide variants which differ in amino acid sequence. A library according to the invention will encompass a repertoire of polypeptides or nucleic acids. According to the present invention, a repertoire of polypeptides is designed to possess a binding site for a generic ligand and a binding site for a target ligand. The binding sites may overlap, or be located in the same region of the molecule, but their specificities will differ. A library used in the present invention will encompass a repertoire of polypeptides comprising at least 1000 members.

As used herein, the term "library" refers to a mixture of heterogeneous polypeptides or nucleic acids. The library is composed of members, each of which have a single polypeptide or nucleic acid sequence. To this extent, *library* is synonymous with *repertoire.* Sequence differences between library members are responsible for the diversity present in the library. The library may take the form of a simple mixture of polypeptides or nucleic acids, or may be in the form of organisms or cells, for example bacteria, viruses, animal or plant cells and the like, transformed with a library of nucleic acids. Preferably, each individual organism or cell contains only one or a limited number of library members. Advantageously, the nucleic acids are incorporated into expression vectors, in order to allow expression of the polypeptides encoded by the nucleic acids. In a preferred aspect, therefore, a library may take the form of a population of host organisms, each organism containing one or more copies of an expression vector containing a single member of the library in nucleic acid form which can be expressed to produce its corresponding polypeptide member. Thus, the population of host organisms has the potential to encode a large repertoire of genetically diverse polypeptide variants.

As used herein, "polymer" refers to a macromolecule made up of repeating monomeric units, and can refer to a synthetic or naturally occurring polymer such as an optionally substituted straight or branched chain polyalkylene, polyalkenylene, or polyoxyalkylene polymer or a branched or unbranched polysaccharide. A "polymer" as used herein, preferably refers to an optionally substituted or branched chain poly(ethylene glycol), poly(propylene glycol), or poly (vinyl alcohol) and derivatives thereof

As used herein, "PEG" or "PEG polymer" refers to polyethylene glycol, and more specifically can refer to a derivatized form of PEG, including, but not limited to N-hydroxylsuccinimide (NHS) active esters of PEG such as succinimidyl propionate, benzotriazole active esters, PEG derivatized with maleimide, vinyl sulfones, or thiol groups. Particular PEG formulations can include PEG-O-$CH_2CH_2CH_2$-$CO_2$-NHS; PEG-O-$CH_2$-NHS; PEG-O-$CH_2CH_2$-$CO_2$-NHS; PEG-S-$CH_2CH_2$-CO-NHS; PEG-$O_2$CNH-CH(R)-$CO_2$-NHS; PEG-NHCO-$CH_2CH_2$-CO-NHS; and PEG-O-$CH_2$-$CO_2$-NHS; where R is $(CH_2)_4)NHCO_2$(mPEG). PEG polymers useful in the invention may be linear molecules, or may be branched wherein multiple PEG moieties are present in a single polymer. Some particularly preferred PEG conformations that are useful in the invention include, but are not limited to the following:

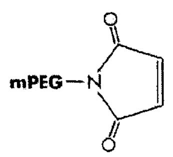

mPEG-MAL

;

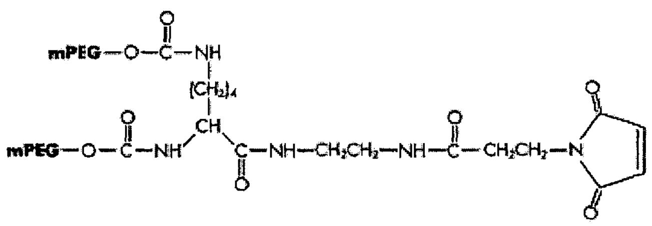

;

mPEG2-MAL

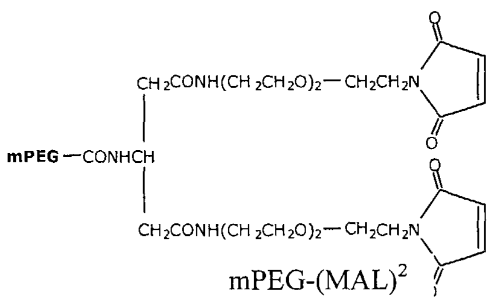

mPEG-(MAL)$^2$

;

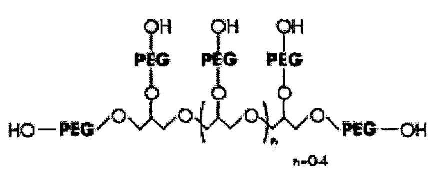

;

multi-arm PEG

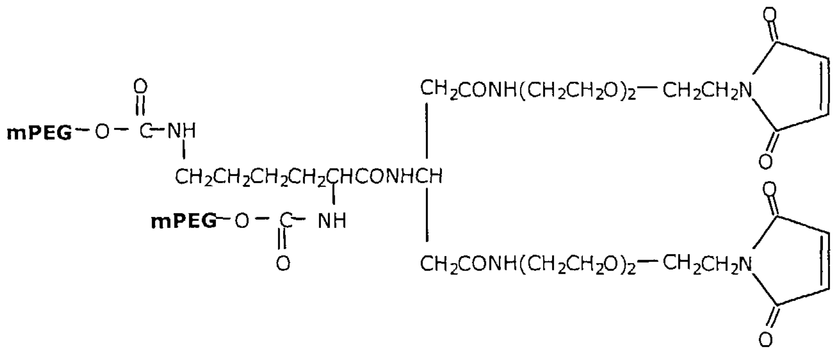

;

mPEG2-(MAL)$^2$

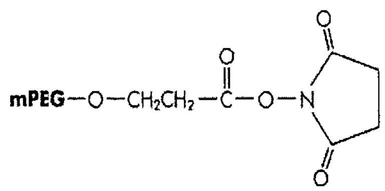

; and

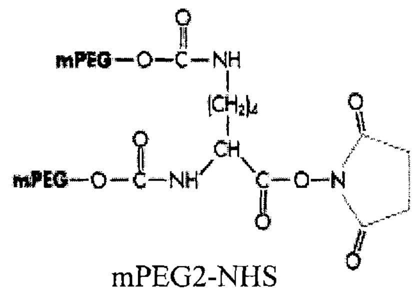

mPEG2-NHS

mPEG-SPA

As used herein, a "sulfhydryl-selective reagent" is a reagent which is useful for the attachment of a PEG polymer to a thiol-containing amino acid. Thiol groups on the amino acid residue cysteine are particularly useful for interaction with a sulfhydryl-selective reagent. Sulfhydryl-selective reagents which are useful in the invention include, but are not limited to maleimide, vinyl sulfone, and thiol. The use of sulfhydryl-selective reagents for coupling to cysteine residues is known in the art and may be adapted as needed according to the present invention (See Eg., Zalipsky, 1995, Bioconjug. Chem. 6:150; Greenwald et al., 2000, Crit. Rev. Ther. Drug Carrier Syst. 17:101; Herman et al 1994, Macromol. Chem. Phys. 195:203).

As used herein, an "antigen" is bound by an antibody or a binding region (e.g., a variable domain) of an antibody. Typically, antigens are capable of raising an antibody response *in vivo.* An antigen can be a peptide, polypeptide, protein, nucleic acid, lipid, carbohydrate, or other molecule, and includes multisubunit molecules. Generally, an immunoglobulin variable domain is selected for target specificity against a particular antigen.

As used herein, the term "epitope" refers to a unit of structure conventionally bound by an antibody single variable domain $V_H/V_L$ pair. Epitopes define the minimum binding site for an antibody, and thus represent the target of specificity of an antibody. In the case of a antibody single variable domain, an epitope represents the unit of structure bound by a variable domain in isolation.

As used herein, the term "neutralizing," when used in reference to an antibody single variable domain polypeptide as described herein, means that the polypeptide interferes (e.g., completely or at least partially suppresses or eradicates) with a measurable activity or function of the target antigen. A polypeptide is a "neutralizing" polypeptide if it reduces a measurable activity or function of the target antigen by at least 50%, and preferably at least 60%, 70%, 80%, 90%, 95% or more, up to and including 100% inhibition (i.e., no detectable effect or function of the target antigen). This reduction of a measurable activity or function of the target antigen can be assessed by one of skill in the art using standard methods of measuring one or more indicators of such activity or function. As an example, where the target is TNF-$\alpha$, neutralizing activity can be assessed using a standard L929 cell killing assay or by measuring the ability of an antibody single variable domain polypeptide to inhibit TNF-$\alpha$-induced expression of ELAM-1 on HUVEC, which measures TNF-$\alpha$-induced cellular activation. Analogous to "neutralizing" as used herein, "inhibit cell cytotoxicity" as used herein refers to a decrease in cell death as measured, for example, using a standard L929 cell killing assay, wherein cell cytotoxicity is inhibited were cell death is reduced by at least 10% or more.

As used herein, a "measurable activity or function of a target antigen" includes, but is not limited to, for example, cell signaling, enzymatic activity, binding activity, ligand-dependent internalization, cell killing, cell activation, promotion

of cell survival, and gene expression. One of skill in the art can perform assays that measure such activities for a given target antigen. Preferably, "activity", as used herein, is defined by (1) ND50 in a cell-based assay; (2) affinity for a target ligand, (3) ELISA binding, or (4) a receptor binding assay. Methods for performing these tests are known to those of skill in the art and are described in further detail below.

As used herein , "dAb activity" or "antibody single variable domain activity" refers to the ability of the antibody single variable domain or polypeptide to bind antigen. As used herein, "retains activity" refers to a level of activity of the PEG-linked antibody single variable domain or polypeptide which is at least 10% of the level of activity of a non-PEG-linked antibody single variable domain or polypeptide, preferably at least 20%, 30%, 40%, 50%, 60%, 70%, 80% and up to 90%, preferably up to 95%, 98%, and up to 100% of the activity of a non-PEG-linked antibody single variable domain or polypeptide comprising the same variable domain as the PEG-linked antibody single variable domain or polypeptide, wherein activity is determined as described above. More specifically, the activity of a PEG-linked antibody single variable domain or polypeptide compared to a non-PEG linked antibody variable domain or polypeptide should be determined on a single antibody variable domain or polypeptide molar basis; that is equivalent numbers of moles of each of the PEG-linked and non-PEG-linked antibody single variable domain should be used in each trial wherein all other conditions are equivalent between trials. In determining whether a particular PEG-linked antibody single variable domain "retains activity", it is preferred that the activity of a PEG-linked antibody single variable domain be compared with the activity of the same antibody single variable domain in the absence of PEG.

As used herein, the phrase "specifically binds" refers to the binding of an antigen by an immunoglobulin variable domain or polypeptide with a dissociation constant (Kd) of 1 $\mu$M or lower as measured by surface plasmon resonance analysis using, for example, a BIAcore™ surface plasmon resonance system and BIAcore™ kinetic evaluation software (e.g., version 2.1). The affinity or Kd for a specific binding interaction is preferably about 1 $\mu$M or lower, preferably 500 nM or lower, more preferably 100 nM or lower, more preferably about 80 nM or lower, and preferably as low as 10 pM.

As used herein, the terms "heterodimer," "heterotrimer", "heterotetramer", and "heteromultimer" refer to molecules comprising two, three or more (e.g., four, five, six, seven and up to eight or more) monomers of two or more different single immunoglobulin variable domain polypeptide sequence, respectively. For example, a heterodimer would include two $V_H$ sequences, such as $V_{H1}$ and $V_{H2}$, or $V_{HH1}$ and $V_{HH2}$, or may alternatively include a combination of $V_H$ and $V_L$. Similar to a homodimer, trimer, or tetramer, the monomers in a heterodimer, heterotrimer, heterotetramer, or heteromultimer can be linked either by expression as a fusion polypeptide, e.g., with a peptide linker between monomers, or, by chemically joining monomers after translation either to each other directly or through a linker by disulfide bonds, or by linkage to a di-, tri- or multivalent linking moiety. In one embodiment, the monomers in a heterodimer, trimer, tetramer, or multimer can be linked by a multi-arm PEG polymer, wherein each monomer of the dimer, trimer, tetramer, or multimer is linked as described above to a PEG moiety of the multi-arm PEG.

As used herein, the term "half-life" refers to the time taken for the serum concentration of a ligand (e.g., a single immunoglobulin variable domain) to reduce by 50%, *in vivo,* for example due to degradation of the ligand and/or clearance or sequestration of the ligand by natural mechanisms. The antibody single variable domains of the invention are stabilized *in vivo* and their half-life increased by binding to molecules which are hypothesized to resist degradation and/or clearance or sequestration, such as PEG. The half-life of a dAb or polypeptide is increased if its functional activity persists (to a degree), *in vivo,* for a longer period than a similar dAb which is not linked to a PEG polymer. Typically, the half life of a PEGylated dAb or polypeptide is increased by 10%, 20%, 30%, 40%, 50% or more relative to a non-PEGylated dAb or polypeptide. Increases in the range of 2x, 3x, 4x, 5x, 10x, 20x, 30x, 40x, 50x or more of the half life are possible. Alternatively, or in addition, increases in the range of up to 30x, 40x, 50x, 60x, 70x, 80x, 90x, 100x, 150x of the half life are possible. According to the invention, a PEG-linked antibody single variable domain or polypeptide has a half-life of between 0.25 and 170 hours, preferably between 1 and 100 hours, more preferably between 30 and 100 hours, and still more preferably between 50 and 100 hours, and up to 170, 180, 190, and 200 hours or more.

As used herein, "resistant to degradation" or "resists degradation" with respect to a PEG or other polymer linked dAb monomer or multimer means that the PEG- or other polymer-linked dAb monomer or multimer is degraded by no more than 10% when exposed to pepsin at pH 2.0 for 30 minutes, and preferably not degraded at all. With specific reference to a PEG- or other polymer-linked dAb multimer (e.g., hetero- or homodimer, trimer, tetramer, etc) of the invention, such a multimer is degraded by less than 5%, and is preferably not degraded at all in the presence of pepsin at pH 2.0 for 30 minutes.

As used herein, "hydrodynamic size" refers to the apparent size of a molecule (e.g., a protein molecule) based on the diffusion of the molecule through an aqueous solution. The diffusion, or motion of a protein through solution can be processed to derive an apparent size of the protein, where the size is given by the "Stokes radius" or "hydrodynamic radius" of the protein particle. The "hydrodynamic size" of a protein depends on both mass and shape (conformation), such that two proteins having the same molecular mass may have differing hydrodynamic sizes based on the overall conformation of the protein. The hydrodynamic size of a PEG-linked antibody single variable domain (including antibody variable domain multimers as described herein) can be in the range of 24 kDa to 500 kDa; 30 to 500 kDa; 40 to 500 kDa; 50 to 500 kDa; 100 to 500 kDa; 150 to 500 kDa; 200 to 500 kDa; 250 to 500 kDa; 300 to 500 kDa; 350 to 500 kDa;

400 to 500 kDa and 450 to 500 kDa. Preferably the hydrodynamic size of a PEGylated dAb of the invention is 30 to 40 kDa; 70 to 80 kDa or 200 to 300 kDa. Where an antibody variable domain multimer is desired for use in imaging applications, the multimer should have a hydrodynamic size of between 50 and 100 kDa. Alternatively, where an antibody single domain multimer is desired for therapeutic applications, the multimer should have a hydrodynamic size of greater than 200 kDa.

As used herein "TAR1" refers to a dAb whose target antigen is TNFα.

As used herein "TAR2" refers to a dAb whose target antigen is the human p55-TNFα receptor.

BRIEF DESCRIPTION OF THE DRAWINGS

Figure 1 shows the results of a receptor binding assay showing the affinity of a range of PEGylated formats of TAR1-5-19.

Figure 2 shows the results of a cell cytotoxicity assay showing the affinity of a range of PEGylated formats of TAR1-5-19.

Figure 3 shows SDS page gels showing the results of affinity binding of various formats of PEGylated HEL4 dAb to lysozyme. Lane descriptions are provided in the Examples.

Figure 4 shows the results of a receptor binding assay showing the affinity of TAR2-10-27 and the 40K PEGylated monomer.

Figure 5 shows the protease stability of TAR1-5-19 and PEGylated variants against the action of pepsin at pH 2.0.

Figure 6 shows schematics of monomer PEGylation of dAbs. Figure 6-1 shows an unmodified $V_H$ or $V_L$ dAb. Figure 6-2 shows a $V_H$ or $V_L$ with surface PEGylation. Figure 6-3 shows $V_H$ or $V_L$ dAb with a C-terminal cysteine which is linked to PEG.

Figure 7 shows schematics of PEGylated $V_H$ or $V_L$ hetero- or homodimeric dAbs. Figure 7-4 shows $V_H$ or $V_L$ disulfide dimer formed by a C-terminal disulfide bond. Figure 7-5 shows $V_H$ or $V_L$ disulfide dimer PEGylated on one subunit. Figure 7-6 shows $V_H$ or $V_L$ disulfide dimer PEGylated on both subunits. Figure 7-7 shows $V_H$ or $V_L$ dimer formed by a branched/forked/multi-arm PEG via a C-terminal cysteine. Figure 7-8 shows a $V_H$ or $V_L$ disulfide dimer formed by a surface disulfide bond. Figure 7-9 shows a $V_H$ or $V_L$ disulfide dimer PEGylated on one subunit. Figure 7-10 shows a $V_H$ or $V_L$ dimer PEGylated on both subunits. Figure 7-11 shows a $V_H$ or $V_L$ dimer formed by a branched/forked/multi-arm PEG via surface cysteine residues.

Figure 8 shows further schematics of PEGylated $V_H$ or $V_L$ hetero- or homodimers of the invention. Figure 8-12 shows a $V_H$ or $V_L$ linked dimer formed by a $(Gly_4Ser)_n$ linker (n=0-10). Figure 8-13 shows a $V_H$ or $V_L$ linker dimer PEGylated on one subunit. Figure 8-14 shows a $V_H$ or $V_L$ linker dimer PEGylated on both subunits. Figure 8-15 shows a $V_H$ or $V_L$ linker dimer PEGylated via the linker. Figure 8-16 shows a $V_H$ or $V_L$ linker dimer with a C-terminal cysteine residue. Figure 8-17 shows two $V_H$ or $V_L$ linker dimers dimerized by disulfide bonds.

Figure 9 shows schematics of PEGylated linker dAb dimers. Figure 9-18 and 9-19 show $V_H$ or $V_L$ linker dimers PEGylated via a C-terminal cysteine residue on one subunit. Figure 9-20 shows a $V_H$ or $V_L$ linker dimer PEGylated via a cysteine present in the linker. Figure 9-21 shows a $V_H$ or $V_L$ or VL linker dimer PEGylated via a cysteine present on one subunit. Figure 9-22 shows a $V_H$ or $V_L$ linker dimer PEGylated via cysteines present on both subunits.

Figure 10 shows schematic representations of PEGylation of $V_H$ or $V_L$ hetero- or homotrimeric dAbs. Figures 10-23 and 10-24 show PEGylation and formation of dAb trimers using 3-arm PEG to covalently trimerize via C-terminal amino acids. Figure 10-25 shows surface PEGylation of one of the dAb monomers, wherein the dAb trimer is formed via linker peptides. Figure 10-26 shows C-terminal PEGylation of one of the monomers of the dAb trimer. Figure 10-27 shows a dual-specific dAb trimer in which two of the dAb monomers have binding affinity for TNFα and the third monomer has a binding specificity for serum albumin. This format can also be PEGylated as shown in either of Figures 10-25 or 10-26.

Figure 11 shows a schematic representation of $V_H$ or $V_L$ hetero- or homotetrameric dAbs. Figure 11-28 shows a dAb

tetramer formed by linking a 4-arm PEG to C-terminal cysteines of each dAb monomer. Figures 11-29 and 11-31 show the formation of a dAb tetramer by linking two dAb linker dimers via a branched/multi-arm PEG where the PEG is linked either to a C-terminal cysteine (11-29) or to the linking peptide (11-31). Figure 11-30 shows a dAb tetramer in which each of the monomers of the tetramer are linked by a single branched PEG to C-terminal cysteine residues of each monomer. Figure 11-32 shows a dAb tetramer in which each of the monomers is linked to the other by a linking peptide. This configuration may be PEGylated using any of the strategies shown in Figures 10-25 or 10-26.

Figure 12 shows other multimeric PEG-linked dAb formats useful in the invention.
Figure 12-31 shows a tetramer of dAb linker dimers which are themselves linked to form the tetramer by a multi-arm PEG wherein the PEG is linked to C-terminal cysteine residues present in one of the monomers of each dimer. Figure 32 shows a tetramer of dAb linker dimers which are themselves linked to form the tetramer by a multi-arm PEG wherein each PEG is linked to cysteine residue present in the linker of each dimer pair.

Figure 13 shows the sequence of the $V_H$ framework based on germline sequence DP47 - JH4b (SEQ ID NO: 1, 2) HCDRs 1-3 are indicated by underlining.

Figure 14 shows the sequence of the $V_\kappa$ framework based on germline sequence DPκ9 - J κ1 (SEQ ID NO: 3,4). LCDRs 1-3 are indicated by underlining.

Figure 15 shows a plot showing the relationship of native hydrodynamic size of the dAb vs the in vivo serum half-life in mouse. Data shown in Table 8 was used to generate the graph.

Figure 16 shows protease stability profile of monomeric and 40K PEGylated TAR1-5-19. The relative activity is as a percentage of the no protease control. The proteases used were pepsin, porcine intestinal mucosa peptidase, elastase, crude bovine pancreatic protease (CBP) and rat intestinal powder (Rat In).

DETAILED DESCRIPTION

The present invention provides polymer linked dAbs and dAb homo- and heteromultimers with increased half-life and resistance to proteolytic degradation relative to non-polymer linked dAbs. The invention relates, in one embodiment, to PEG-linked dAbs and dAb multimers, and still further to PEG-linked dAb monomers, dimers, trimers, and tetramers having a half-life of at least 0.25 hours, and further having a hydrodynamic size of at least 24 kDa. The invention also relates to a PEG-linked antibody single variable domain which retains its activity relative to a non-PEG-linked antibody single variable domain comprising the same antibody variable domain as the PEG-linked antibody variable domain. This provides dAb molecules with increased therapeutic efficacy due to their prolonged circulation time and potent and efficacious activity.

In one embodiment, the invention provides PEG-linked dAb multimers which comprise at least two non-complementary variable domains. For example, the dAbs may comprise a pair of $V_H$ domains or a pair of $V_L$ domains. Advantageously, the domains are of non-camelid origin; preferably they are human domains or comprise human framework regions (FWs) and one or more heterologous CDRs. CDRs and framework regions are those regions of an immunoglobulin variable domain as defined in the Kabat database of Sequences of Proteins of Immunological Interest. In one embodiment, the dAb domains are of camelid origin.

Preferred human framework regions are those encoded by germline gene segments DP47 and DPK9. Advantageously, FW1, FW2 and FW3 of a $V_H$ or $V_L$ domain have the sequence of FW1, FW2 or FW3 from DP47 or DPK9. The human frameworks may optionally contain mutations, for example up to about 5 amino acid changes or up to about 10 amino acid changes collectively in the human frameworks used in the dAbs of the invention.

Preparation of Single Immunoglobulin Variable Domains:

The antibody single variable domains (or dAbs) of the invention are a folded polypeptide domain which comprises sequences characteristic of immunoglobulin variable domains and which specifically binds an antigen (i.e., dissociation constant of 500 nM or less), and which binds antigen as a single variable domain; that is, without any complementary variable domain. An antibody single variable domain therefore includes complete antibody variable domains as well as modified variable domains, for example in which one or more loops have been replaced by sequences which are not characteristic of antibody variable domains or antibody variable domains which have been truncated or comprise N- or C-terminal extensions, as well as folded fragments of variable domains which retain a dissociation constant of 500 nM or less (e.g., 450 nM or less, 400 nM or less, 350 nM or less, 300 nM or less, 250 nM or less, 200 nM or less, 150 nM or less, 100 nM or less) and the target antigen specificity of the full-length domain. Preferably an antibody single variable

domain useful in the invention is selected from the group of $V_{HH}$, VH and $V_L$, including $V_{kappa}$ and $V_{lambda}$.

Single immunoglobulin variable domains are prepared in a number of ways. For each of these approaches, well-known methods of preparing (e.g., amplifying, mutating, etc.) and manipulating nucleic acid sequences are applicable.

One means is to amplify and express the $V_H$ or $V_L$ region of a heavy chain or light chain gene for a cloned antibody known to bind the desired antigen. The boundaries of $V_H$ and $V_L$ domains are set out by Kabat et al. (1991, supra). The information regarding the boundaries of the $V_H$ and $V_L$ domains of heavy and light chain genes is used to design PCR primers that amplify the V domain from a cloned heavy or light chain coding sequence encoding an antibody known to bind a given antigen. The amplified V domain is inserted into a suitable expression vector, e.g., pHEN-1 (Hoogenboom et al., 1991, Nucleic Acids Res. 19:4133-4137) and expressed, either alone or as a fusion with another polypeptide sequence. The expressed $V_H$ or $V_L$ domain is then screened for high affinity binding to the desired antigen in isolation from the remainder of the heavy or light chain polypeptide. For all aspects of the present invention, screening for binding is performed as known in the art or as described herein below.

A repertoire of $V_H$ or $V_L$ domains is screened by, for example, phage display, panning against the desired antigen. Methods for the construction of bacteriophage display libraries and lambda phage expression libraries are well known in the art, and taught, for example, by: McCafferty et al., 1990, Nature 348: 552; Kang et al., 1991, Proc. Natl. Acad. Sci. U.S.A., 88:4363; Clackson et al., 1991, Nature 352: 624; Lowman et al., 1991, Biochemistry 30: 10832; Burton et al., 1991, Proc. Natl. Acad. Sci U.S.A. 88: 10134; Hoogenboom et al., 1991, Nucleic Acids Res. 19: 4133; Chang et al., 1991, J. Immunol. 147: 3610; Breitling et al., 1991, Gene 104: 147; Marks et al., 1991, J. Mol. Biol. 222: 581; Barbas et al., 1992, Proc. Natl. Acad. Sci. U.S.A. 89: 4457; Hawkins and Winter (1992) J. Immunol., 22: 867; Marks et al. (1992) J. Biol. Chem., 267: 16007; and Lerner et al. (1992) Science, 258: 1313. scFv phage libraries are taught, for example, by Huston et al., 1988, Proc. Natl. Acad. Sci U.S.A. 85:5879-5883; Chaudhary et al., 1990, Proc. Natl. Acad. Sci U.S.A. 87: 1066-1070; McCafferty et al., 1990, supra; Clackson et al., 1991, supra; Marks et al., 1991, supra; Chiswell et al., 1992, Trends Biotech. 10: 80; and Marks et al., 1992, supra. Various embodiments of scFv libraries displayed on bacteriophage coat proteins have been described. Refinements of phage display approaches are also known, for example as described in WO96/0621 and WO92/01047 (Medical Research Council et al.) and WO97/08320 (Morphosys, supra).

The repertoire of $V_H$ or $V_L$ domains can be a naturally-occurring repertoire of immunoglobulin sequences or a synthetic repertoire. A naturally-occurring repertoire is one prepared, for example, from immunoglobulin-expressing cells harvested from one or more animals, including humans. Such repertoires can be "naïve," i.e., prepared, for example, from human fetal or newborn immunoglobulin-expressing cells, or rearranged, i.e., prepared from, for example, adult human B cells. Natural repertoires are described, for example, by Marks et al., 1991, J. Mol. Biol. 222: 581 and Vaughan et al., 1996, Nature Biotech. 14: 309. If desired, clones identified from a natural repertoire, or any repertoire, for that matter, that bind the target antigen are then subjected to mutagenesis and further screening in order to produce and select variants with improved binding characteristics.

Synthetic repertoires of single immunoglobulin variable domains are prepared by artificially introducing diversity into a cloned V domain. Synthetic repertoires are described, for example, by Hoogenboom & Winter, 1992, J. Mol. Biol. 227: 381; Barbas et al., 1992, Proc. Natl. Acad. Sci. U.S.A. 89: 4457; Nissim et al., 1994, EMBO J. 13: 692; Griffiths et al., 1994, EMBO J. 13: 3245; DeKriuf et al., 1995, J. Mol. Biol. 248: 97; and WO 99/20749.

The antigen binding domain of a conventional antibody comprises two separate regions: a heavy chain variable domain ($V_H$) and a light chain variable domain ($V_L$ : which can be either $V_\kappa$ or $V_\lambda$). The antigen binding site of such an antibody is formed by six polypeptide loops: three from the $V_H$ domain (H1, H2 and H3) and three from the $V_L$ domain (L1, L2 and L3). The boundaries of these loops are described, for example, in Kabat et al. (1991, supra). A diverse primary repertoire of V genes that encode the $V_H$ and $V_L$ domains is produced *in vivo* by the combinatorial rearrangement of gene segments. The $V_H$ gene is produced by the recombination of three gene segments, $V_H$, D and JH. In humans, there are approximately 51 functional $V_H$ segments (Cook and Tomlinson (1995) Immunol Today 16:237), 25 functional D segments (Corbett et al. (1997) J. Mol. Biol. 268: 69) and 6 functional JH segments (Ravetch et al. (1981) Cell 27: 583), depending on the haplotype. The $V_H$ segment encodes the region of the polypeptide chain which forms the first and second antigen binding loops of the $V_H$ domain (H1 and H2), while the $V_H$, D and JH segments combine to form the third antigen binding loop of the $V_H$ domain (H3).

The $V_L$ gene is produced by the recombination of only two gene segments, $V_L$ and JL. In humans, there are approximately 40 functional $V_\kappa$ segments (Schäble and Zachau (1993) Biol. Chem. Hoppe-Seyler 374: 1001), 31 functional $V_\lambda$ segments (Williams et al. (1996) J. Mol. Biol. 264: 220; Kawasaki et al. (1997) Genome Res. 7: 250), 5 functional $J_\kappa$ segments (Hieter et al. (1982) J. Biol. Chem. 257: 1516) and 4 functional Jλ segments (Vasicek and Leder (1990) J. Exp. Med. 172: 609), depending on the haplotype. The $V_L$ segment encodes the region of the polypeptide chain which forms the first and second antigen binding loops of the $V_L$ domain (L1 and L2), while the $V_L$ and $J_L$ segments combine to form the third antigen binding loop of the $V_L$ domain (L3). Antibodies selected from this primary repertoire are believed to be sufficiently diverse to bind almost all antigens with at least moderate affinity. High affinity antibodies are produced *in vivo* by "affinity maturation" of the rearranged genes, in which point mutations are generated and selected by the

immune system on the basis of improved binding.

Analysis of the structures and sequences of antibodies has shown that five of the six antigen binding loops (H1, H2, L1, L2, L3) possess a limited number of main-chain conformations or canonical structures (Chothia and Lesk (1987) J. Mol. Biol. 196: 901; Chothia et al. (1989) Nature 342: 877). The main-chain conformations are determined by (i) the length of the antigen binding loop, and (ii) particular residues, or types of residue, at certain key position in the antigen binding loop and the antibody framework. Analysis of the loop lengths and key residues has enabled the prediction of the main-chain conformations of H1, H2, L1, L2 and L3 encoded by the majority of human antibody sequences (Chothia et al. (1992) J. Mol. Biol. 227: 799; Tomlinson et al. (1995) EMBO J. 14: 4628; Williams et al. (1996) J. Mol. Biol. 264: 220). Although the H3 region is much more diverse in terms of sequence, length and structure (due to the use of D segments), it also forms a limited number of main-chain conformations for short loop lengths which depend on the length and the presence of particular residues, or types of residue, at key positions in the loop and the antibody framework (Martin et al. (1996) J. Mol. Biol. 263: 800; Shirai et al. (1996) FEBS Letters 399: 1.

While, according to one embodiment of the invention, diversity can be added to synthetic repertoires at any site in the CDRs of the various antigen-binding loops, this approach results in a greater proportion of V domains that do not properly fold and therefore contribute to a lower proportion of molecules with the potential to bind antigen. An understanding of the residues contributing to the main chain conformation of the antigen-binding loops permits the identification of specific residues to diversify in a synthetic repertoire of $V_H$ or $V_L$ domains. That is, diversity is best introduced in residues that are not essential to maintaining the main chain conformation. As an example, for the diversification of loop L2, the conventional approach would be to diversify all the residues in the corresponding CDR (CDR2) as defined by Kabat et al. (1991, supra), some seven residues. However, for L2, it is known that positions 50 and 53 are diverse in naturally occurring antibodies and are observed to make contact with the antigen. The preferred approach would be to diversify only those two residues in this loop. This represents a significant improvement in terms of the functional diversity required to create a range of antigen binding specificities.

In one aspect, synthetic variable domain repertoires are prepared in $V_H$ or $V_\kappa$ backgrounds, based on artificially diversified germline $V_H$ or $V_\kappa$ sequences. For example, the $V_H$ domain repertoire is based on cloned germline $V_H$ gene segments V3-23/DP47 (Tomlinson et al., 1992, J. Mol. Biol. 227: 7768) and JH4b. The $V_\kappa$ domain repertoire is based, for example, on germline $V_\kappa$ gene segments O2/O12/DPK9 (Cox et al., 1994, Eur. J. Immunol. 24: 827) and J$\kappa$1. Diversity is introduced into these or other gene segments by, for example, PCR mutagenesis. Diversity can be randomly introduced, for example, by error prone PCR (Hawkins, et al., 1992, J. Mol. Biol. 226: 889) or chemical mutagenesis. As discussed above, however it is preferred that the introduction of diversity is targeted to particular residues. It is further preferred that the desired residues are targeted by introduction of the codon NNK using mutagenic primers (using the IUPAC nomenclature, where N = G, A, T or C, and K = G or T), which encodes all amino acids and the TAG stop codon. Other codons which achieve similar ends are also of use, including the NNN codon (which leads to the production of the additional stop codons TGA and TAA), DVT codon ((A/G/T) (A/G/C)T), DVC codon ((A/G/T)(A/G/C)C), and DVY codon((A/G/T)(A/G/C)(C/T). The DVT codon encodes 22% serine and 11% tyrosine, aspargine, glycine, alanine, aspartate, threonine and cysteine, which most closely mimics the distribution of amino acid residues for the antigen binding sites of natural human antibodies. Repertoires are made using PCR primers having the selected degenerate codon or codons at each site to be diversified. PCR mutagenesis is well known in the art; however, considerations for primer design and PCR mutagenesis useful in the methods of the invention are discussed below in the section titled "PCR Mutagenesis."

Diversified repertoires are cloned into phage display vectors as known in the art and as described, for example, in WO 99/20749. In general, the nucleic acid molecules and vector constructs required for the performance of the present invention are available in the art and are constructed and manipulated as set forth in standard laboratory manuals, such as Sambrook et al. (1989). Molecular Cloning: A Laboratory Manual, Cold Spring Harbor, USA.

The manipulation of nucleic acids in the present invention is typically carried out in recombinant vectors. As used herein, "vector" refers to a discrete element that is used to introduce heterologous DNA into cells for the expression and/or replication thereof. Methods by which to select or construct and, subsequently, use such vectors are well known to one of skill in the art. Numerous vectors are publicly available, including bacterial plasmids, bacteriophage, artificial chromosomes and episomal vectors. Such vectors may be used for simple cloning and mutagenesis; alternatively, as is typical of vectors in which repertoire (or pre-repertoire) members of the invention are carried, a gene expression vector is employed. A vector of use according to the invention is selected to accommodate a polypeptide coding sequence of a desired size, typically from 0.25 kilobase (kb) to 40 kb in length. A suitable host cell is transformed with the vector after *in vitro* cloning manipulations. Each vector contains various functional components, which generally include a cloning (or "polylinker") site, an origin of replication and at least one selectable marker gene. If a given vector is an expression vector, it additionally possesses one or more of the following: enhancer element, promoter, transcription termination and signal sequences, each positioned in the vicinity of the cloning site, such that they are operatively linked to the gene encoding a polypeptide repertoire member according to the invention.

Both cloning and expression vectors generally contain nucleic acid sequences that enable the vector to replicate in one or more selected host cells. Typically in cloning vectors, this sequence is one that enables the vector to replicate

independently of the host chromosomal DNA and includes origins of replication or autonomously replicating sequences. Such sequences are well known for a variety of bacteria, yeast and viruses. The origin of replication from the plasmid pBR322 is suitable for most Gram-negative bacteria, the 2 micron plasmid origin is suitable for yeast, and various viral origins (e.g. SV 40, adenovirus) are useful for cloning vectors in mammalian cells. Generally, the origin of replication is not needed for mammalian expression vectors unless these are used in mammalian cells able to replicate high levels of DNA, such as COS cells.

Advantageously, a cloning or expression vector also contains a selection gene also referred to as selectable marker. This gene encodes a protein necessary for the survival or growth of transformed host cells grown in a selective culture medium. Host cells not transformed with the vector containing the selection gene will therefore not survive in the culture medium. Typical selection genes encode proteins that confer resistance to antibiotics and other toxins, e.g. ampicillin, neomycin, methotrexate or tetracycline, complement auxotrophic deficiencies, or supply critical nutrients not available in the growth media.

Because the replication of vectors according to the present invention is most conveniently performed in *E. coli,* an *E. coli*-selectable marker, for example, the β-lactamase gene that confers resistance to the antibiotic ampicillin, is of use. These can be obtained from *E. coli* plasmids, such as pBR322 or a pUC plasmid such as pUC18 or pUC19.

Expression vectors usually contain a promoter that is recognized by the host organism and is operably linked to the coding sequence of interest. Such a promoter may be inducible or constitutive. The term "operably linked" refers to a juxtaposition wherein the components described are in a relationship permitting them to function in their intended manner. A control sequence "operably linked" to a coding sequence is ligated in such a way that expression of the coding sequence is achieved under conditions compatible with the control sequences.

Promoters suitable for use with prokaryotic hosts include, for example, the β-lactamase and lactose promoter systems, alkaline phosphatase, the tryptophan (trp) promoter system and hybrid promoters such as the tac promoter. Promoters for use in bacterial systems will also generally contain a Shine-Dalgarno sequence operably linked to the coding sequence.

In libraries or repertoires as described herein, the preferred vectors are expression vectors that enable the expression of a nucleotide sequence corresponding to a polypeptide library member. Thus, selection is performed by separate propagation and expression of a single clone expressing the polypeptide library member or by use of any selection display system. As described above, a preferred selection display system uses bacteriophage display. Thus, phage or phagemid vectors can be used. Preferred vectors are phagemid vectors, which have an *E. coli* origin of replication (for double stranded replication) and also a phage origin of replication (for production of single-stranded DNA). The manipulation and expression of such vectors is well known in the art (Hoogenboom and Winter (1992) *supra;* Nissim *et al.* (1994) *supra*). Briefly, the vector contains a β-lactamase or other selectable marker gene to confer selectivity on the phagemid, and a lac promoter upstream of a expression cassette that consists (N to C terminal) of a leader sequence (which directs the expressed polypeptide to the periplasmic space), a multiple cloning site (for cloning the nucleotide version of the library member), optionally, one or more peptide tags (for detection), optionally, one or more TAG stop codons and the phage protein pIII. Leader sequences, which may be used in bacterial expression and/or phage or phagemid display, include pelB, stII, ompA, phoA, bla, and pelA. Using various suppressor and non-suppressor strains of *E. coli* and with the addition of glucose, isopropyl thio-β-D-galactoside (IPTG) or a helper phage, such as VCS M13, the vector is able to replicate as a plasmid with no expression, produce large quantities of the polypeptide library member only, or produce phage, some of which contain at least one copy of the polypeptide-pIII fusion on their surface.

An example of a preferred vector is the pHEN1 phagemid vector (Hoogenboom et al., 1991, Nucl. Acids Res. 19: 4133-4137; sequence is available, e.g., as SEQ ID NO: 7 in WO 03/031611), in which the production of pIII fusion protein is under the control of the LacZ promoter, which is inhibited in the presence of glucose and induced with IPTG. When grown in suppressor strains of B. coli, e.g., TG1, the gene III fusion protein is produced and packaged into phage, while growth in non-suppressor strains, e.g., HB2151, permits the secretion of soluble fusion protein into the bacterial periplasm and into the culture medium. Because the expression of gene III prevents later infection with helper phage, the bacteria harboring the phagemid vectors are propagated in the presence of glucose before infection with VCSM13 helper phage for phage rescue.

Construction of vectors according to the invention employs conventional ligation techniques. Isolated vectors or DNA fragments are cleaved, tailored, and re-ligated in the form desired to generate the required vector. If desired, sequence analysis to confirm that the correct sequences are present in the constructed vector is performed using standard methods. Suitable methods for constructing expression vectors, preparing *in vitro* transcripts, introducing DNA into host cells, and performing analyses for assessing expression and function are known to those skilled in the art. The presence of a gene sequence in a sample is detected, or its amplification and/or expression quantified by conventional methods, such as Southern or Northern analysis, Western blotting, dot blotting of DNA, RNA or protein, *in situ* hybridization, immunocytochemistry or sequence analysis of nucleic acid or protein molecules. Those skilled in the art will readily envisage how these methods may be modified, if desired.

Scaffolds for use in Constructing Antibody Single Variable Domains

i. Selection of the main-chain conformation

The members of the immunoglobulin superfamily all share a similar fold for their polypeptide chain. For example, although antibodies are highly diverse in terms of their primary sequence, comparison of sequences and crystallographic structures has revealed that, contrary to expectation, five of the six antigen binding loops of antibodies (H1, H2, L1, L2, L3) adopt a limited number of main-chain conformations, or canonical structures (Chothia and Lesk (1987) J. Mol. Biol., 196: 901; Chothia et al. (1989) Nature, 342: 877). Analysis of loop lengths and key residues has therefore enabled prediction of the main-chain conformations of H1, H2, L1, L2 and L3 found in the majority of human antibodies (Chothia et al. (1992).J. Mol. Biol., 227: 799; Tomlinson et al. (1995) EMBO J., 14: 4628; Williams et al. (1996).J. Mol. Biol., 264: 220). Although the H3 region is much more diverse in terms of sequence, length and structure (due to the use of D segments), it also forms a limited number of main-chain conformations for short loop lengths which depend on the length and the presence of particular residues, or types of residue, at key positions in the loop and the antibody framework (Martin et al. (1996) J. Mol. Biol., 263: 800; Shirai et al. (1996) FEBS Letters, 399: 1).

The PEG-linked antibody single variable domain monomers and multimers of the present invention are advantageously assembled from libraries of domains, such as libraries of $V_H$ domains and/or libraries of $V_L$ domains. Moreover, the PEG-linked dAbs of the invention may themselves be provided in the form of libraries. In one aspect of the present invention, libraries of antibody single variable domains are designed in which certain loop lengths and key residues have been chosen to ensure that the main-chain conformation of the members is known. Advantageously, these are real conformations of immunoglobulin superfamily molecules found in nature, to minimize the chances that they are non-functional, as discussed above. Germline V gene segments serve as one suitable basic framework for constructing antibody or T-cell receptor libraries; other sequences are also of use. Variations may occur at a low frequency, such that a small number of functional members may possess an altered main-chain conformation, which does not affect its function.

Canonical structure theory is also of use to assess the number of different main-chain conformations encoded by ligands, to predict the main-chain conformation based on ligand sequences and to chose residues for diversification which do not affect the canonical structure. It is known that, in the human $V_\kappa$ domain, the L1 loop can adopt one of four canonical structures, the L2 loop has a single canonical structure and that 90% of human $V_\kappa$ domains adopt one of four or five canonical structures for the L3 loop (Tomlinson *et al.* (1995) supra); thus, in the $V_\kappa$ domain alone, different canonical structures can combine to create a range of different main-chain conformations. Given that the $V_\lambda$ domain encodes a different range of canonical structures for the L1, L2 and L3 loops and that $V_\kappa$ and $V_\lambda$ domains can pair with any $V_H$ domain which can encode several canonical structures for the H1 and H2 loops, the number of canonical structure combinations observed for these five loops is very large. This implies that the generation of diversity in the main-chain conformation may be essential for the production of a wide range of binding specificities. However, by constructing an antibody library based on a single known main-chain conformation it has been found, contrary to expectation, that diversity in the main-chain conformation is not required to generate sufficient diversity to target substantially all antigens. Even more surprisingly, the single main-chain conformation need not be a consensus structure - a single naturally occurring conformation can be used as the basis for an entire library. Thus, in one aspect, the polymer-linked antibody single variable domains of the invention possess a single known main-chain conformation.

The single main-chain conformation that is chosen is preferably commonplace among molecules of the immunoglobulin superfamily type in question. A conformation is commonplace when a significant number of naturally occurring molecules are observed to adopt it. Accordingly, in a preferred aspect of the invention, the natural occurrence of the different main-chain conformations for each binding loop of an immunoglobulin domain are considered separately and then a naturally occurring variable domain is chosen which possesses the desired combination of main-chain conformations for the different loops. If none is available, the nearest equivalent may be chosen. It is preferable that the desired combination of main-chain conformations for the different loops is created by selecting germline gene segments which encode the desired main-chain conformations. It is more preferable, that the selected germline gene segments are frequently expressed in nature, and most preferable that they are the most frequently expressed of all natural germline gene segments.

In designing antibody single variable domains or libraries thereof the incidence of the different main-chain conformations for each of the six antigen binding loops may be considered separately. For H1, H2, L1, L2 and L3, a given conformation that is adopted by between 20% and 100% of the antigen binding loops of naturally occurring molecules is chosen. Typically, its observed incidence is above 35% (i.e. between 35% and 100%) and, ideally, above 50% or even above 65%. Since the vast majority of H3 loops do not have canonical structures, it is preferable to select a main-chain conformation which is commonplace among those loops which do display canonical structures. For each of the loops, the conformation which is observed most often in the natural repertoire is therefore selected. In human antibodies, the most popular canonical structures (CS) for each loop are as follows: H1- CS 1 (79% of the expressed

repertoire), H2 - CS 3 (46%), L1 - CS 2 of $V_\kappa$ (39%), L2 - CS 1 (100%), L3 - CS 1 of $V_\kappa$ (36%) (calculation assumes a $\kappa:\lambda$ ratio of 70:30, Hood et al. (1967) Cold Spring Harbor Symp. Quant. Biol., 48: 133). For H3 loops that have canonical structures, a CDR3 length (Kabat et al. (1991) Sequences of proteins of immunological interest, U.S. Department of Health and Human Services) of seven residues with a salt-bridge from residue 94 to residue 101 appears to be the most common. There are at least 16 human antibody sequences in the EMBL data library with the required H3 length and key residues to form this conformation and at least two crystallographic structures in the protein data bank which can be used as a basis for antibody modeling (2cgr and Itet). The most frequently expressed germline gene segments that this combination of canonical structures are the $V_H$ segment 3-23 (DP-47), the $J_H$ segment JH4b, the $V_\kappa$ segment O2/O12 (DPK9) and the $J_\kappa$ segment $J_\kappa$1. $V_H$ segments DP45 and DP38 are also suitable. These segments can therefore be used in combination as a basis to construct a library with the desired single main-chain conformation.

Alternatively, instead of choosing the single main-chain conformation based on the natural occurrence of the different main-chain conformations for each of the binding loops in isolation, the natural occurrence of combinations of main-chain conformations is used as the basis for choosing the single main-chain conformation. In the case of antibodies, for example, the natural occurrence of canonical structure combinations for any two, three, four, five or for all six of the antigen binding loops can be determined. Here, it is preferable that the chosen conformation is commonplace in naturally occurring antibodies and most preferable that it observed most frequently in the natural repertoire. Thus, in human antibodies, for example, when natural combinations of the five antigen binding loops, H1, H2, L1, L2 and L3, are considered, the most frequent combination of canonical structures is determined and then combined with the most popular conformation for the H3 loop, as a basis for choosing the single main-chain conformation.

Diversification of the canonical sequence

Having selected several known main-chain conformations or, preferably a single known main-chain conformation, antibody single variable domains according to the invention or libraries for use in the invention can be constructed by varying the binding site of the molecule in order to generate a repertoire with structural and/or functional diversity. This means that variants are generated such that they possess sufficient diversity in their structure and/or in their function so that they are capable of providing a range of activities.

The desired diversity is typically generated by varying the selected molecule at one or more positions. The positions to be changed can be chosen at random or are preferably selected. The variation can then be achieved either by randomization, during which the resident amino acid is replaced by any amino acid or analogue thereof, natural or synthetic, producing a very large number of variants or by replacing the resident amino acid with one or more of a defined subset of amino acids, producing a more limited number of variants.

Various methods have been reported for introducing such diversity. Error-prone PCR (Hawkins et al. (1992) J. Mol. Biol., 226: 889), chemical mutagenesis (Deng et al. (1994) J. Biol. Chem., 269: 9533) or bacterial mutator strains (Low et al. (1996) J Mol. Biol., 260: 359) can be used to introduce random mutations into the genes that encode the molecule. Methods for mutating selected positions are also well known in the art and include the use of mismatched oligonucleotides or degenerate oligonucleotides, with or without the use of PCR. For example, several synthetic antibody libraries have been created by targeting mutations to the antigen binding loops. The H3 region of a human tetanus toxoid-binding Fab has been randomised to create a range of new binding specificities (Barbas et al. (1992) Proc. Natl. Acad. Sci. USA, 89: 4457). Random or semi-random H3 and L3 regions have been appended to germline V gene segments to produce large libraries with unmutated framework regions (Hoogenboom & Winter (1992) J. Mol. Biol., 227: 381; Barbas et al. (1992) Proc. Natl. Acad. Sci. USA, 89: 4457; Nissim et al. (1994) EMBO J., 13: 692; Griffiths et al. (1994) EMBO J., 13: 3245; De Kruif et al. (1995) J. Mol. Biol., 248: 97). Such diversification has been extended to include some or all of the other antigen binding loops (Crameri et al. (1996) Nature Med., 2: 100; Riechmann et al. (1995) Bio/Technology, 13: 475; Morphosys, WO97/08320, supra).

Since loop randomization has the potential to create approximately more than $10^{15}$ structures for H3 alone and a similarly large number of variants for the other five loops, it is not feasible using current transformation technology or even by using cell free systems to produce a library representing all possible combinations. For example, in one of the largest libraries constructed to date, $6 \times 10^{10}$ different antibodies, which is only a fraction of the potential diversity for a library of this design, were generated (Griffiths *et al.* (1994) supra).

In a preferred embodiment, only those residues which are directly involved in creating or modifying the desired function of the molecule are diversified. For many molecules, the function will be to bind a target and therefore diversity should be concentrated in the target binding site, while avoiding changing residues which are crucial to the overall packing of the molecule or to maintaining the chosen main-chain conformation.

Diversification of the canonical sequences as it applies to antibody domains

In the case of antibody single variable domains, the binding site for the target is most often the antigen binding

site. Thus, in a highly preferred aspect, the invention provides libraries of or for the assembly of antibody single variable domains in which only those residues in the antigen binding site are varied. These residues are extremely diverse in the human antibody repertoire and are known to make contacts in high-resolution antibody/antigen complexes. For example, in L2 it is known that positions 50 and 53 are diverse in naturally occurring antibodies and are observed to make contact with the antigen. In contrast, the conventional approach would have been to diversify all the residues in the corresponding Complementarity Determining Region (CDR1) as defined by Kabat *et al.* (1991, supra), some seven residues compared to the two diversified in the library for use according to the invention. This represents a significant improvement in terms of the functional diversity required to create a range of antigen binding specificities.

In nature, antibody diversity is the result of two processes: somatic recombination of germline V, D and J gene segments to create a naive primary repertoire (so called germline and junctional diversity) and somatic hypermutation of the resulting rearranged V genes. Analysis of human antibody sequences has shown that diversity in the primary repertoire is focused at the center of the antigen binding site whereas somatic hypermutation spreads diversity to regions at the periphery of the antigen binding site that are highly conserved in the primary repertoire (see Tomlinson et al. (1996) J. Mol. Biol., 256: 813). This complementarity has probably evolved as an efficient strategy for searching sequence space and, although apparently unique to antibodies, it can easily be applied to other polypeptide repertoires. The residues which are varied are a subset of those that form the binding site for the target. Different (including overlapping) subsets of residues in the target binding site are diversified at different stages during selection, if desired.

In the case of an antibody repertoire, an initial 'naive' repertoire is created where some, but not all, of the residues in the antigen binding site are diversified. As used herein in this context, the term "naive" refers to antibody molecules that have no pre-determined target. These molecules resemble those which are encoded by the immunoglobulin genes of an individual who has not undergone immune diversification, as is the case with fetal and newborn individuals, whose immune systems have not yet been challenged by a wide variety of antigenic stimuli. This repertoire is then selected against a range of antigens or epitopes. If required, further diversity can then be introduced outside the region diversified in the initial repertoire. This matured repertoire can be selected for modified function, specificity or affinity.

In the construction of libraries for use in the invention, diversification of chosen positions is typically achieved at the nucleic acid level, by altering the coding sequence which specifies the sequence of the polypeptide such that a number of possible amino acids (all 20 or a subset thereof) can be incorporated at that position. Using the IUPAC nomenclature, the most versatile codon is NNK, which encodes all amino acids as well as the TAG stop codon. The NNK codon is preferably used in order to introduce the required diversity. Other codons which achieve the same ends are also of use, including the NNN codon, which leads to the production of the additional stop codons TGA and TAA.

A feature of side-chain diversity in the antigen binding site of human antibodies is a pronounced bias which favors certain amino acid residues. If the amino acid composition of the ten most diverse positions in each of the $V_H$, $V_\kappa$ and $V_\lambda$ regions are summed, more than 76% of the side-chain diversity comes from only seven different residues, these being, serine (24%), tyrosine (14%), asparagine (11%), glycine (9%), alanine (7%), aspartate (6%) and threonine (6%). This bias towards hydrophilic residues and small residues which can provide main-chain flexibility probably reflects the evolution of surfaces which are predisposed to binding a wide range of antigens or epitopes and may help to explain the required promiscuity of antibodies in the primary repertoire.

Since it is preferable to mimic this distribution of amino acids, the distribution of amino acids at the positions to be varied preferably mimics that seen in the antigen binding site of antibodies. Such bias in the substitution of amino acids that permits selection of certain polypeptides (not just antibody polypeptides) against a range of target antigens is easily applied to any polypeptide repertoire. There are various methods for biasing the amino acid distribution at the position to be varied (including the use of tri-nucleotide mutagenesis, see WO97/08320), of which the preferred method, due to ease of synthesis, is the use of conventional degenerate codons. By comparing the amino acid profile encoded by all combinations of degenerate codons (with single, double, triple and quadruple degeneracy in equal ratios at each position) with the natural amino acid use it is possible to calculate the most representative codon. The codons (AGT)(AGC)T, (AGT)(AGC)C and (AGT)(AGC)(CT) - that is, DVT, DVC and DVY, respectively using IUPAC nomenclature - are those closest to the desired amino acid profile: they encode 22% serine and 11% tyrosine, asparagine, glycine, alanine, aspartate, threonine and cysteine. Preferably, therefore, libraries

are constructed using either the DVT, DVC or DVY codon at each of the diversified positions.

PCR Mutagenesis:

The primer is complementary to a portion of a target molecule present in a pool of nucleic acid molecules used in the preparation of sets of nucleic acid repertoire members encoding polypeptide repertoire members. Most often, primers are prepared by synthetic methods, either chemical or enzymatic. Mutagenic oligonucleotide primers are generally 15 to 100 nucleotides in length, ideally from 20 to 40 nucleotides, although oligonucleotides of different length are of use.

Typically, selective hybridization occurs when two nucleic acid sequences are substantially complementary (at

least about 65% complementary over a stretch of at least 14 to 25 nucleotides, preferably at least about 75%, more preferably at least about 85% or 90% complementary). See Kanehisa, 1984, Nucleic Acids Res. 12: 203, incorporated herein by reference. As a result, it is expected that a certain degree of mismatch at the priming site is tolerated. Such mismatch may be small, such as a mono-, di- or tri-nucleotide. Alternatively, it may comprise nucleotide loops, which are defined herein as regions in which mismatch encompasses an uninterrupted series of four or more nucleotides.

Overall, five factors influence the efficiency and selectivity of hybridization of the primer to a second nucleic acid molecule. These factors, which are (i) primer length, (ii) the nucleotide sequence and/or composition, (iii) hybridization temperature, (iv) buffer chemistry and (v) the potential for steric hindrance in the region to which the primer is required to hybridize, are important considerations when non-random priming sequences are designed.

There is a positive correlation between primer length and both the efficiency and accuracy with which a primer will anneal to a target sequence; longer sequences have a higher melting temperature ($T_M$) than do shorter ones, and are less likely to be repeated within a given target sequence, thereby minimizing promiscuous hybridization. Primer sequences with a high G-C content or that comprise palindromic sequences tend to self-hybridize, as do their intended target sites, since unimolecular, rather than bimolecular, hybridization kinetics are generally favored in solution; at the same time, it is important to design a primer containing sufficient numbers of G-C nucleotide pairings to bind the target sequence tightly, since each such pair is bound by three hydrogen bonds, rather than the two that are found when A and T bases pair. Hybridization temperature varies inversely with primer annealing efficiency, as does the concentration of organic solvents, *e.g.* formamide, that might be included in a hybridization mixture, while increases in salt concentration facilitate binding. Under stringent hybridization conditions, longer probes hybridize more efficiently than do shorter ones, which are sufficient under more permissive conditions. Stringent hybridization conditions for primers typically include salt concentrations of less than about 1M, more usually less than about 500 mM and preferably less than about 200 mM. Hybridization temperatures range from as low as 0˚C to greater than 22˚C, greater than about 30˚C, and (most often) in excess of about 37˚C. Longer fragments may require higher hybridization temperatures for specific hybridization. As several factors affect the stringency of hybridization, the combination of parameters is more important than the absolute measure of any one alone.

Primers are designed with these considerations in mind. While estimates of the relative merits of numerous sequences may be made mentally by one of skill in the art, computer programs have been designed to assist in the evaluation of these several parameters and the optimization of primer sequences. Examples of such programs are "PrimerSelect" of the DNAStar™ software package (DNAStar, Inc.; Madison, WI) and OLIGO 4.0 (National Biosciences, Inc.). Once designed, suitable oligonucleotides are prepared by a suitable method, *e.g.* the phosphoramidite method described by Beaucage and Carruthers, 1981, Tetrahedron Lett. 22: 1859) or the triester method according to Matteucci and Caruthers, 1981, J. Am. Chem. Soc. 103: 3185, both incorporated herein by reference, or by other chemical methods using either a commercial automated oligonucleotide synthesizer or, for example, VLSIPS™ technology.

PCR is performed using template DNA (at least 1fg; more usefully, 1-1000 ng) and at least 25 pmol of oligonucleotide primers; it maybe advantageous to use a larger amount of primer when the primer pool is heavily heterogeneous, as each sequence is represented by only a small fraction of the molecules of the pool, and amounts become limiting in the later amplification cycles. A typical reaction mixture includes: 2 μl of DNA, 25 pmol of oligonucleotide primer, 2.5 μl of 10X PCR buffer 1 (Perkin-Elmer), 0.4 μl of 1.25 μM dNTP, 0.15 μl (or 2.5 units) of Taq DNA polymerase (Perkin Elmer) and deionized water to a total volume of 25 μl. Mineral oil is overlaid and the PCR is performed using a programmable thermal cycler.

The length and temperature of each step of a PCR cycle, as well as the number of cycles, is adjusted in accordance to the stringency requirements in effect. Annealing temperature and timing are determined both by the efficiency with which a primer is expected to anneal to a template and the degree of mismatch that is to be tolerated; obviously, when nucleic acid molecules are simultaneously amplified and mutagenized, mismatch is required, at least in the first round of synthesis. In attempting to amplify a population of molecules using a mixed pool of mutagenic primers, the loss, under stringent (high-temperature) annealing conditions, of potential mutant products that would only result from low melting temperatures is weighed against the promiscuous annealing of primers to sequences other than the target site. The ability to optimize the stringency of primer annealing conditions is well within the knowledge of one of skill in the art. An annealing temperature of between 30˚C and 72˚C is used. Initial denaturation of the template molecules normally occurs at between 92˚C and 99˚C for 4 minutes, followed by 20-40 cycles consisting of denaturation (94-99˚C for 15 seconds to 1 minute), annealing (temperature determined as discussed above; 1-2 minutes), and extension (72˚C for 1-5 minutes, depending on the length of the amplified product). Final extension is generally for 4 minutes at 72˚C, and may be followed by an indefinite (0-24 hour) step at 4˚C.

## Screening Single Immunoglobulin Variable Domains for Antigen Binding

Following expression of a repertoire of single immunoglobulin variable domains on the surface of phage, selection is performed by contacting the phage repertoire with immobilized target antigen, washing to remove unbound

phage, and propagation of the bound phage, the whole process frequently referred to as "panning." Alternatively, phage are preselected for the expression of properly folded member variants by panning against an immobilized generic ligand (e.g., protein A or protein L) that is only bound by folded members. This has the advantage of reducing the proportion of non-functional members, thereby increasing the proportion of members likely to bind a target antigen. Pre-selection with generic ligands is taught in WO 99/20749. The screening of phage antibody libraries is generally described, for example, by Harrison et al., 1996, Meth. Enzymol. 267: 83-109.

Screening is commonly performed using purified antigen immobilized on a solid support, for example, plastic tubes or wells, or on a chromatography matrix, for example Sepharose™ (Pharmacia). Screening or selection can also be performed on complex antigens, such as the surface of cells (Marks et al., 1993, BioTechnology 11:1145; de Kruif et al., 1995, Proc. Natl. Acad. Sci. U.S.A. 92: 3938). Another alternative involves selection by binding biotinylated antigen in solution, followed by capture on streptavidin-coated beads.

In a preferred aspect, panning is performed by immobilizing antigen (generic or specific) on tubes or wells in a plate, e.g., Nunc MAXISORP™ immunotube 8 well strips. Wells are coated with 150 μl of antigen (100 μg/ml in PBS) and incubated overnight. The wells are then washed 3 times with PBS and blocked with 400 μl PBS-2% skim milk (2%MPBS) at 37°C for 2 hr. The wells are rinsed 3 times with PBS and phage are added in 2%MPBS. The mixture is incubated at room temperature for 90 minutes and the liquid, containing unbound phage, is removed. Wells are rinsed 10 times with PBS-0.1% tween 20, and then 10 times with PBS to remove detergent. Bound phage are eluted by adding 200 μl of freshly prepared 100 mM triethylamine, mixing well and incubating for 10 mm at room temperature. Eluted phage are transferred to a tube containing 100 μl of 1M Tris-HCl, pH 7.4 and vortexed to neutralize the triethylamine. Exponentially-growing E. coli host cells (e.g., TG1) are infected with, for example, 150 ml of the eluted phage by incubating for 30 min at 37°C. Infected cells are spun down, resuspended in fresh medium and plated in top agarose. Phage plaques are eluted or picked into fresh cultures of host cells to propagate for analysis or for further rounds of selection. One or more rounds of plaque purification are performed if necessary to ensure pure populations of selected phage. Other screening approaches are described by Harrison et al., 1996, *supra.*

Following identification of phage expressing a single immunoglobulin variable domain that binds a desired target, if a phagemid vector such as pHEN1 has been used, the variable domain fusion protein are easily produced in soluble form by infecting non-suppressor strains of bacteria, e.g., HB2151 that permit the secretion of soluble gene III fusion protein. Alternatively, the V domain sequence can be sub-cloned into an appropriate expression vector to produce soluble protein according to methods known in the art.

Purification and Concentration of Single Immunoglobulin Variable Domains:

Single immunoglobulin variable domain polypeptides secreted into the periplasmic space or into the medium of bacteria are harvested and purified according to known methods (Harrison et al., 1996, supra). Skerra & Pluckthun (1988, Science 240: 1038) and Breitling et al. (1991, Gene 104: 147) describe the harvest of antibody polypeptides from the periplasm, and Better et al. (1988, Science 240: 1041) describes harvest from the culture supernatant. Purification can also be achieved by binding to generic ligands, such as protein A or Protein L. Alternatively, the variable domains can be expressed with a peptide tag, e.g., the Myc, HA or 6X-His tags, which facilitates purification by affinity chromatography.

Polypeptides are concentrated by several methods well known in the art, including, for example, ultrafiltration, diafiltration and tangential flow filtration. The process of ultrafiltration uses semi-permeable membranes and pressure to separate molecular species on the basis of size and shape. The pressure is provided by gas pressure or by centrifugation. Commercial ultrafiltration products are widely available, e.g., from Millipore (Bedford, MA; examples include the Centricon™ and Microcon™ concentrators) and Vivascience (Hannover, Germany; examples include the Vivaspin™ concentrators). By selection of a molecular weight cutoff smaller than the target polypeptide (usually 1/3 to 1/6 the molecular weight of the target polypeptide, although differences of as little as 10 kD can be used successfully), the polypeptide is retained when solvent and smaller solutes pass through the membrane. Thus, a molecular weight cutoff of about 5 kD is useful for concentration of single immunoglobulin variable domain polypeptides described herein.

Diafiltration, which uses ultrafiltration membranes with a "washing" process, is used where it is desired to remove or exchange the salt or buffer in a polypeptide preparation. The polypeptide is concentrated by the passage of solvent and small solutes through the membrane, and remaining salts or buffer are removed by dilution of the retained polypeptide with a new buffer or salt solution or water, as desired, accompanied by continued ultrafiltration. In continuous diafiltration, new buffer is added at the same rate that filtrate passes through the membrane. A diafiltration volume is the volume of polypeptide solution prior to the start of diafiltration - using continuous diafiltration, greater than 99.5% of a fully permeable solute can be removed by washing through six diafiltration volumes with the new buffer. Alternatively, the process can be performed in a discontinuous manner, wherein the sample is repeatedly diluted and then filtered back to its original volume to remove or exchange salt or buffer and ultimately concentrate the polypeptide. Equipment for diafiltration and detailed methodologies for its use are available, for example, from Pall Life Sciences (Ann Arbor,

MI) and Sartorius AG/Vivascience (Hannover, Germany).

Tangential flow filtration (TFF), also known as "cross-flow filtration," also uses ultrafiltration membrane. Fluid containing the target polypeptide is pumped tangentially along the surface of the membrane. The pressure causes a portion of the fluid to pass through the membrane while the target polypeptide is retained above the filter. In contrast to standard ultrafiltration, however, the retained molecules do not accumulate on the surface of the membrane, but are carried along by the tangential flow. The solution that does not pass through the filter (containing the target polypeptide) can be repeatedly circulated across the membrane to achieve the desired degree of concentration. Equipment for TFF and detailed methodologies for its use are available, for example, from Millipore (e.g., the ProFlux M12™ Benchtop TFF system and the Pellicon™ systems), Pall Life Sciences (e.g., the Minim™ Tangential Flow Filtration system).

Protein concentration is measured in a number of ways that are well known in the art. These include, for example, amino acid analysis, absorbance at 280 nm, the "Bradford" and "Lowry" methods, and SDS-PAGE. The most accurate method is total hydrolysis followed by amino acid analysis by HPLC, concentration is then determined then comparison with the known sequence of the single immunoglobulin variable domain polypeptide. While this method is the most accurate, it is expensive and time-consuming. Protein determination by measurement of UV absorbance at 280 nm faster and much less expensive, yet relatively accurate and is preferred as a compromise over amino acid analysis. Absorbance at 280 nm was used to determine protein concentrations reported in the Examples described herein.

"Bradford" and "Lowry" protein assays (Bradford, 1976, Anal. Biochem. 72: 248-254; Lowry et al., 1951, J. Biol. Chem. 193: 265-275) compare sample protein concentration to a standard curve most often based on bovine serum albumin (BSA). These methods are less accurate, tending to underestimate the concentration of single immunoglobulin variable domains. Their accuracy could be improved, however, by using a $V_H$ or $V_\kappa$ single domain polypeptide as a standard.

An additional protein assay method is the bicinchoninic acid assay described in U.S. Patent No. 4,839,295 (incorporated herein by reference) and marketed by Pierce Biotechnology (Rockford, IL) as the "BCA Protein Assay" (e.g., Pierce Catalog No. 23227).

The SDS-PAGE method uses gel electrophoresis and Coomassie Blue staining in comparison to known concentration standards, e.g., known amounts of a single immunoglobulin variable domain polypeptide. Quantitation can be done by eye or by densitometry.

Single human immunoglobulin variable domain antigen-binding polypeptides described herein retain solubility at high concentration (e.g., at least 4.8 mg (~400 μM) in aqueous solution (e.g., PBS), and preferably at least 5 mg/ml (~417 μM), 10 mg/ml (~833 μM), 20 mg/ml (~1.7 mM), 25 mg/ml (~2.1 mM), 30 mg/ml (~2.5 mM), 35 mg/ml (~2.9 mM), 40 mg/ml (~3.3 mM), 45 mg/ml (~3.75 mM), 50 mg/ml (~4.2 mM), 55 mg/ml (~4.6 mM), 60 mg/ml (~5.0 mM), 65 mg/ml (~5.4 mM), 70 mg/ml (~5.8 mM), 75 mg/ml (~6.3 mM), 100 mg/ml (~8.33 mM), 150 mg/ml (~12.5 mM), 200 mg/ml (~16.7 mM), 240 mg/ml (~20 mM) or higher). One structural feature that promotes high solubility is the relatively small size of the single immunoglobulin variable domain polypeptides. A full length conventional four chain antibody, e.g., IgG is about 150 kD in size. In contrast, single immunoglobulin variable domains, which all have a general structure comprising 4 framework (FW) regions and 3 CDRs, have a size of approximately 12 kD, or less than 1/10 the size of a conventional antibody. Similarly, single immunoglobulin variable domains are approximately ½ the size of an scFv molecule (~26 kD), and approximately 1/5 the size of a Fab molecule (~60 kD). It is preferred that the size of a single immunoglobulin variable domain-containing structure disclosed herein is 100 kD or less, including structures of, for example, about 90 kD or less, 80 kD or less, 70 kD or less, 60 kD or less, 50 kD or less, 40 kD or less, 30 kD or less, 20 kD or less, down to and including about 12 kD, or a single immunoglobulin variable domain in isolation.

The solubility of a polypeptide is primarily determined by the interactions of the amino acid side chains with the surrounding solvent. Hydrophobic side chains tend to be localized internally as a polypeptide folds, away from the solvent-interacting surfaces of the polypeptide. Conversely, hydrophilic residues tend to be localized at the solvent-interacting surfaces of a polypeptide. Generally, polypeptides having a primary sequence that permits the molecule to fold to expose more hydrophilic residues to the aqueous environment are more soluble than one that folds to expose fewer hydrophilic residues to the surface. Thus, the arrangement and number of hydrophobic and hydrophilic residues is an important determinant of solubility. Other parameters that determine polypeptide solubility include solvent pH, temperature, and ionic strength. In a common practice, the solubility of polypeptides can be maintained or enhanced by the addition of glycerol (e.g., ~10% v/v) to the solution.

As discussed above, specific amino acid residues have been identified in conserved residues of human $V_H$ domains that vary in the $V_H$ domains of camelid species, which are generally more soluble than human $V_H$ domains. These include, for example, Gly 44 (Glu in camelids), Leu 45 (Arg in camelids) and Trp 47 (Gly in camelids). Amino acid residue 103 of $V_H$ is also implicated in solubility, with mutation from Trp to Arg tending to confer increased $V_H$ solubility.

In preferred aspects of the invention, single immunoglobulin variable domain polypeptides are based on the DP47 germline $V_H$ gene segment or the DPK9 germline $V_\kappa$ gene segment. Thus, these germline gene segments are capable, particularly when diversified at selected structural locations described herein, of producing specific binding single immunoglobulin variable domain polypeptides that are highly soluble. In particular, the four framework regions,

which are preferably not diversified, can contribute to the high solubility of the resulting proteins.

It is expected that a single human immunoglobulin variable domain that is highly homologous to one having a known high solubility will also tend to be highly soluble. Thus, as one means of prediction or recognition that a given single immunoglobulin variable domain would have the high solubility recited herein, one can compare the sequence of a single immunoglobulin variable domain polypeptide to one or more single immunoglobulin variable domain polypeptides having known solubility. Thus, when a single immunoglobulin variable domain polypeptide is identified that has high binding affinity but unknown solubility, comparison of its amino acid sequence with that of one or more (preferably more) human single immunoglobulin variable domain polypeptides known to have high solubility (e.g., a dAb sequence disclosed herein) can permit prediction of its solubility. While it is not an absolute predictor, where there is a high degree of similarity to a known highly soluble sequence, e.g., 90-95% or greater similarity, and particularly where there is a high degree of similarity with respect to hydrophilic amino acid residues, or residues likely to be exposed at the solvent interface, it is more likely that a newly identified binding polypeptide will have solubility similar to that of the known highly soluble sequence.

Molecular modeling software can also be used to predict the solubility of a polypeptide sequence relative to that of a polypeptide of known solubility. For example, the substitution or addition of a hydrophobic residue at the solvent-exposed surface, relative to a molecule of known solubility that has a less hydrophobic or even hydrophilic residue exposed in that position is expected to decrease the relative solubility of the polypeptide. Similarly, the substitution or addition of a more hydrophilic residue at such a location is expected to increase the relative solubility. That is, a change in the net number of hydrophilic or hydrophobic residues located at the surface of the molecule (or the overall hydrophobic or hydrophilic nature of the surface-exposed residues) relative to a single immunoglobulin variable domain polypeptide structure with known solubility can predict the relative solubility of a single immunoglobulin variable domain polypeptide.

Alternatively, or in conjunction with such prediction, one can determine limits of a single immunoglobulin variable domain polypeptide's solubility by simply concentrating the polypeptide.

Affinity/Activity Determination:

Isolated single human immunoglobulin variable domain-containing polypeptides as described herein have affinities (dissociation constant, $K_d$, = $K_{off}/K_{on}$) of at least 300 nM or less, and preferably at least 300 nM - 50 pM, 200 nM - 50 pM, and more preferably at least 100nM - 50 pM, 75 nM - 50 pM, 50 nM - 50 pM, 25 nM - 50 pM, 10 nM - 50 pM, 5 nM - 50 pM, 1 nM - 50 pM, 950 pM - 50 pM, 900 pM - 50 pM, 850 pM - 50 pM, 800 pM - 50 pM, 750 pM - 50 pM, 700 pM - 50 pM, 650 pM - 50 pM, 600 pM - 50 pM, 550 pM - 50 pM, 500 pM - 50 pM, 450 pM - 50 pM, 400 pM - 50 pM, 350 pM - 50 pM, 300 pM - 50 pM, 250 pM - 50 pM, 200 pM - 50 pM, 150 pM - 50 pM, 100 pM - 50 pM, 90 pM - 50 pM, 80 pM - 50 pM, 70 pM - 50 pM, 60 pM - 50 pM, or even as low as 50 pM.

The antigen-binding affinity of a variable domain polypeptide can be conveniently measured by surface plasmon resonance (SPR) using the BIAcore system (Pharmacia Biosensor, Piscataway, N.J.). In this method, antigen is coupled to the BIAcore chip at known concentrations, and variable domain polypeptides are introduced. Specific binding between the variable domain polypeptide and the immobilized antigen results in increased protein concentration on the chip matrix and a change in the SPR signal. Changes in SPR signal are recorded as resonance units (RU) and displayed with respect to time along the Y axis of a sensorgram. Baseline signal is taken with solvent alone (e.g., PBS) passing over the chip. The net difference between baseline signal and signal after completion of variable domain polypeptide injection represents the binding value of a given sample. To determine the off rate ($K_{off}$), on rate ($K_{on}$) and dissociation rate ($K_d$) constants, BIAcore kinetic evaluation software (e.g., version 2.1) is used.

High affinity is dependent upon the complementarity between a surface of the antigen and the CDRs of the antibody or antibody fragment. Complementarity is determined by the type and strength of the molecular interactions possible between portions of the target and the CDR, for example, the potential ionic interactions, van der Waals attractions, hydrogen bonding or other interactions that can occur. CDR3 tends to contribute more to antigen binding interactions than CDRs 1 and 2, probably due to its generally larger size, which provides more opportunity for favorable surface interactions. (See, e.g., Padlan et al., 1994, Mol. Immunol. 31: 169-217; Chothia & Lesk, 1987, J. Mol. Biol. 196: 904-917; and Chothia et al., 1985, J. Mol. Biol. 186: 651-663.) High affinity indicates single immunoglobulin variable domain/antigen pairings that have a high degree of complementarity, which is directly related to the structures of the variable domain and the target.

The structures conferring high affinity of a single immunoglobulin variable domain polypeptide for a given antigen can be highlighted using molecular modeling software that permits the docking of an antigen with the polypeptide structure. Generally, a computer model of the structure of a single immunoglobulin variable domain of known affinity can be docked with a computer model of a polypeptide or other target antigen of known structure to determine the interaction surfaces. Given the structure of the interaction surfaces for such a known interaction, one can then predict the impact, positive or negative, of conservative or less-conservative substitutions in the variable domain sequence on the strength of the interaction, thereby permitting the rational design of improved binding molecules.

Multimeric Forms of Antibody Single Variable Domains:

In one aspect, a single immunoglobulin variable domain as described herein is multimerized, as for example, hetero- or homodimers, hetero- or homotrimers, hetero- or homotetramers, or higher order hetero- or homomultimers (e.g., hetero- or homo-pentamer and up to octomers). Multimerization can increase the strength of antigen binding through the avidity effect, wherein the strength of binding is related to the sum of the binding affinities of the multiple binding sites.

Hetero- and Homomultimers are prepared through expression of single immunoglobulin variable domains fused, for example, through a peptide linker, leading to the configuration dAb-linker-dAb or a higher multiple of that arrangement. The multimers can also be linked to additional moieties, e.g., a polypeptide sequence that increases serum half-life or another effector moiety, e.g., a toxin or targeting moiety; e.g., PEG. Any linker peptide sequence can be used to generate hetero- or homomultimers, e.g., a linker sequence as would be used in the art to generate an scFv. One commonly useful linker comprises repeats of the peptide sequence $(Gly_4Ser)_n$, wherein n= 1 to about 10 (e.g., n=1, 2, 3, 4, 5, 6, 7, 8, 9, or 10). For example, the linker can be $(Gly_4Ser)_3$, $(Gly_4Ser)_5$, $(Gly_4Ser)_7$ or another multiple of the $(Gly_4Ser)$ sequence.

An alternative to the expression of multimers as monomers linked by peptide sequences is linkage of the monomeric single immunoglobulin variable domains post-translationally through, for example, disulfide bonding or other chemical linkage. For example, a free cysteine is engineered, e.g., at the C-terminus of the monomeric polypeptide, permits disulfide bonding between monomers. In this aspect or others requiring a free cysteine, the cysteine is introduced by including a cysteine codon (TGT, TGC) into a PCR primer adjacent to the last codon of the dAb sequence (for a C-terminal cysteine, the sequence in the primer will actually be the reverse complement, i.e., ACA or GCA, because it will be incorporated into the downstream PCR primer) and immediately before one or more stop codons. If desired, a linker peptide sequence, e.g., $(Gly_4Ser)_n$ is placed between the dAb sequence and the free cysteine. Expression of the monomers having a free cysteine residue results in a mixture of monomeric and dimeric forms in approximately a 1:1 mixture. Dimers are separated from monomers using gel chromatography, e.g., ion-exchange chromatography with salt gradient elution.

Alternatively, an engineered free cysteine is used to couple monomers through thiol linkages to a multivalent chemical linker, such as a trimeric maleimide molecule (e.g., Tris[2-maleimidoethyl]amine, TMEA) or a bi-maleimide PEG molecule (available from, for example, Nektar (Shearwater).

In one embodiment, a homodimer or heterodimer of the invention includes $V_H$ or $V_L$ domains which are covalently attached at a C-terminal amino acid to an immunoglobulin $C_H1$ domain or $C_\kappa$ domain, respectively. Thus the hetero- or homodimer may be a Fab-like molecule wherein the antigen binding domain contains associated $V_H$ and/or $V_L$ domains covalently linked at their C-termini to a $C_{H1}$ and $C_\kappa$ domain respectively. In addition, or alternatively, a dAb. multimer of the invention may be modeled on the camelid species which express a large proportion of fully functional, highly specific antibodies that are devoid of light chain sequences. The camelid heavy chain antibodies are found as homodimers of a single heavy chain, dimerized via their constant regions. The variable domains of these camelid heavy chain antibodies are referred to as $V_HH$ domains and retain the ability, when isolated as fragments of the $V_H$ chain, to bind antigen with high specificity ((Hamers-Casterman et al., 1993, Nature 363: 446-448; Gahroudi et al., 1997, FEBS Lett. 414: 521-526). Thus, an antibody single variable domain multimer of the invention may be constructed, using methods known in the art, and described above, to possess the $V_HH$ conformation of the camelid species heavy chain antibodies.

Target Antigens

Target antigens for antibody single variable domain polypeptides as described herein are human antigens related to a disease or disorder. That is, target antigens as described herein are therapeutically relevant targets. A "therapeutically relevant target" is one which, when bound by a single immunoglobulin variable domain or other antibody polypeptide that binds target antigen and acts as an antagonist or agonist of that target's activity, has a beneficial effect on the individual (preferably mammalian, preferably human) in which the target is bound. A "beneficial effect" is demonstrated by at least a 10% improvement in one or more clinical indicia of a disease or disorder, or, alternatively, where a prophylactic use of the single immunoglobulin variable domain polypeptide is desired, by an increase of at least 10% in the time before symptoms of the targeted disease or disorder are observed, relative to an individual not treated with the single immunoglobulin variable domain polypeptide preparation. Non-limiting examples of antigens that are suitable targets for single immunoglobulin variable domain polypeptides as described herein include cytokines, cytokine receptors, enzymes, enzyme co-factors, or DNA binding proteins. Suitable cytokines and growth factors include but are not limited to: ApoE, Apo-SAA, BDNF, Cardiotrophin-1, EGF, EGF receptor, ENA-78, Eotaxin, Eotaxin-2, Exodus-2, FGF-acidic, FGF-basic, fibroblast growth factor-10, FLT3 ligand, Fractalkine (CX3C), GDNF, G-CSF, GM-CSF, GF-$\beta$1, insulin, IFN-g, IGF-I, IGF-II, IL-1$\alpha$, IL-1$\beta$, IL-2, IL-3, IL-4, IL-5, IL-6, IL-7, IL-8 (72 a.a.), IL-8 (77 a.a.), IL-9, IL-10, IL-11, IL-12, IL-13, IL-15, IL-16, IL-17, IL-18 (IGIF), Inhibin $\alpha$, Inhibin $\beta$, IP-10, keratinocyte growth factor-2 (KGF-2), KGF, Leptin, LIF,

Lymphotactin, Mullerian inhibitory substance, monocyte colony inhibitory factor, monocyte attractant protein, M-CSF, MDC (67 a.a.), MDC (69 a.a.), MCP-1 (MCAF), MCP-2, MCP-3, MCP-4, MDC (67 a.a.), MDC (69 a.a.), MIG, MIP-la, MIP-1β, MIP-3α, Mix-3β, MIP-4, myeloid progenitor inhibitor factor-1 (MPIF-1), NAP-2, Neurturin, Nerve growth factor, β-NGF, NT-3, NT-4, Oncostatin M, PDGF-AA, PDGF-AB, PDGF-BB, PF-4, RANTES, SDF1α, SDF1β, SCF, SCGF, stem cell factor (SCF), TARC, TACE recognition site, TGF-α, TGF-β, TGF-β 2, TGF-β 3, tumor necrosis factor (TNF), TNF-α , TNF-β, TNF receptor I (p55), TNF receptor II, TNIL-1, TPO, VEGF, VEGF receptor 1, VEGF receptor 2, VEGF receptor 3, GCP-2, GRO/MGSA, GRO-β, GRO-γ, HCC1, 1-309, HER 1, HER 2, HER 3 and HER 4. Cytokine receptors include receptors for each of the foregoing cytokines, e.g., IL-1R, IL-6R, IL-10R, IL-18R, etc. It will be appreciated that this list is by no means exhaustive. Preferred targets for antigen single variable domain polypeptides according to the invention are disclosed in WO04/041867 (the contents of which are incorporated herein in their entirety) and include, but are not limited to TNFα, IgE, IFNγ, MMP-12, EGFR, CEA, H. pylori, TB, influenza, PDK-1, GSK1, Bad, caspase, Forkhead and VonWillebrand Factor (vWF). Targets may also be fragments of the above targets. Thus, a target is also a fragment of the above targets capable of eliciting an immune response. A target is also a fragment of the above targets, capable of binding to an antibody single variable domain polypeptide raised against the full length target.

In one aspect, a single immunoglobulin variable domain is linked to another single immunoglobulin variable domain to form a homodimer or heterodimer in which each individual domain is capable of binding its cognate antigen. Fusing single immunoglobulin variable domains as homodimers can increase the efficiency of target binding. e.g., through the avidity effect. Fusing single immunoglobulin variable domains as heterodimers, wherein each monomer binds a different target antigen, can produce a dual-specific ligand capable, for example, of bridging the respective target antigens. Such dual specific ligands may be used to target cytokines and other molecules which cooperate synergistically in therapeutic situations in the body of an organism. Thus, there is provided a method for synergising the activity of two or more cytokines, comprising administering a dual specific single immunoglobulin variable domain heterodimer capable of binding to the two or more cytokines. In this aspect, the dual specific ligand may be any dual specific ligand, including a ligand composed of complementary and/or non-complementary domains. For example, this aspect relates to combinations of $V_H$ domains and $V_L$ domains, $V_H$ domains only and $V_L$ domains only.

Preferably, the cytokines bound by the dual specific single immunoglobulin variable domain heterodimer of this aspect of the invention are selected from the following list:

| **Pairing** | **Evidence for therapeutic impact** |
|---|---|
| TNF/TGF-β | • TGF-β and TNF when injected into the ankle joint of mouse collagen induced arthritis model significantly enhanced joint inflammation. In non-collagen challenged mice there was no effect. |
| TNF/IL-1 | • TNF and IL-1 synergize in the pathology of uveitis.<br>• TNF and IL-1 synergize in the pathology of malaria (hypoglycaemia, NO).<br>• TNF and IL-1 synergize in the induction of polymorphonuclear (PMN) cells migration in inflammation.<br>• IL-1 and TNF synergize to induce PMN infiltration into the mouse peritoneum.<br>• IL-1 and TNF synergize to induce the secretion of IL-1 by endothelial cells. Important in inflammation.<br>• IL-1 or TNF alone induced some cellular infiltration into rabbit knee synovium. IL-1 induced PMNs, TNF - monocytes. Together they induced a more severe infiltration due to increased PMNs.<br>• Circulating myocardial depressant substance (present in sepsis) is low levels of IL-1 and TNF acting synergistically. |
| TNF/IL-2 | • References relating to synergisitic activation of killer T-cells. |
| TNF/IL-3 | • |
| TNF/IL-4 | • IL-4 and TNF synergize to induce VCAM expression on endothelial cells. Implied to have a role in asthma. Same for synovium - implicated in RA.<br>• TNF and IL-4 synergize to induce IL-6 expression in keratinocytes. |
| TNF/IL-6 | • |
| TNF/IL-8 | • TNF and IL-8 synergized with PMNs to activate platelets. Implicated in Acute Respiratory Distress Syndrome. |
| TNF/IL-10 | • IL-10 induces and synergizes with TNF in the induction of HIV expression in chronically infected T-cells. |

(continued)

| Pairing | Evidence for therapeutic impact |
|---|---|
| TNF/IL-12 | • |
| TNF/IFN-γ | • MHC induction in the brain.<br>• Synergize in anti-viral response/IFN-b induction.<br>• Neutrophil activation/ respiratory burst.<br>• Endothelial cell activation<br>• Toxicities noted when patients treated with TNF/IFN-γ as anti-viral therapy (will find out more).<br>• Fractalkine expression by human astrocytes.<br>• Many papers on inflammatory responses - i.e. LPS, also macrophage activation.<br>• Anti-TNF and anti-IFN-γ synergize to protect mice from lethal endotoxemia. |
| TGF-β/IL-1 | • Prostaglndin synthesis by osteoblasts |
| | • IL-6 production by intestinal epithelial cells (inflammation model)<br>• Stimulates IL-11 and IL-6 in lung fibroblasts (inflammation model)<br>• IL-6 and IL-8 production in the retina |
| TGF-β/IL-6 | • Chondrocarcoma proliferation |
| IL-1/IL-2 | • B-cell activation • LAK cell activation |
| | • T-cell activation |
| IL-1/IL-3 | • |
| IL-1/IL-4 | • B-cell activation<br>• IL-4 induces IL-1 expression in endothelial cell activation. |
| IL-1/IL-6<br><br><br><br><br>IL-1/IL-8 | • B cell activation<br>• T cell activation (can replace accessory cells)<br>• IL-1 induces IL-6 expression<br>• C3 and serum amyloid expression (acute phase response)<br>• HIV expression<br>• Cartilage collagen breakdown. • |
| IL-1/IL-10 | • |
| IL-1/IFN-g | • |
| IL-2/IL-3 | • T-cell proliferation<br>• B cell proliferation |
| IL-2/IL-4 | • B-cell proliferation • T-cell proliferation |
| IL-2/IL-5 | • B-cell proliferation/ Ig secretion<br>• IL-5 induces IL-2 receptors on B-cells |
| IL-2/IL-6 | • Development of cytotoxic T-cells |
| IL-2/IL-7 | • |
| IL-2/IL-10 | • B-cell activation |
| IL-2/IL-12 | • |
| IL-2/IL-15 | • |
| IL-2/IFN-γ | • Ig secretion by B-cells<br>• IL-2 induces IFN-g expression by T-cells |
| IL-2/IFN-α/β | • |
| IL-3/IL-4 | • Synergize in mast cell growth |
| IL-3/IL-5 | • |

(continued)

| Pairing | Evidence for therapeutic impact |
|---|---|
| IL-3/IL-6 | • |
| IL-3/IFN-y | • |
| IL-4/IL-5 | • Enhanced mast cell histamine etc. secretion in response to IgE |
| IL-4/IL-6 | • |
| IL-4/IL-10<br>IL-4/IL-12 | •<br>• |
| IL-4/IL-13 | • |
| IL-4/IFN-γ | • |
| IL-4/SCF | • Mast cell proliferation |
| IL-5/IL-6 | • |
| IL-5/IFN-γ | • |
| IL-6/IL-10 | • |
| IL-6/IL-11 | • |
| IL-6/IFN-γ | • |
| IL-10/IL-12 | • |
| IL-10/IFN-γ | • |
| IL-12/IL-18 | • |
| IL-12/IFN-γ | • IL-12 induces IFN-g expression by B and T-cells as part of immune stimulation. |
| IL-18/IFN-γ | • |
| Anti-TNF/anti-CD4 | • Synergistic therapeutic effect in DBA/1 arthritic mice. |

The amino acid and nucleotide sequences for the target antigens listed above and others are known and available to those of skill in the art. Standard methods of recombinant protein expression are used by one of skill in the art to express and purify these and other antigens where necessary, e.g., to pan for single immunoglobulin variable domains that bind the target antigen.

Functional Assays

In one embodiment, antibody single variable domains (and single domain multimers) as described herein have neutralizing activity (e.g., antagonizing activity) or agonizing activity towards their target antigens. The activity (whether neutralizing or agonizing) of a single immunoglobulin variable domain polypeptide as described herein is measured relative to the activity of the target antigen in the absence of the polypeptide in any accepted assay for such activity. For example, if the target antigen is an enzyme, an *in vivo* or *in vitro* functional assay that monitors the activity of that enzyme is used to monitor the activity or effect of an antibody single variable domain polypeptide.

Where, for example, the target antigen is a receptor, e.g., a cytokine receptor, activity is measured in terms of reduced or increased ligand binding to the receptor or in terms of reduced or increased signaling activity by the receptor in the presence of the single immunoglobulin variable domain polypeptide. Receptor signaling activity is measured by monitoring, for example, receptor conformation, co-factor or partner polypeptide binding, GDP for GTP exchange, a kinase, phosphatase or other enzymatic activity possessed by the activated receptor, or by monitoring a downstream result of such activity, such as expression of a gene (including a reporter gene) or other effect, including, for example, cell death, DNA replication, cell adhesion, or secretion of one or more molecules normally occurring as a result of receptor activation.

Where the target antigen is, for example, a cytokine or growth factor, activity is monitored by assaying binding of the cytokine to its receptor or by monitoring the activation of the receptor, e.g., by monitoring receptor signaling activity as discussed above. An example of a functional assay that measures a downstream effect of a cytokine is the L929 cell killing assay for TNF-α activity, which is well known in the art (see, for example, U.S. 6,090,382). The following L929

cytotoxicity assay is referred to herein as the "standard" L929 cytotoxicity assay. Anti-TNF single immunoglobulin variable domains ("anti-TNF dAbs") are tested for the ability to neutralize the cytotoxic activity of TNF on mouse L929 fibroblasts (Evans, T. (2000) Molecular Biotechnology 15, 243-248). Briefly, L929 cells plated in microtiter plates are incubated overnight with anti-TNF dAbs, 100pg/ml TNF and 1mg/ml actinomycin D (Sigma, Poole, UK). Cell viability is measured by reading absorbance at 490 nm following an incubation with [3-(4,5-dimethylthiazol-2-yl)-5-(3-carbboxymethoxyphenyl)-2-(4-sulfophenyl)-2H-tetrazolium (Promega, Madison, USA). Anti-TNF dAb activity leads to a decrease in TNF cytotoxicity and therefore an increase in absorbance compared with the TNF only control. A single immunoglobulin variable domain polypeptide described herein that is specific for TNF-α or TNF-α receptor has an $IC_{50}$ of 500 nM or less in this standard L929 cell assay, preferably 50 nM or less, 5 nM or less, 500 pM or less, 200 pM or less, 100 pM or less or even 50 pM.

Assays for the measurement of receptor binding by a ligand, e.g., a cytokine, are known in the art. As an example, anti-TNF dAbs can be tested for the ability to inhibit the binding of TNF to recombinant TNF receptor 1 (p55). Briefly, Maxisorp plates are incubated overnight with 30mg/ml anti-human Fc mouse monoclonal antibody (Zymed, San Francisco, USA). The wells are washed with phosphate buffered saline (PBS) containing 0.05% Tween-20 and then blocked with 1% BSA in PBS before being incubated with 100ng/ml TNF receptor 1 Fc fusion protein (R&D Systems, Minneapolis, USA). Anti-TNF dAb is mixed with TNF which is added to the washed wells at a final concentration of 10ng/ml. TNF binding is detected with 0.2mg/ml biotinylated anti-TNF antibody (HyCult biotechnology, Uben, Netherlands) followed by 1 in 500 dilution of horse radish peroxidase labeled streptavidin (Amersham Biosciences, UK) and incubation with TMB substrate (KPL, Gaithersburg, MD). The reaction is stopped by the addition of HCl and the absorbance is read at 450nm. Anti-TNF dAb inhibitory activity beads to a decrease in TNF binding and therefore to a decrease in absorbance compared with the TNF only control.

Assays for the measurement of receptor binding by a ligand, e.g., a cytokine, are known in the art. As an example, anti-TNF receptor I dAbs can be tested for the ability to inhibit the binding of TNF to recombinant TNF receptor 1 (p55). Briefly, Maxisorp plates are incubated overnight with 30mg/ml anti-human Fc mouse monoclonal antibody (Zymed, San Francisco, USA). The wells are washed with phosphate buffered saline (PBS) containing 0.05% Tween-20 and then blocked with 1% BSA in PBS before being incubated with 100ng/ml TNF receptor 1 Fc fusion protein (R&D Systems, Minneapolis, USA). Anti-TNF, receptor I dAb is incubated on the plate for 30 mins prior to the addition of TNF which is added to a final concentration of 3ng/ml and left to incubate for a further 60mins. The plate is washed to remove any unbound protein before the detection step. TNF binding is detected with 0.2mg/ml biotinylated anti-TNF antibody (HyCult biotechnology, Uben, Netherlands) followed by 1 in 500 dilution of horse radish peroxidase labeled streptavidin (Amersham Biosciences, UK) and incubation with TMB substrate (KPL, Gaithersburg, MD). The reaction is stopped by the addition of HC1 and the absorbance is read at 450nm. Anti-TNF dAb inhibitory activity beads to a decrease in TNF binding and therefore to a decrease in absorbance compared with the TNF only control.

As an alternative when evaluating the effect of a single immunoglobulin variable domain polypeptide on the p55 TNF-α receptor, the following HeLa cell assay based on the induction of IL-8 secretion by TNF in HeLa cells can be used (method is adapted from that of Alceson, L. et al (1996) Journal of Biological Chemistry 271, 30517-30523, describing the induction of IL-8 by IL-1 in HUVEC; here we look at induction by human TNF-α and we use HeLa cells instead of the HUVEC cell line). Briefly, HeLa cells plated in microtitre plates are incubated overnight with dAb and 300pg/ml TNF. Following incubation, the supernatant is aspirated off the cells and the IL-8 concentration is measured via a sandwich ELISA (R&D Systems). Anti-TNFR1 dAb activity leads to a decrease in IL-8 secretion into the supernatant compared with the TNF only control.

As an alternative when evaluating the effect of a single immunoglobulin variable domain polypeptide on the p55 TNF-α receptor, the following MRC-5 cell assay based on the induction of IL-8 secretion by TNF in MRC-5 cells can be used (method is adapted from that of Alceson, L. et al (1996) Journal of Biological Chemistry 271, 30517-30523, describing the induction of IL-8 by IL-1 in HUVEC; here we look at induction by human TNF-α and we use MRC-5 cells instead of the HUVEC cell line). Briefly, MRC-5 cells plated in microtitre plates are incubated overnight with dAb and 300pg/ml TNF. Following incubation, the supernatant is aspirated off the cells and the IL-8 concentration is measured via a sandwich ELISA (R&D Systems). Anti-TNFR1 dAb activity leads to a decrease in IL-8 secretion into the supernatant compared with the TNF only control.

Similar functional assays for the activity of other ligands (cytokines, growth factors, etc.) or their receptors are known to those of skill in the art and can be employed to evaluate the antagonistic or agonistic effect of single immunoglobulin variable domain polypeptides.

In one embodiment of the invention, the PEGylated dAb polypeptides (monomers and/or multimers) retain activity relative to non-PEGylated dAb monomers or multimers, wherein activity is measured as described above; that is, measured by affinity of the PEGylated dAb to a target molecule. A PEGylated dAb monomer or multimer of the invention will retain a level of activity (e.g., target affinity) which is at least 10% of the level of activity of a non-PEG-linked antibody single variable domain, preferably at least 20%, 30%, 40%, 50%, 60%, 70%, 80% and up to 90% or more of the activity of a non-PEG-linked antibody single variable domain, wherein activity is determined as described

above. In a preferred embodiment, a PEGylated dAb monomer or multimer retains at least 90% of the activity of a non-PEGylated dAb monomer or multimer, and still more preferably, retains all (100%) of the activity of a non-PEGylated dAb monomer or multimer.

Homologous sequences

The invention encompasses antibody single variable domain clones and clones with substantial sequence similarity or homology to them that also bind target antigen with high affinity and are soluble at high concentration (as well as such antibody single variable domain clones incorporated into multimers). As used herein, "substantial" sequence similarity or homology is at least 85% similarity or homology.

Calculations of "homology" or "sequence identity" between two sequences (the terms are used interchangeably herein) are performed as follows. The sequences are aligned for optimal comparison purposes (e.g., gaps can be introduced in one or both of a first and a second amino acid or nucleic acid sequence for optimal alignment and non-homologous sequences can be disregarded for comparison purposes). In a preferred embodiment, the length of a reference sequence aligned for comparison purposes is at least 30%, preferably at least 40%, more preferably at least 50%, even more preferably at least 60%, and even more preferably at least 70%, 80%, 90%, 100% of the length of the reference sequence. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein, amino acid or nucleic acid "homology" is equivalent to amino acid or nucleic acid "identity"). The percent identity between the two sequences is a function of the number of identical positions shared by the sequences, taking into account the number of gaps, and the length of each gap, which need to be introduced for optimal alignment of the two sequences.

As used herein, sequence "similarity" is a measure of the degree to which amino acid sequences share similar amino acid residues at corresponding positions in an alignment of the sequences. Amino acids are similar to each other where their side chains are similar. Specifically, "similarity" encompasses amino acids that are conservative substitutes for each other. A "conservative" substitution is any substitution that has a positive score in the blosum62 substitution matrix (Hentikoff and Hentikoff, 1992, Proc. Natl. Acad. Sci. USA 89:10915-10919). By the statement "sequence A is n% similar to sequence B" is meant that n% of the positions of an optimal global alignment between sequences A and B consists of identical amino acids or conservative substitutions. Optimal global alignments can be performed using the following parameters in the Needleman-Wunsch alignment algorithm:

For polypeptides:

Substitution matrix: biosum62.

Gap scoring function: -A -B*LG, where A=11 (the gap penalty), B=1 (the gap length penalty) and LG is the length of the gap.

For nucleotide sequences:

Substitution matrix: 10 for matches, 0 for mismatches.

Gap scoring function: -A -B*LG where A=50 (the gap penalty), B=3 (the gap length penalty) and LG is the length of the gap.

Typical conservative substitutions are among Met, Val, Leu and Ile; among Ser and Thr; among the residues Asp, Glu and Asn; among the residues Gin, Lys and Arg; or aromatic residues Phe and Tyr. In calculating the degree (most often as a percentage) of similarity between two polypeptide sequences, one considers the number of positions at which identity or similarity is observed between corresponding amino acid residues in the two polypeptide sequences in relation to the entire lengths of the two molecules being compared.

Alternatively, the BLAST (Basic Local Alignment Search Tool) algorithm is employed for sequence alignment, with parameters set to default values. The BLAST algorithm "BLAST 2 Sequences" is available at the world wide web site ("www") of the National Center for Biotechnology Information (".ncbi"), of the National Library of Medicine (".nlm") of the National Institutes of Health ("nih") of the U.S. government (".gov"), in the "/blast/" directory, sub-directories "bl2seq/bl2.html." This algorithm aligns two sequences for comparison and is described by Tatusova & Madden, 1999, FEMS Microbiol Lett. 174:247-250.

An additional measure of homology or similarity is the ability to hybridize under highly stringent hybridization conditions. Thus, a first sequence encoding a single immunoglobulin variable domain polypeptide is substantially similar to a second coding sequence if the first sequence hybridizes to the second sequence (or its complement) under highly stringent hybridization conditions (such as those described by Sambrook et al., Molecular Cloning, Laboratory Manual, Cold Spring, Harbor Laboratory Press, New York). "Highly stringent hybridization conditions" refer to hybridization in 6X

SSC at about 45°C, followed by one or more washes in 0.2X SSC, 0.1% SDS at 65°C. "Very highly stringent hybridization conditions" refer to hybridization in 0.5M sodium phosphate, 7% SDS at 65°C, followed by one or more washes at 0.2X SSC, 1% SDS at 65°C.

## PEGylation of dAbs

The present invention provides PEGylated dAb monomers and multimers which provide increased half-life and resistance to degradation without a loss in activity (e.g., binding affinity) relative to non-PEGylated dAbs.

dAb molecules of the invention may be coupled, using methods known in the art, to polymer molecules (preferably PEG) useful for achieving the increased half-life and degradation resistance properties encompassed by the present invention. Polymer moieties which may be utilized in the invention may be synthetic or naturally occurring and include, but are not limited to, straight or branched chain polyalkylene, polyalkenylene or polyoxyalkylene polymers, or a branched or unbranched polysaccharide such as a homo- or heteropolysaccharide. Preferred examples of synthetic polymers which may be used in the invention include straight or branched chain poly(ethylene glycol) (PEG), poly (propylene glycol), or poly(vinyl alcohol) and derivatives or substituted forms thereof. Particularly preferred substituted polymers useful in the invention include substituted PEG, including methoxy(polyethylene glycol). Naturally occurring polymer moieties which may be used according to the invention in addition to or in place of PEG include lactose, amylose, dextran, or glycogen, as well as derivatives thereof which would be recognized by one of skill in the art. Derivatized forms of polymer molecules of the invention include, for example, derivatives which have additional moieties or reactive groups present therein to permit interaction with amino acid residues of the dAb polypeptides described herein. Such derivatives include N-hydroxylsuccinimide (NHS) active esters, succinimidyl propionate polymers, and sulfhydryl-selective reactive agents such as maleimide, vinyl sulfone, and thiol. Particularly preferred derivatized polymers include, but are not limited to, PEG polymers having the formulae: PEG-O-$CH_2CH_2CH_2$-$CO_2$-NHS; PEG-O-$CH_2$-NHS; PEG-O-$CH_2CH_2$-$CO_2$-NHS; PEG-S-$CH_2CH_2$-CO-NHS; PEG-$O_2$CNH-CH(R)-$CO_2$-NHS; PEG-NHCO-$CH_2CH_2$-CO-NHS; and PEG-O-$CH_2$-$CO_2$-NHS where R is $(CH_2)_4$)NH$CO_2$(mPEG). PEG polymers useful in the invention may be linear molecules, or may be branched wherein multiple PEG moieties are present in a single polymer. Some particularly preferred PEG derivatives which are useful in the invention include, but are not limited to the following:

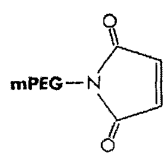

mPEG-MAL

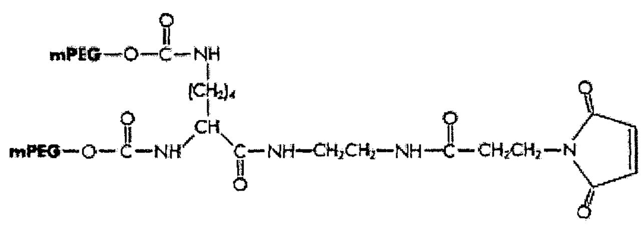

mPEG2-MAL

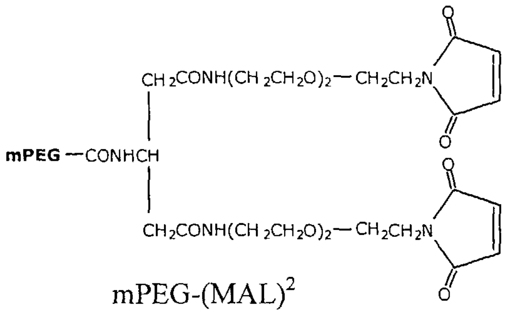

mPEG-(MAL)$^2$

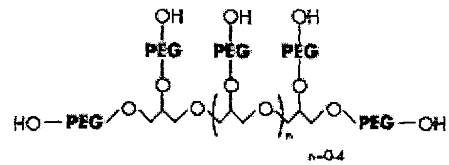

multi-arm PEG

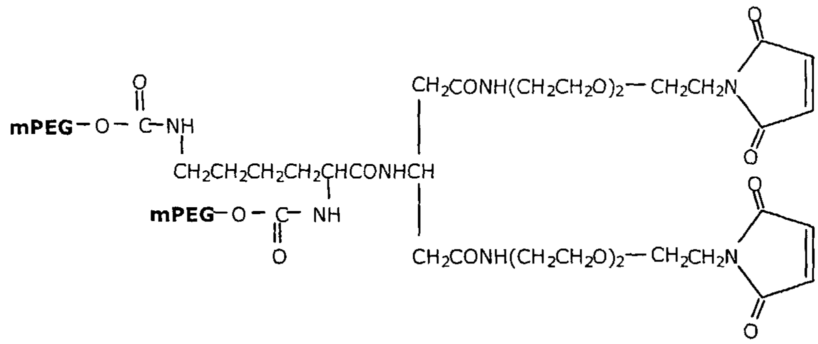

mPEG2-(MAL)$^2$

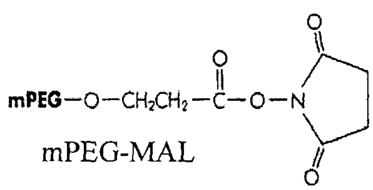

mPEG-MAL

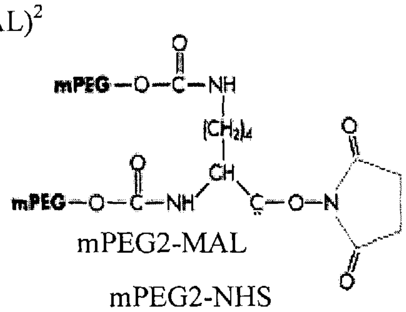

mPEG2-MAL

mPEG-SPA

mPEG2-NHS

The reactive group (e.g., MAL, NHS, SPA, VS, or Thiol) may be attached directly to the PEG polymer or may be attached to PEG via a linker molecule.

The size of polymers useful in the invention may be in the range of between 500 Da to 60 kDa, for example, between 1000 Da and 60 kDa, 10 kDa and 60 kDa, 20 kDa and 60 kDa, 30 kDa and 60 kDa, 40 kDa and 60 kDa, and up to between 50 kDa and 60 kDa. The polymers used in the invention, particularly PEG, may be straight chain polymers or may possess a branched conformation. Depending on the combination of molecular weight and conformation, the polymer molecules useful in the invention, when attached to a dAb monomer or multimer, will yield a molecule having an average hydrodynamic size of between 24 and 500 kDa. The hydrodynamic size of a polymer molecule used herein refers to the apparent size of a molecule (e.g., a protein molecule) based on the diffusion of the molecule through an aqueous solution. The diffusion, or motion of a protein through solution, can be processed to derive an apparent size of the protein, where the size is given by the Stokes radius or hydrodynamic radius of the protein particle. The "hydrodynamic size" of a protein depends on both mass and shape (conformation), such that two proteins having the same molecular mass may have differing hydrodynamic sizes based on the overall conformation of the protein. The hydrodynamic size of a PEG-linked antibody single variable domain (including antibody variable domain multimers as described herein) can be in the range of 24 kDa to 500 kDa; 30 to 500 kDa; 40 to 500 kDa; 50 to 500 kDa; 100 to 500 kDa; 150 to 500 kDa; 200 to 500 kDa; 250 to 500 kDa; 300 to 500 kDa; 350 to 500 kDa; 400 to 500 kDa and 450 to 500 kDa. Preferably the hydrodynamic size of a PEGylated dAb of the invention is 30 to 40 kDa; 70 to 80 kDa or 200 to 300 kDa.

Hydrodynamic size of the PEG-linked dAb monomers and multimers of the invention may be determined using methods which are well known in the art. For example, gel filtration may be used to determine the hydrodynamic size of a PEG- or non-PEG-linked dAb monomer or multimer, wherein the size of the dAb band on the gel is compared to a molecular weight standard. Other methods for the determination of hydrodynamic size include, but are not limited to, SDS-PAGE size exclusion chromatography columns such as, for example, Superose 12HR (Amersham Pharmacia, Piscataway, NJ). Other methods for determining the hydrodynamic size of a PEG- or non-PEG-linked dAb monomer or multimer of the invention will be readily appreciated by one of skill in the art. In one embodiment, hydrodynamic size of PEG- or other polymer-linked antibody single variable domain polypeptides of the invention may be determined using gel filtration matricies. The gel filtration matrices used to determine the hydrodynamic sizes of various PEG-linked antibody single variable domain polypeptides may be based upon highly cross-linked agarose. For example, the fractionation range of the two columns for globular proteins can be; Superose 12 HR 1000-3x105 Mr and Superose 6 HR 5000-5x106 Mr. The globular protein size exclusion limits are ~2x106 for the Superose 12 HR and ~4x107 Mr for the Superose 6HR.

The size of a polymer molecule attached to a dAb or dAb multimer of the invention can be thus varied depending on the desired application. For example, where the PEGylated dAb is intended to leave the circulation and enter into peripheral tissues, it is desirable to keep the size of the attached polymer low to facilitate extravazation from the blood stream. Alternatively, where it is desired to have the PEGylated dAb remain in the circulation for a longer period of time, a higher molecular weight polymer can be used (e.g., a 30 to 60 kDa polymer).

The polymer (PEG) molecules useful in the invention may be attached to dAb polypeptides (and polypeptide multimers) using methods which are well known in the art. The first step in the attachment of PEG or other polymer moieties to a dAb monomer or multimer of the invention is the substitution of the hydroxyl end-groups of the PEG polymer by electrophile-containing functional groups. Particularly, PEG polymers are attached to either cysteine or lysine residues present in the dAb monomers or multimers of the invention. The cysteine and lysine residues may be naturally occurring, or may be engineered into the dAb molecule. For example, cysteine residues may be recombinantly engineered at the C-terminus of dAb polypeptides, or residues at specific solvent accessible locations in the dAb may be substituted with cysteine or lysine. In a preferred embodiment, a PEG moiety is attached to a cysteine residue which is present in the hinge region at the C-terminus of a dAb monomer or multimer of the invention. In a further preferred embodiment a PEG moiety or other polymer is attached to a cysteine or lysine residue which is either naturally occurring at or engineered into the N-terminus of antibody single variable domain polypeptide of the invention. In a still further embodiment, a PEG moiety or other polymer is attached to an antibody single variable domain according to the invention at a cysteine or lysine residue (either naturally occurring or engineered) which is at least 2 residues away from (e.g., internal to) the C- and/or N-terminus of the antibody single variable domain polypeptide.

In one embodiment, the PEG polymer(s) is attached to one or more cysteine or lysine residues present in a framework region (FWs) and one or more heterologous CDRs of an antibody single variable domain or the invention. CDRs and framework regions are those regions of an immunoglobulin variable domain as defined in the Kabat database of Sequences of Proteins of Immunological Interest (Kabat et al. (1991) Sequences of proteins of immunological interest, U.S. Department of Health and Human Services). In a preferred embodiment, a PEG polymer is linked to a cysteine or lysine residue in the $V_H$ framework segment DP47, or the $V_k$ framework segment DPK9. Cysteine and/or lysine residues of DP47 which may be linked to PEG according to the invention include the cysteine at positions 22, or 96 and the lysine at positions 43, 65, 76, or 98 of SEQ ID NO: 2 (Figure 13). Cysteine and/or lysine residues of DPK9 which may be linked to PEG according to the invention include the cysteine residues at positions 23, or 88 and the lysine residues at positions 39, 42, 45, 103, or 107 of SEQ ID NO: 4 (Figure 14). In addition, specific cysteine or lysine residues may be linked to PEG in the $V_H$ canonical framework region DP38, or DP45.

In addition, specific solvent accessible sites in the dAb molecule which are not naturally occurring cysteine or lysine residues may be mutated to a cysteine or lysine for attachment of a PEG polymer. Solvent accessible residues in any given dAb monomer or multimer may be determined using methods known in the art such as analysis of the crystal structure of a given dAb. For example, using the solved crystal structure of the $V_H$ dAb HEL4 (SEQ ID NO: 5), the residues Gln-13, Pro-14, Gly-15, Pro-41, Gly-42, Lys-43, Asp-62, Lys-65, Arg-87, Ala-88, Glu-89, Gln-112, Leu-115, Thr-117, Ser-119, and Ser-120 have been identified as being solvent accessible, and according to the present invention would be attractive candidates for mutation to cysteine or lysine residues for the attachment of a PEG polymer. In addition, using the solved crystal structure of the $V_k$ dummy dAb (SEQ ID NO: 6), the residues Val-15, Pro-40, Gly-41, Ser-56, Gly-57, Ser-60, Pro-80, Glu-81, Gln-100, Lys-107, and Arg-108 have been identified as being solvent accessible, and according to the present invention would be attractive candidates for mutation to cysteine or lysine residues for the attachment of a PEG polymer. Preferably, a PEG moiety or other polymer is attached to a cysteine or lysine residue

which is substituted into one or more of the positions Gln13, Pro41 or Leu115, or residues having similar solvent accessibility in other antibody single variable domain polypeptides according to the invention. In one embodiment of the invention, a PEG polymer is linked to multiple solvent accessible cysteine, or lysine residues, or to solvent accessible residues which have been mutated to a cysteine or lysine residue. Alternatively, only one solvent accessible residue is linked to PEG, either where the particular dAb only possesses one solvent accessible cysteine or lysine (or residue modified to a cysteine or lysine) or where a particular solvent accessible residue is selected from among several such residues for PEGylation.

Primary amino acid sequence of HEL4 (SEQ ID NO: 3).

1 EVQLLESGGG LVQPGGSLRL SCAASGFRIS DEDMGWVRQA PGKGLEWVSS

51 IYGPSGSTYY ADSVKGRFTI SRDNSKNTLY LQMNSLRAED TAVYYCASAL

101 EPLSEPLGFW GQGTLVTVSS

Primary amino acid sequence of $V_k$ dummy (SEQ ID NO: 4).

1 DIQMTQSPSS LSASVGDRVT ITCRASQSIS SYLNWYQQKP GKAPKLLIYA

51 ASSLQSGVPS RFSGSGSGTD FTLTISSLQP EDFATYYCQQ SYSTPNTFGQ

101 GTKVEIKR

Several attachment schemes which are useful in the invention are provided by the company Nektar (San Carlos, CA). For example, where attachment of PEG or other polymer to a lysine residue is desired, active esters of PEG polymers which have been derivatized with N-hydroxylsuccinimide, such as succinimidyl propionate may be used. Where attachment to a cysteine residue is intended, PEG polymers which have been derivatized with sulfhydryl-selective reagents such as maleimide, vinyl sulfone, or thiols may be used. Other examples of specific embodiments of PEG derivatives which may be used according to the invention to generate PEGylated dAbs may be found in the Nektar Catalog (available on the world wide web at nektar.com). In addition, several derivatized forms of PEG may be used according to the invention to facilitate attachment of the PEG polymer to a dAb monomer or multimer of the invention. PEG derivatives useful in the invention include, but are not limited to PEG-succinimidyl succinate, urethane linked PEG, PEG phenylcarbonate, PEG succinimidyl carbonate, PEG-carboxymethyl azide, dimethylmaleic anhydride PEG, PEG dithiocarbonate derivatives, PEG-tresylates (2,2,2-trifluoroethanesolfonates), mPEG imidoesters, and other as described in Zalipsky and Lee, (1992) ("Use of functionalized poly(ethylene glycol)s for modification of peptides" in Poly(Ethylene Glycol Chemistry: Biotechnical and Biomedical Applications, J. Milton Harris, Ed., Plenum Press, NY).

Figures 6-11 show several embodiments of the PEGylated dAb and dAb multimers of the invention. In each of the figures "X" represents the chemical modification of an amino acid in a dAb with a chemically activated PEG, e.g., PEG-NHS, SPA, MAL, VS, thiol, etc. The modified amino acid may be any residue in the dAb, but is preferably a cysteine or lysine, and is more preferably a solvent accessible residue. "PEG" as used in the figures represents a linear, branched, forked, and/or multi-arm PEG. "S" represents the amino acid cysteine.

Figure 7 shows various dimerized dAb formats wherein the dimer is formed by a disulfide bond between cysteine residues internal to the dAb structure, or which are present at the C-terminus of the dAb. In one embodiment, the C-terminal cysteine residue is present in the hinge region. The dAb dimers can be PEGylated at internal sites (see Figure 7-5) on either one or both of the dAb monomers. Alternatively, the dAb dimers may be formed by the attachment of branched, forked, or multi-arm PEG polymers linked to C-terminal cysteines of each dAb monomer (Figure 7-7), or linked to amino acid residues internal to each dAb monomer (Figure 7-11).

Figure 8 shows various embodiments of dAb homo- or heterodimers in which the dAb monomers are linked to form a dimer by means of a linking peptide such as a $(Gly_4Ser)_n$ linker, wherein n= 1 to about 10. Once the dAb monomers are linked to form dimers, derivatized PEG polymer(s) may then be attached randomly to the dimers, either

at cysteine or lysine residues, or may be specifically targeted to cysteine residues at the C-terminus or internal residues of one or both of the dAb monomers using any of the PEG linking moieties described herein (e.g., MAL, NHS, SPA, VS, or Thiol). Alternatively, the PEG polymer may be attached to thiol reactive residues present in the linker peptide which is used to link the dAb monomers (Figure 8-15). In addition, as shown in Figure 8-17, two or more dAb dimers, each formed using linker peptides, can be coupled via C-terminal disulfide bonds. Figure 9 specifically shows various embodiments of PEG attachment to specific cysteine residues present at internal sites, in the linker peptide, or at the C-terminus of one or both of the dAb monomers.

As can be seen from Figure 10, homo- or heterotrimeric dAbs can be formed by linking three dAb monomers together using either a series of linker peptides (e.g., a Gly Ser linker; Figure 10-25, 26, or 27), or using a multi-arm PEG polymer (Figure 10-23, 24), such that each PEG polymer of the multi-arm polymer is linked (via cysteine, lysine, or other solvent accessible residue) to the C-terminus of each of the dAb monomers. Where the dAb monomers are linked to one another via a linker peptide, the PEG polymers can be attached to the trimeric dAb at a C-terminal cysteine, or, as described above, may be attached via MAL, NETS, SPA, VS, or Thiol moieties to any other solvent accessible residue in one, two, or all three of the dAb monomers. Figure 10-27 shows one embodiment of the invention comprising a dAb trimer with dual specificity. In this embodiment, one or more (but not all) of the dAb monomers has a binding specificity for a different antigen than the remaining dAb monomers. dAb monomers in this type of dual-specific embodiment may be linked via a linking peptide as shown in Figure 10-27, or may alternatively be linked via a branched or multi-arm PEG in which each monomer of the trimer is linked to a PEG moiety. Where the monomers of the dual-specific trimer shown in Figure 10-27 are linked by means of a linker peptide, the trimer may be linked to one or more PEG polymers via any of the mechanisms described above. That is, PEG may be linked to a cysteine or lysine residue in one or more of the dAb monomers or present at the C-terminus of one or more of the monomers, or further still, engineered into one or more of the dAb monomers comprising the trimer. In any of the monomeric, dimeric, or trimeric dAbs described above, and shown in Figures 6 to 11, a PEG polymer may be attached to an existing cysteine or lysine residue, or may be attached to a cysteine or lysine residue which has been engineered into one or more of the dAb monomers, or alternatively, engineered into one or more of the linking peptides.

Figure 11 shows various embodiments for the PEGylation of a dAb hetero- or homotetramer of the invention. dAb monomers can be linked via one or more linking peptides (Figure 11-29), or alternatively may be linked to form a tetramer using a multi-arm or branched PEG polymer (Figure 11-28, 30). Alternatively, two or more dAb dimers, formed either by linking two monomers via a linker peptide may be subsequently linked to form a tetramer by a branched or multi-arm PEG polymer (Figure 11-29, 31), where the PEG polymer is attached to a lysine or cysteine residue present in the dAb monomer, at the C-terminus of the dAb monomer (Figure 11-29), or present in the linker peptide (Figure 11-31). As described above for dAb monomers, dimers, and trimers, one or more PEG polymers can be attached at any desired position in a dAb tetramer of the invention. For example, a PEG polymer may be attached at a C-terminal cysteine, or other residue, or may be attached to one or more residues in the linking peptides, or still further may be attached to any cysteine or lysine residue present or engineered into the dAb monomers comprising the tetramer.

Figure 12 shows examples of higher-order PEG-linked dAb multimers which may be generated according to the present invention. For example, a plurality of dAb dimers, linked either by means of a linker peptide (as shown in Figure 12-31) or alternatively linked via disulfide bonds between the monomers of each dimer, are themselves linked to form a tetramer of dimers by a multi-arm PEG polymer wherein each PEG of the multi-arm PEG polymer is linked to a C-terminal cysteine residue present in one of the monomers of each dimer pair. Figure 12-32 shows that, in an alternate embodiment, the PEG polymers of the multi-arm PEG may be linked to cysteine residues which are present in the linker peptide of each dimer. The means of dimer, trimer and tetramer formation (e.g., using linker peptides or disulfide bonding), as well as the location and means for PEG attachment as described above may be varied according to the invention to generate large PEG-linked dAb multimers, such as octamers, decamers, etc. For example, similar to the strategy shown in Figure 12 to generate large PEG-linked dAb multimers, trimers or tetramers of dAbs may themselves be linked together by, for example, a multi-arm PEG, a linker peptide, or disulfide bonding, to generate a large PEG-linked dAb multimer. Where a plurality of dAb dimers, trimers, tetramers, etc. are linked together by means of linking peptides, or disulfide bonds (e.g., instead of using a multi-arm PEG as shown in Figure 12), PEG polymers can then be linked to the resulting multimer by any of the means described herein. For example, the PEG polymer may be linked to a cysteine or lysine residue on the surface of one or more of the dAb monomers comprising the multimer, the PEG polymer may be linked to a C-terminal cysteine present in one or more of the dAb monomers comprising the multimer, or PEG polymer(s) may be linked to cysteine or lysine residues present in any of the linking peptides which link together the components of the multimer (i.e., the linking peptides which link the individual monomers, and/or the linking peptides which link the dimers, trimers, or tetramers together).

In each of the above embodiments, the PEG polymers can be attached to any amenable residue present in the dAb peptides, or, preferably, one or more residues of the dAb may be modified or mutated to a cysteine or lysine residue which may then be used as an attachment point for a PEG polymer. Preferably, a residue to be modified in this manner is a solvent accessible residue; that is, a residue, which when the dAb is in its natural folded configuration is

accessible to an aqueous environment and to a derivatized PEG polymer. Once one or more of these residues is mutated to a cysteine residue according to the invention, it is available for PEG attachment using a linear or branched MAL derivatized PEG (MAL-PEG).

In one embodiment, the invention provides an antibody single variable domain composition comprising an antibody single variable domain and PEG polymer wherein the ratio of PEG polymer to antibody single variable domain is a molar ratio of at least 0.25:1. In a further embodiment, the molar ratio of PEG polymer to antibody single variable domain is 0.33:1 or greater. In a still further embodiment, the molar ratio of PEG polymer to antibody single variable domain is 0.5:1 or greater.

Increased Half-life

The PEGylated dAb monomers and multimers of the invention confer a distinct advantage over those dAb molecules taught in the art, in that the PEGylated dAb molecules of the invention have a greatly prolonged half-life. Without being bound to one particular theory, it is believed that the increased half-life of the dAb molecules of the invention is conferred by the increased hydrodynamic size of the dAb resulting from the attachment of PEG polymer(s). More specifically, it is believed that two parameters play an important role in determining the serum half-life of PEGylated dAbs and dAb multimers. The first criterion is the nature and size of the PEG attachment, i.e., if the polymer used is simply a linear chain or a branched/forked chain, wherein the branched/forked chain gives rise to a longer half-life. The second is the location of the dAb on the final format and how many "free" unmodified PEG arms the molecule has. The resulting hydrodynamic size of the PEGylated dAb, as estimated, for example, by size exclusion chromatography, reflects the serum half-life of the molecule. Accordingly, the larger the hydrodynamic size of the PEGylated molecule, the greater the serum half-life.

Increased half-life is useful in *in vivo* applications of immunoglobulins, especially antibodies and most especially antibody fragments of small size. Such fragments (Fvs, Fabs, scFvs, dAbs) suffer from rapid clearance from the body; thus, while they are able to reach most parts of the body rapidly, and are quick to produce and easier to handle, their *in vivo* applications have been limited by their only brief persistence *in vivo.*

In one aspect, an antibody single variable domain polypeptide as described herein is stabilized *in vivo* by fusion with a moiety, such as PEG, that increases the hydrodynamic size of the dAb polypeptide. Methods for pharmacokinetic analysis and determination of dAb half-life will be familiar to those skilled in the art. Details may be found in Kenneth, A et al., Chemical Stability of Pharmaceuticals: A Handbook for Pharmacists and in Peters et al., Pharmacokinetic analysis: A Practical Approach (1996). Reference is also made to "Pharmacokinetics", M Gibaldi & D Perron, published by Marcel Dekker, 2nd Rev. ex edition (1982), which describes pharmacokinetic parameters such as t alpha and t beta half lives and area under the curve (AUC).

Typically, the half-life of a PEGylated dAb monomer or multimer of the invention is increased by 10%, 20%, 30%, 40%, 50% or more relative to a non-PEGylated dAb (wherein the dAb of the PEGylated dAb and non-PEGylated dAb are the same). Increases in the range of 2x, 3x, 4x, 5x, 7x, 10x, 20x, 30x, 40x, and up to 50x or more of the half-life are possible. Alternatively, or in addition, increases in the range of up to 30x, 40x, 50x, 60x, 70x, 80x, 90x, 100x, 150x of the half-life are possible.

Half lives ($t\frac{1}{2}$ alpha and $t\frac{1}{2}$ beta) and AUC can be determined from a curve of serum concentration of ligand against time. The WinNonlin analysis package (available from Pharsight Corp., Mountain View, CA 94040, USA) can be used, for example, to model the curve. In a first phase (the alpha phase) the ligand is undergoing mainly distribution in the patient, with some elimination. A second phase (beta phase) is the terminal phase when the ligand has been distributed and the serum concentration is decreasing as the ligand is cleared from the patient. The ta half-life is the half-life of the first phase and the tβ half-life is the half-life of the second phase. "Half-life" as used herein, unless otherwise noted, refers to the overall half-life of an antibody single variable domain of the invention determined by non-compartment modeling (as contrasted with biphasic modeling, for example). Beta half-life is a measurement of the time it takes for the amount of dAb monomer or multimer to be cleared from the mammal to which it is administered. Thus, advantageously, the present invention provides a dAb-containing composition, e.g., a dAb-effector group composition, having a ta half-life in the range of 0.25 hours to 6 hours or more. In one embodiment, the lower end of the range is 30 minutes, 45 minutes, 1 hour, 1.3 hours, 2 hours, 3 hours, 4 hours, 5 hours, 6 hours, 7 hours, 10 hours, 11 hours or 12 hours. In addition or alternatively, a dAb containing composition will have a ta half-life in the range of up to and including 12 hours. In one embodiment, the upper end of the range is 11, 10, 9, 8, 7, 6, or 5 hours. An example of a suitable range is 1.3 to 6 hours, 2 to 5 hours or 3 to 4 hours.

Advantageously, the present invention provides a dAb containing composition comprising a ligand according to the invention having a tβ half-life in the range of 1-170 hours or more. In one embodiment, the lower end of the range is 2.5 hours, 3 hours, 4 hours, 5 hours, 6 hours, 7 hours, 8 hours, 9 hours, 10 hours, 11 hours or 12 hours. In addition, or alternatively, a dAb containing composition, e.g. a dAb-effector group composition has a tβ half-life in the range of up to and including 21 days. In one embodiment, the upper end of the range is 12 hours, 24 hours, 2 days, 3 days, 5

days, 10 days, 15 days, or 20 days. Advantageously a dAb containing composition according to the invention will have a tβ half-life in the range 2-100 hours, 4-80 hours, and 10-40 hours. In a further embodiment, it will be in the range 12-48 hours. In a further embodiment still, it will be in the range 12-26 hours. The present invention provides a dAb containing composition comprising a ligand according to the invention having a half-life in the range of 1-170 hours or more. In one embodiment, the lower end of the range is 1.3 hours, 2.5 hours, 3 hours, 4 hours, 5 hours, 6 hours, 7 hours, 8 hours, 9 hours, 10 hours, 11 hours or 12 hours. In addition, or alternatively, a dAb containing composition, e.g. a dAb-effector group composition has a half-life in the range of up to and including 21 days. In one embodiment, the upper end of the range is 12 hours, 24 hours, 2 days, 3 days, 5 days, 10 days, 15 days, or 20 days.

In addition, or alternatively to the above criteria, the present invention provides a dAb containing composition comprising a ligand according to the invention having an AUC value (area under the curve) in the range of 1 mg.min/ml or more. In one embodiment, the lower end of the range is 5, 10, 15, 20, 30, 100, 200 or 300 mg.min/ml. In addition, or alternatively, a ligand or composition according to the invention has an AUC in the range of up to 600 mg.min/ml. In one embodiment, the upper end of the range is 500, 400, 300, 200, 150, 100, 75 or 50 mg.min/ml. Advantageously a ligand according to the invention will have an AUC in the range selected from the group consisting of the following: 15 to 150 mg.min/ml, 15 to 100 mg.min/ml, 15 to 75 mg.min/ml, and 15 to 50 mg.min/ml.

Increased Protease Stability

A further advantage of the present invention is that the PEGylated dAbs and dAb 5 multimers described herein possess increased stability to the action of proteases. Depending on the assay conditions, dAbs are generally intrinsically stable to the action of proteases. In the presence of pepsin, however, many dAbs are totally degraded at pH 2 because the protein is unfolded under the acid conditions, thus making the protein more accessible to the protease enzyme. The present invention provides PEGylated dAb molecules, including dAb multimers, wherein it is believed that the PEG polymer provides protection of the polypeptide backbone due the physical coverage of the backbone by the PEG polymer, thereby preventing the protease from gaining access to the polypeptide backbone and cleaving it. In a preferred embodiment a PEGylated dAb having a higher hydrodynamic size (e.g., 200 to 500 kDa) is generated according to the invention, because the larger hydrodynamic size will confirm a greater level of protection from protease degradation than a PEGylated dAb having a lower hydrodynamic size. In one embodiment, a PEG- or other polymer-linked antibody single variable domain monomer or multimer is degraded by no more than 10% when exposed to one or more of pepsin, trypsin, elastase, chymotrypsin, or carboxypeptidase, wherein if the protease is pepsin then exposure is carried out at pH 2.0 for 30 minutes, and if the protease is one or more of trypsin, elastase, chymotrypsin, or carboxypeptidase, then exposure is carried out at pH 8.0 for 30 minutes. In a preferred embodiment, a PEG- or other polymer-linked dAb monomer or multimer is degraded by no more than 10% when exposed to pepsin at pH 2.0 for 30 minutes, preferably no more than 5%, and preferably not degraded at all. In a further preferred embodiment, a PEG- or other polymer-linked dAb multimer (e.g., hetero- or homodimer, trimer, tetramer, octamer, etc.) of the invention is degraded by less than 5%, and is preferably not degraded at all in the presence of pepsin at pH 2.0 for 30 minutes. In a preferred embodiment, a PEG- or other polymer-linked dAb monomer or multimer is degraded by no more than 10% when exposed to trypsin, elastase, chymotrypsin, or carboxypeptidase at pH 8.0 for 30 minutes, preferably no more than 5%, and preferably not degraded at all. In a further preferred embodiment, a PEG- or other polymer-linked dAb multimer (e.g., hetero- or homodimer, trimer, tetramer, octamer, etc.) of the invention is degraded by less than 5%, and is preferably not degraded at all in the presence of trypsin, elastase, chymotrypsin, or carboxypeptidase at pH 8.0 for 30 minutes.

The relative ratios of protease:antibody single variable domain polypeptide may be altered according to the invention to achieve the desired level of degradation as described above. For example the ratio or protease to antibody single variable domain may be from about 1:30, to about 10:40, to about 20:50, to about 30:50, about 40:50, about 50: 50, about 50:40, about 50:30, about 50:20, about 50:10, about 50:1, about 40:1, and about 30:1.

Accordingly, the present invention provides a method for decreasing the degradation of an antibody single variable domain comprising linking an antibody single variable domain monomer or multimer to a PEG polymer according to any of the embodiments described herein. According to this aspect of the invention, the antibody single variable domain is degraded by no more than 10% in the presence of pepsin at pH2.0 for 30 minutes. In particular, a PEG-linked dAb multimer is degraded by no more than 5%, and preferably not degraded at all in the presence of pepsin at pH 2.0 for 30 minutes. In an alternate embodiment, the antibody single variable domain is degraded by no more than 10% when exposed to trypsin, elastase, chymotrypsin, or carboxypeptidase at pH 8.0 for 30 minutes, preferably no more than 5%, and preferably not degraded at all.

Degradation of PEG-linked dAb monomers and multimers according to the invention may be measured using methods which are well known to those of skill in the art. For example, following incubation of a PEG-linked dAb with pepsin at pH 2.0 for 30 minutes, or with trypsin, elastase, chymotrypsin, or carboxypeptidase at pH 8.0 for 30 minutes, the dAb samples may be analyzed by gel filtration, wherein degradation of the dAb monomer or multimer is evidenced by a gel band of a smaller molecular weight than an un-degraded (i.e., control dAb not treated with pepsin, trypsin,

chymotrypsin, elastase, or carboxypeptidase) dAb. Molecular weight of the dAb bands on the gel may be determined by comparing the migration of the band with the migration of a molecular weight ladder (see Figure 5). Other methods of measuring protein degradation are known in the art and may be adapted to evaluate the PEG-linked dAb monomers and multimers of the present invention.

Uses of Single Immunoglobulin Variable Domain Polypeptides:

PEGylated Antibody single variable domain polypeptides as described herein are useful for a variety of *in vivo* and *in vitro* diagnostic, and therapeutic and prophylactic applications. For example, the polypeptides can be incorporated into immunoassays (e.g., ELISAs, RIA, etc.) for the detection of their target antigens in biological samples. Single immunoglobulin variable domain polypeptides can also be of use in, for example, Western blotting applications and in affinity chromatography methods. Such techniques are well known to those of skill in the art.

A very important field of use for single immunoglobulin variable domain polypeptides is the treatment or prophylaxis of diseases or disorders related to the target antigen.

Essentially any disease or disorder that is a candidate for treatment or prophylaxis with an antibody preparation is a candidate for treatment or prophylaxis with a single immunoglobulin variable domain polypeptide as described herein. The high binding affinity, human sequence origin, small size and high solubility of the single immunoglobulin variable domain polypeptides described herein render them superior to, for example, full length antibodies or even, for example, scFv for the treatment or prophylaxis of human disease.

Among the diseases or disorders treatable or preventable using the single immunoglobulin variable domain polypeptides described herein are, for example, inflammation, sepsis (including, for example, septic shock, endotoxic shock, Gram negative sepsis and toxic shock syndrome), allergic hypersensitivity, cancer or other hyperproliferative disorders, autoimmune disorders (including, for example, diabetes, rheumatoid arthritis, multiple sclerosis, lupus erythematosis, myasthenia gravis, scleroderma, Crohn's disease, ulcerative colitis, Hashimoto's disease, Graves' disease, Sjögren' s syndrome, polyendocrine failure, vitiligo, peripheral neuropathy, graft-versus-host disease, autoimmune polyglandular syndrome type I, acute glomerulonephritis, Addison's disease, adult-onset idiopathic hypoparathyroidism (AOIH), alopecia totalis, amyotrophic lateral sclerosis, ankylosing spondylitis, autoimmune aplastic anemia, autoimmune hemolytic anemia, Behcet's disease, Celiac disease, chronic active hepatitis, CREST syndrome, dermatomyositis, dilated cardiomyopathy, eosinophilia-myalgia syndrome, epidermolisis bullosa acquisita (EBA), giant cell arteritis, Goodpasture's syndrome, Guillain-Barré syndrome, hemochromatosis, Henoch-Schönlein purpura, idiopathic IgA nephropathy, insulin-dependent diabetes mellitus (IDDM), juvenile rheumatoid arthritis, Lambert-Eaton syndrome, linear IgA dermatosis, myocarditis, narcolepsy, necrotizing vasculitis, neonatal lupus syndrome (NLE), nephrotic syndrome, pemphigoid, pemphigus, polymyositis, primary sclerosing cholangitis, psoriasis, rapidly-progressive glomerulonephritis (RPGN), Reiter's syndrome, stiff-man syndrome and thyroiditis), effects of infectious disease (e.g., by limiting inflammation, cachexia or cytokine-mediated tissue damage), transplant rejection and graft versus host disease, pulmonary disorders (e.g., respiratory distress syndrome, shock lung, chronic pulmonary inflammatory disease, pulmonary sarcoidosis, pulmonary fibrosis and silicosis), cardiac disorders (e.g., ischemia of the heart, heart insufficiency), inflammatory bone disorders and bone resorption disease, hepatitis (including alcoholic hepatitis and viral hepatitis), coagulation disturbances, reperfusion injury, keloid formation, scar tissue formation and pyrexia.

Cancers can be treated, for example, by targeting one or more molecules, e.g., cytokines or growth factors, cell surface receptors or antigens, or enzymes, necessary for the growth and/or metabolic activity of the tumor, or, for example, by using a single immunoglobulin variable domain polypeptide specific for a tumor-specific or tumor-enriched antigen to target a liked cytotoxic or apoptosis-inducing agent to the tumor cells. Other diseases or disorders, e.g., inflammatory or autoimmune disorders, can be treated in a similar manner, by targeting one or more mediators of the pathology with a neutralizing single immunoglobulin variable domain polypeptide as described herein. Most commonly, such mediators will be, for example, endogenous cytokines (e.g., TNF-$\alpha$) or their receptors that mediate inflammation or other tissue damage.

Pharmaceutical Compositions, Dosage and Administration

The single immunoglobulin variable domain polypeptides of the invention can be incorporated into pharmaceutical compositions suitable for administration to a subject.

Typically, the pharmaceutical composition comprises a single immunoglobulin variable domain polypeptide and a pharmaceutically acceptable carrier. As used herein, "pharmaceutically acceptable carrier" or "carrier" includes any and all solvents, dispersion media, coatings, antibacterial and antifungal agents, isotonic and absorption delaying agents, and the like that are physiologically compatible. The term "pharmaceutically acceptable carrier" excludes tissue culture medium comprising bovine or horse serum. Examples of pharmaceutically acceptable carriers include one or more of water, saline, phosphate buffered saline, dextrose, glycerol, ethanol and the like, as well as combinations thereof.

In many cases, it will be preferable to include isotonic agents, for example, sugars, polyalcohols such as mannitol, sorbitol, or sodium chloride in the composition. Pharmaceutically acceptable substances include minor amounts of auxiliary substances such as wetting or emulsifying agents, preservatives or buffers, which enhance the shelf life or effectiveness of the single immunoglobulin variable domain polypeptide.

The compositions as described herein may be in a variety of forms. These include, for example, liquid, semi-solid and solid dosage forms, such as liquid solutions (e.g., injectable and infusible solutions), dispersions or suspensions, tablets, pills, powders, liposomes and suppositories. The preferred form depends on the intended mode of administration and therapeutic application. Typical preferred compositions are in the form of injectable or infusible solutions, such as compositions similar to those used for passive immunization of humans with other antibodies. The preferred mode of administration is parenteral (e.g., intravenous, subcutaneous, intraperitoneal, intramuscular).

Therapeutic compositions typically must be sterile and stable under the conditions of manufacture and storage. The composition can be formulated as a solution, microemulsion, dispersion, liposome, or other ordered structure suitable to high drug concentration. Sterile injectable solutions can be prepared by incorporating the active compound in the required amount in an appropriate solvent with one or a combination of ingredients enumerated above, as required, followed by filter sterilization. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying that yields a powder of the active ingredient plus any additional desired ingredient from a previously sterile-filtered solution thereof. The proper fluidity of a solution can be maintained, for example, by the use of a coating such as lecithin, by the maintenance of the required particle size in the case of dispersion and by the use of surfactants.

The single immunoglobulin variable domain polypeptides described herein can be administered by a variety of methods known in the art, although for many therapeutic applications, the preferred route/mode of administration is intravenous injection or infusion. The polypeptide can also be administered by intramuscular or subcutaneous injection. PEGylated immunoglobulin variable region polypeptides of the invention may alternatively or in addition to the foregoing, be administered in via a delayed release mechanism. The delayed release mechanism may include suitable cellulose based polymers which are known to those of skill in the art, and may further include osmotic pumps which may be implanted in an individual mammal and which will release the PEGylated variable region polypeptide slowly over time. Release rates for osmotic pumps according to the invention include from about 0.5 ml over a 6 week period, to 0.1 ml over a 2 week period. Release rates are preferably about 0.2 ml over a 4 week period. It will be understood by one of skill in the art that the specific release rate may be varied depending on the particular outcome desired, or PEGylated variable region polypeptide employed. Preparations according to the invention include concentrated solutions of the PEGylated antibody single variable domain (or PEGylated multimer), e.g., solutions of at least 5 mg/ml (~417 $\mu$M) in aqueous solution (e.g., PBS), and preferably at least 10 mg/ml (~833 $\mu$M), 20 mg/ml (~1.7 mM), 25 mg/ml (~2.1 mM), 30 mg/ml (~2.5 mM), 35 mg/ml (~2.9 mM), 40 mg/ml (~3.3 mM), 45 mg/ml (~3.75 mM), 50 mg/ml (~4.2 mM), 55 mg/ml (~4.6 mM) 60 mg/ml (~5.0 mM), 65 mg/ml (~5.4 mM), 70 mg/ml (~5.8 mM), 75 mg/ml (~6.3 mM), 100 mg/m (~8.33 mM), 150 mg/ml (~12.5 mM), 200 mg/ml (~16.7 mM) or higher. In some embodiments, preparations can be, for example, 250 mg/ml (~20.8 mM), 300 mg/ml (~25 mM), 350 mg/m (29.2 mM) or even higher, but be diluted down to 200 mg/ml or below prior to use.

As will be appreciated by the skilled artisan, the route and/or mode of administration will vary depending upon the desired results. In certain embodiments, the active compound may be prepared with a carrier that will protect the compound against rapid release, such as a controlled release formulation, including implants, transdermal patches, and microencapsulated delivery systems. Single immunoglobulin variable domains are well suited for formulation as extended release preparations due, in part, to their small size - the number of moles per dose can be significantly higher than the dosage of, for example, full sized antibodies. Biodegradable, biocompatible polymers can be used, such as ethylene vinyl acetate, polyanhydrides, polyglycolic acid, collagen, polyorthoesters, and polylactic acid. Prolonged absorption of injectable compositions can be brought about by including in the composition an agent that delays absorption, for example, monostearate salts and gelatin. Many methods for the preparation of such formulations are patented or generally known to those skilled in the art. See, e.g., Sustained and Controlled Release Drug Delivery Systems, J. R. Robinson, ed., Marcel Dekker, Inc., New York, 1978. Additional methods applicable to the controlled or extended release of polypeptide agents such as the single immunoglobulin variable domain polypeptides disclosed herein are described, for example, in U.S. Patent Nos. 6,306,406 and 6,346,274, as well as, for example, in U.S. Patent Application Nos. US20020182254 and US20020051808, all of which are incorporated herein by reference.

In certain embodiments, a single immunoglobulin variable domain polypeptide may be orally administered, for example, with an inert diligent or an assimilable edible carrier. The compound (and other ingredients, if desired) may also be enclosed in a hard or soft shell gelatin capsule, compressed into tablets, or incorporated directly into the individual's diet. For oral therapeutic administration, the compounds may be incorporated with excipients and used in the form of ingestible tablets, buccal tablets, troches, capsules, elixirs, suspensions, syrups, wafers, and the like. To administer a

compound of the invention by other than parenteral administration, it may be necessary to coat the compound with, or coadminister the compound with, a material to prevent its inactivation.

Another embodiment of the present invention is a method for delivering a therapeutic polypeptide, agent or antigen across a natural barrier by covalently or non-covalently attaching thereto a polypeptide construct comprising at least one single domain antibody directed against an internalizing cellular receptor, wherein said construct internalizes upon binding to said receptor. According to the invention, a natural barrier includes, but is not limited to, the blood-brain, lung-blood, gut-blood, vaginal-blood, rectal-blood and nasal-blood barriers. For example, a peptide construct delivered via the upper respiratory tract and lung can be used for transport of therapeutic polypeptides or agents from the lung lumen to the blood. The construct binds specifically to a receptor present on the mucosal surface (bronchial epithelial cells) resulting in transport, via cellular internalization, of the therapeutic polypeptides or agents specific for bloodstream targets from the lung lumen to the blood. In another example, a therapeutic polypeptide or agent is linked to a polypeptide construct comprising at least one single domain antibody directed against an internalizing cellular receptor present on the intestinal wall into the bloodstream. Said construct induces a transfer through the wall, via cellular internalization, of said therapeutic polypeptide or agent.

Methods for the delivery and administration of compounds such as the antibody single variable domains of the present invention are known in the art and may be found in, for example, the teachings of WO04/041867, the contents of which are incorporated herein in their entirety.

The present invention also contemplates a method to determine which antibody single variable domain polypeptides (e.g., dAbs; VHH) cross a natural barrier into the bloodstream upon administration using, for example, oral, nasal, lung, skin. In a non-limiting example, the method comprises administering a naive, synthetic or immune antibody single variable domain phage library to a small animal such as a mouse. At different time points after administration, blood is retrieved to rescue phages that have been actively transferred to the bloodstream. Additionally, after administration, organs can be isolated and bound phages can be stripped off. A non-limiting example of a receptor for active transport from the lung lumen to the bloodstream is the Fc receptor N (FcRn). The method of the invention thus identifies single domain antibodies which are not only actively transported to the blood, but are also able to target specific organs. The method may identify which antibody single variable domain polypeptides are transported across the gut and into the blood; across the tongue (or beneath) and into the blood; across the skin and into the blood etc. One aspect of the invention is the single domain antibodies obtained by using said method. Methods for determining which antibody single variable domain polypeptides may be best suited for administration in a pharmaceutical formulation according to the invention are taught in WO04/041867, the contents of which are incorporated herein in their entirety.

Additional active compounds can also be incorporated into the compositions. In certain embodiments, a single immunoglobulin variable domain polypeptide is coformulated with and/or coadministered with one or more additional therapeutic agents. For example, a single immunoglobulin variable domain polypeptide may be coformulated and/or coadministered with one or more additional antibodies that bind other targets (e.g., antibodies that bind other cytokines or that bind cell surface molecules), or, for example, one or more cytokines. Such combination therapies may utilize lower dosages of the administered therapeutic agents, thus avoiding possible toxicities or complications associated with the various monotherapies.

The pharmaceutical compositions of the invention may include a "therapeutically effective amount" or a "prophylactically effective amount" of a single immunoglobulin variable domain polypeptide. A "therapeutically effective amount" refers to an amount effective, at dosages and for periods of time necessary, to achieve the desired therapeutic result. A therapeutically effective amount of the single immunoglobulin variable domain polypeptide may vary according to factors such as the disease state, age, sex, and weight of the individual, and the ability of the single immunoglobulin variable domain polypeptide to elicit a desired response in the individual. A therapeutically effective amount is also one in which any toxic or detrimental effects of the antibody or antibody portion are outweighed by the therapeutically beneficial effects. A "prophylactically effective amount" refers to an amount effective, at dosages and for periods of time necessary, to achieve the desired prophylactic result. Typically, because a prophylactic dose is used in subjects prior to or at an earlier stage of disease, the prophylactically effective amount will be less than the therapeutically effective amount.

Dosage regimens may be adjusted to provide the optimum desired response (e.g., a therapeutic or prophylactic response). For example, a single bolus may be administered, several divided doses may be administered over time or the dose may be proportionally reduced or increased as indicated by the exigencies of the therapeutic situation. It is advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active compound calculated to produce the desired therapeutic effect in association with the required pharmaceutical carrier.

A non-limiting range for a therapeutically or prophylactically effective amount of a single immunoglobulin variable domain polypeptide is 0.1-20 mg/kg, more preferably 1-10 mg/kg. It is to be noted that dosage values may vary with the type and severity of the condition to be alleviated. It is to be further understood that for any particular subject, specific dosage regimens should be adjusted over time according to the individual need and the professional judgment of the

administering clinician.

The efficacy of treatment with a single immunoglobulin variable domain polypeptide as described herein is judged by the skilled clinician on the basis of improvement in one or more symptoms or indicators of the disease state or disorder being treated. An improvement of at least 10% (increase or decrease, depending upon the indicator being measured) in one or more clinical indicators is considered "effective treatment," although greater improvements are preferred, such as 20%, 30%, 40%, 50%, 75%, 90%, or even 100%, or, depending upon the indicator being measured, more than 100% (e.g., two-fold, three-fold, ten-fold, etc., up to and including attainment of a disease-free state). Indicators can be physical measurements, e.g., enzyme, cytokine, growth factor or metabolite levels, rate of cell growth or cell death, or the presence or amount of abnormal cells. One can also measure, for example, differences in the amount of time between flare-ups of symptoms of the disease or disorder (e.g., for remitting/relapsing diseases, such as multiple sclerosis). Alternatively, non-physical measurements, such as a reported reduction in pain or discomfort or other indicator of disease status can be relied upon to gauge the effectiveness of treatment. Where non-physical measurements are made, various clinically acceptable scales or indices can be used, for example, the Crohn's Disease Activity Index, or CDAI (Best et al., 1976, Gastroenterology 70:439), which combines both physical indicators, such as hematocrit and the number of liquid or very soft stools, among others, with patient-reported factors such as the severity of abdominal pain or cramping and general well-being, to assign a disease score.

As the term is used herein, "prophylaxis" performed using a composition as described herein is "effective" if the onset or severity of one or more symptoms is delayed or reduced by at least 10%, or abolished, relative to such symptoms in a similar individual (human or animal model) not treated with the composition.

Accepted animal models of human disease can be used to assess the efficacy of a single immunoglobulin variable domain polypeptide as described herein for treatment or prophylaxis of a disease or disorder. Examples of such disease models include, for example: a guinea pig model for allergic asthma as described by Savoie et al., 1995, Am. J. Respir. Cell Biol. 13:133-143; an animal model for multiple sclerosis, experimental autoimmune encephalomyelitis (EAE), which can be induced in a number of species, e.g., guinea pig (Suckling et al., 1984, Lab. Anim. 18:36-39), Lewis rat (Feurer et al., 1985, J. Neuroimmunol. 10:159-166), rabbits (Brenner et al., 1985, Isr. J. Med. Sci. 21:945-949), and mice (Zamvil et al., 1985, Nature 317:355-358); animal models known in the art for diabetes, including models for both insulin-dependent diabetes mellitus (IDDM) and non-insulin-dependent diabetes mellitus (NIDDM) - examples include the non-obese diabetic (NOD) mouse (e.g., Li et al., 1994, Proc. Natl. Acad. Sci. U.S.A. 91:11128-11132), the BB/DP rat (Okwueze et al., 1994, Am. J. Physiol. 266:R572-R577), the Wistar fatty rat (Jiao et al., 1991, Int. J. Obesity 15: 487-495), and the Zucker diabetic fatty rat (Lee et al., 1994, Proc. Natl. Acad. Sci. U.S.A. 91:10878-10882); animal models for prostate disease (Loweth et al., 1990, Vet. Pathol. 27:347-353), models for atherosclerosis (numerous models, including those described by Chao et al., 1994, J. Lipid Res. 35:71-83; Yoshida et al., 1990, Lab. Anim. Sci. 40:486-489; and Hara et al., 1990, Jpn. J. Exp. Med. 60:315-318); nephrotic syndrome (Ogura et al., 1989, Lab. Anim. 23:169-174); autoimmune thyroiditis (Dietrich et al., 1989, Lab. Anim. 23:345-352); hyperuricemia/gout (Wu et al., 1994, Proc. Natl. Acad. Sci. U.S.A. 91:742-746), gastritis (Engstrand et al., 1990, Infect. Immunity 58:1763-1768); proteinuria/kidney glomerular defect (Hyun et al., 1991, Lab. Anim. Sci. 41:442-446); food allergy (e.g., Ermel et al., 1997, Lab. Anim. Sci. 47:40-49; Knippels et al., 1998, Clin. Exp. Allergy 28:368-375; Adel-Patient et al., 2000, J. Immunol. Meth. 235:21-32; Kitagawa et al., 1995, Am. J. Med. Sci. 310:183-187; Panush et al., 1990, J. Rheumatol. 17:285-290); rheumatoid disease (Mauri et al., 1997, J. Immunol. 159:5032-5041; Saegusa et al., 1997, J. Vet. Med. Sci. 59:897-903; Takeshita et al., 1997, Exp. Anim. 46:165-169); osteoarthritis (Rothschild et al., 1997, Clin. Exp. Rheumatol. 15:45-51; Matyas et al., 1995, Arthritis Rheum. 38:420-425); lupus (Walker et al., 1983, Vet. Immunol. Immunopathol. 15:97-104; Walker et al., 1978, J. Lab. Clin. Med. 92:932-943); and Crohn's disease (Dieleman et al., 1997, Scand. J. Gastroenterol. Supp. 223:99-104; Anthony et al., 1995, Int. J. Exp. Pathol. 76:215-224; Osborne et al., 1993, Br. J. Surg. 80:226-229). Other animal models are known to those skilled in the art.

Whereas the single immunoglobulin variable domain polypeptides described herein must bind a human antigen with high affinity, where one is to evaluate its effect in an animal model system, the polypeptide must cross-react with one or more antigens in the animal model system, preferably at high affinity. One of skill in the art can readily determine if this condition is satisfied for a given animal model system and a given single immunoglobulin variable domain polypeptide. If this condition is satisfied, the efficacy of the single immunoglobulin variable domain polypeptide can be examined by administering it to an animal model under conditions which mimic a disease state and monitoring one or more indicators of that disease state for at least a 10% improvement.

EXAMPLES

All patents, patent applications, and published references cited herein are hereby incorporated by reference in their entirety. While this invention has been particularly shown and described with references to preferred embodiments thereof, it will be understood by those skilled in the art that various changes in form and details may be made therein without departing from the scope of the invention encompassed by the appended claims.

SUMMARY

Site specific maleimide-PEGylation of VH and Vk dAbs requires a solvent accessible cysteine to be provided on the surface of the protein, in this example at the C-terminus. The cysteine residue, once reduced to give the free thiol, can then be used to specifically couple the protein to maleimide or thiol-PEG dAb. A wide range of chemical modified PEGs of different sizes and branched formats are available from Nektar (formally known as Shearwater Corp). This allows the basic dAb-cys monomer to be formatted in a variety of ways for example as a PEGylated monomer, dimer, trimer, tetramer etc. The size of the PEGs is given in kDa but can also be referred to as K (i.e. "40K PEG" = 40 kDa PEG).

**1.0 Example 1: PEGylation of a Vk dAb TAR1-5-19**

**1.1 PCR construction of TAR1-5-19 cys**

TAR1-5-19 will be used as an example involving the engineering of a C-terminal cys onto a Vk cLAb (Figure 6-3). The site of attachment for the PEG may be placed elsewhere on the surface of the dAb as long as the targeted amino acid is solvent accessible and the resultant PEGylated protein still maintains antigen binding. Thus it is also possible to engineer the cys into any one of frameworks 1-4 of the dAb for PEGylation and still maintain some antigen binding. The following oligonucleotides were used to specifically PCR TAR1-5-19 with a *Sal*I and *Bam*HI sites for cloning and also to introduce a C-terminal cysteine residue. The sequence below is the DNA sequence of TAR1-5-19 cys and the PCR primers used to amplify the engineered dAb.

*SalI*

```
      Trp Ser Ala Ser Thr Asp Ile Gln Met Thr Gln Ser Pro Ser Ser Leu Ser Ala Ser Val
1     TGG AGC GCG TCG ACG GAC ATC CAG ATG ACC CAG TCT CCA TCC TCT CTG TCT GCA TCT GTA
      ACC TCG CGC AGC TGC CTG TAG GTC TAC TGG GTC AGA GGT AGG AGA GAC AGA CGT AGA CAT

      Gly Asp Arg Val Thr Ile Thr Cys Arg Ala Ser Gln Ser Ile Asp Ser Tyr Leu His Trp
61    GGA GAC CGT GTC ACC ATC ACT TGC CGG GCA AGT CAG AGC ATT GAT AGT TAT TTA CAT TGG
      CCT CTG GCA CAG TGG TAG TGA ACG GCC CGT TCA GTC TCG TAA CTA TCA ATA AAT GTA ACC
```

Tyr Gln Gln Lys Pro Gly Lys Ala Pro Lys Leu Leu Ile Tyr Ser Ala Ser Glu Leu Gln
121 TAC CAG CAG AAA CCA GGG AAA GCC CCT AAG CTC CTG ATC TAT AGT GCA TCC GAG TTG CAA
ATG GTC GTC TTT GGT CCC TTT CGG GGA TTC GAG GAC TAG ATA TCA CGT AGG CTC AAC GTT

Ser Gly Val Pro Ser Arg Phe Ser Gly Ser Gly Ser Gly Thr Asp Phe Thr Leu Thr Ile
181 AGT GGG GTC CCA TCA CGT TTC AGT GGC AGT GGA TCT GGG ACA GAT TTC ACT CTC ACC ATC
TCA CCC CAG GGT AGT GCA AAG TCA CCG TCA CCT AGA CCC TGT CTA AAG TGA GAG TGG TAG

Ser Ser Leu Gln Pro Glu Asp Phe Ala Thr Tyr Tyr Cys Gln Gln Val Val Trp Arg Pro
241 AGC AGT CTG CAA CCT GAA GAT TTT GCT ACG TAC TAC TGT CAA CAG GTT GTG TGG CGT CCT
TCG TCA GAC GTT GGA CTT CTA AAA CGA TGC ATG ATG ACA GTT GTC CAA CAC ACC GCA GGA

*Bam*HI

Phe Thr Phe Gly Gln Gly Thr Lys Val Glu Ile Lys Arg Cys *** *** Gly Ser Gly
301 TTT ACG TTC GGC CAA GGG ACC AAG GTG GAA ATC AAA CGG TGC TAA TAA GGA TCC GGC
AAA TGC AAG CCG GTT CCC TGG TTC CAC CTT TAG TTT GCC ACG ATT ATT CCT AGG CCG
(SEQ ID NO: 9)
(SEQ ID NO: 8)
(SEQ ID NO: 7)

Forward primer (SEQ ID NO: 10)

5'-TGGAGCGCGTCGACGGACATCCAGATGACCCAGTCTCCA-3'

Reverse primer (SEQ ID NO: 11)

5'-TTAGCAGCCGGATCCTTATTAGCACCGTTTGATTTCCAC-3'

The PCR reaction (50 μL volume) was set up as follows: 200 μM dNTP's, 0.4 μM of each primer, 5 μL of 10x P*fu*Turbo buffer (Stratagene), 100 ng of template plasmid (TAR1-5-19), 1 μL of P*fu*Turbo enzyme (Stratagene) and the volume adjusted to 50 μL using sterile water. The following PCR conditions were used: initial denaturing step 94 °C for 2 mins, then 25 cycles of 94 °C for 30 secs, 64 °C for 30 sec and 72 °C for 30 sec. A final extension step was also included of 72 °C for 5 mins. The PCR product was purified and digested with *Sal*I and *Bam*HI and ligated into the vector which had also been cut with the same restriction enzymes. Correct clones were verified by DNA sequencing.

**1.2 Expression and purification of TAR1-5-19 cys**

TAR1-5-19 cys vector was transformed into BL21 (DE3) pLysS chemically competent cells (Novagen) following the manufacturer's protocol. Cells carrying the dAb plasmid were selected for using 100 μg/mL carbenicillin and 37 μg/mL chloramphenicol. Cultures were set up in 2L baffled flasks containing 500 mL of terrific broth (Sigma-Aldrich), 100 μg/mL carbenicillin and 37 μg/mL chloramphenicol. The cultures were grown at 30 °C at 200rpm to an O.D.600 of 1-1.5 and then induced with 1mM IPTG (isopropyl-beta-D-thiogalactopyranoside, from Melford Laboratories). The expression of the dAb was allowed to continue for 12-16 hrs at 30 °C. It was found that most of the dAb was present in the culture media. Therefore, the cells were separated from the media by centrifugation (8,000xg for 30 mins), and the supernatant used to purify the dAb. Per litre of supernatant, 30 mL of Protein L agarose (Affitech) was added and the dAb allowed to batch bind with stirring for 2 hours. The resin was then allowed to settle under gravity for a further hour before the supernatant was siphoned off. The agarose was then packed into a XK 50 column (Amersham Pharmacia) and was washed with 10 column volumes of 2xPBS. The bound dAb was eluted with 100 mM glycine pH 2.0 and protein containing fractions were then neutralized by the addition of 1/5 volume of 1M Tris pH 8.0. Per litre of culture supernatant 20-30 mg of pure protein was isolated, which contained a 50:50 ratio of monomer to TAR1-5-19 disulphide dimer [Figure 7-4].

### 1.3 PEGylation of TAR1-5-19 cys using MAL activated PEG

#### 1.3.1 Monomer PEGylation

The cysteine residue which has been engineered onto the surface of the VH or Vk dAb may be specifically modified with a single linear or branched PEG-MAL to give monomeric modified protein. Shown below are two mPEG-MAL formats which may be used to PEGylate a monomeric VH or Vk dAb. The PEGs may be of MW from 500 to 60,000 (e.g., from 2,000 to 40,000) in size.

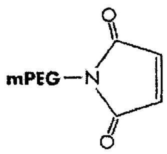

mPEG-MAL

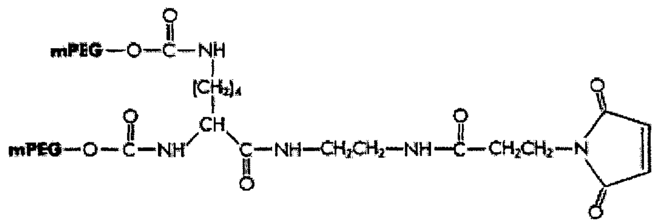

mPEG2-MAL

2.5 ml of 500 μM TAR1-5-19 cys was reduced with 5 mM dithiothreitol and left at room temperature for 20 minutes. The sample was then buffer exchanged using a PD-10 column (Amersham Pharmacia). The column had been pre-equilibrated with 5 mM EDTA, 20 mM sodium phosphate pH 6.5, 10% glycerol, and the sample applied and eluted following the manufactures guidelines. The sample (3.5 ml of ~360 μM dAb) was placed on ice until required. A four fold molar excess of 40K PEG-MAL (~200 mgs) was weighed out and solubilised in 100% methanol prior to mixing with the reduced dAb solution. The reaction was left to proceed at room temperature for 3 hours.

#### 1.3.2 Purification of PEGylated TAR1-5-19 cys monomer

In this example the Vk dAb was purified using cation exchange chromatography as the isoelectric point (pI) of the protein is ~8.8. If the pI of the protein was low, for example 4.0, then anion exchange chromatography would be used. 40 μL of 40% glacial acetic acid was added per mL of the 40K PEG TAR1-5-19 cys reaction to reduce the pH to ~4. The sample was then applied to a 1 mL Resource 5 cation exchange column (Amersham Pharmacia), which had been pre-equilibrated with 50 mM sodium acetate pH 4.0. The PEGylated material was separated from the unmodified dAb by running a linear sodium chloride gradient from 0 to 500 mM over 20 column volumes. Fractions containing PEGylated dAb only were identified using SDS-PAGE and then pooled and the pH increased to 8 by the addition of 1/5 volume of 1M Tris pH 8.0.

#### 1.3.3 The multimerisation of TAR1-5-19 cys using mPEG-MALs

The multimerisation of dAbs can be achieved by using a wide range of forked/branched PEGs which have been modified with multiple reactive groups to which the dAb may be covalently attached [See, e.g., Figures 7, and 10]. It has been shown that for multi-subunit targets such as TNF, multimerisation of the dAb (to dimers, trimers and tetramers) has a significant increase in the avidity for its antigen (see Table 1).

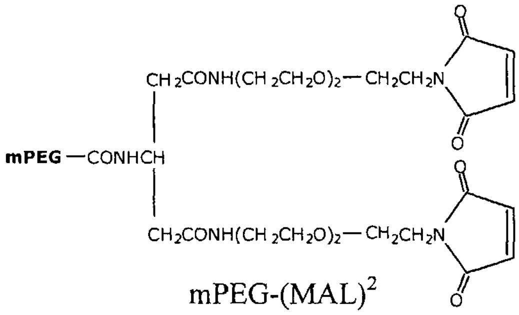

mPEG-(MAL)$^2$

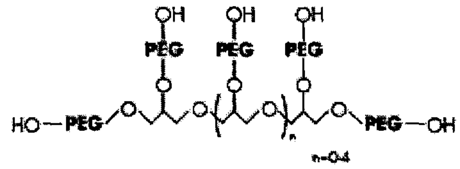

multi-arm PEG

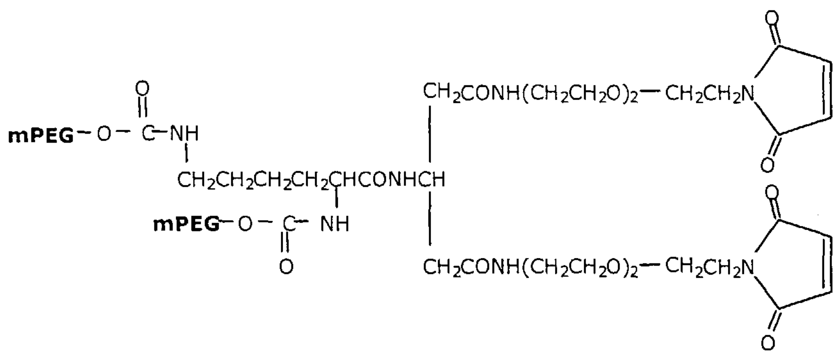

mPEG2-(MAL)$^2$

The above structures show mPEG-MAL formats which may be used to multimerise VH and Vk dAbs. The mPEG MW range from 500 to 60,000 (e.g., from 2,000 to 40,000) in size. dAb dimers are produced using mPEG(MAL) 2 or mPEG2(MAL)2; trimers and tetramers using 3 or 4-arm PEGs respectively. The multi-arm PEG shown would require to be modified with MAL to allow the attachment of the dAbs.

To produce the multimerised dAb formats the experimental methodology was identical to that used to produce the monomer except that the molar ratio of the PEG-MAL:dAb was varied depending upon the format of the PEG-MAL being used. For example, to produce TAR1-5-19 dimer [shown in Figure 7-7] using mPEG2-(MAL)2 the PEG-MAL:dAb molar ratio used was 0.5:1. To purify the PEGylated dimer, again cation exchange chromatography was used as described for the PEGylated monomer with the following changes. The sodium chloride gradient used was 0 to 250 mM salt over 30 column volumes. To produce TAR1-5-19 trimer [shown in Figure 10-23] using 3-arm PEG-MAL the PEG-MAL:dAb molar ratio used was 0.33:1. To purify the PEGylated trimer, again cation exchange chromatography was used as described for the PEGylated monomer with the following changes. The sodium chloride gradient used was 0 to 250 mM salt over 30 column volumes. To produce TAR1-5-19 tetramer [shown in Figure 11-28] using 4-arm PEG-MAL the PEG-MAL:dAb molar ratio used was 0.25:1. To purify the PEGylated tetramer, again cation exchange chromatography was used as described for the PEGylated monomer with the following changes. The sodium chloride gradient used was 0 to 250 mM salt over 30 column volumes. For the TAR1-5-19 PEG-trimer and tetramer, if required, the samples were further purified using size exclusion chromatography. A Superose 6 HR column (Amersham Pharmacia) was equilibrated with phosphate buffered saline (PBS) prior to loading on the sample. The column was run at 0.5 ml/min and the protein elution monitored by following the absorption at 280 nm. Fractions containing PEGylated dAb only were identified using SDS-PAGE and then pooled.

**2.0 Example 2: MAL-PEGylation of a VH dAb TAR2-10-27**

**2.1 PCR construction of TAR2-10-27 cys**

TAR2-10-27 will be used as an example the engineering of a C-terminal cys onto a VH dAb. As with Vk dAbs

the site of attachment for the PEG may be placed elsewhere on the surface of the dAb as long as the targeted amino acid is solvent accessible and the resultant PEGylated protein still maintains antigen binding. The following oligonucleotides were used to specifically PCR TAR1-5-19 with a *Sal*I and *Bam*HI sites for cloning and also to introduce a C-terminal cysteine residue. The PCR reaction conditions and cloning was done as outlined in Section 1.1, with the only changes being that the template used was plasmid DNA containing TAR2-10-27.

<u>*Sal*I</u>

| | | | | | | | | | | | | | | | |
|---|---|---|---|---|---|---|---|---|---|---|---|---|---|---|---|
| | Ala | Ser | Thr | Glu | Val | Gln | Leu | Leu | Glu | Ser | Gly | Gly | Gly | Leu | Val |
| 1 | GCG | TCG | ACG | GAG | GTC | CAG | CTG | TTG | GAG | TCT | GGG | GGA | GGC | TTG | GTA |
| | CGC | AGC | TGC | CTC | CAC | GTC | GAC | AAC | CTC | AGA | CCC | CCT | CCG | AAC | CAT |
| | Gln | Pro | Gly | Gly | Ser | Leu | Arg | Leu | Ser | Cys | Ala | Ala | Ser | Gly | Phe |
| 46 | CAG | CCT | GGG | GGG | TCC | CTG | CGT | CTC | TCC | TGT | GCA | GCC | TCC | GGA | TTC |
| | GTC | GGA | CCC | CCC | AGG | GAC | GCA | GAG | AGG | ACA | CGT | CGG | AGG | CCT | AAG |
| | Thr | Phe | Glu | Trp | Tyr | Trp | Met | Gly | Trp | Val | Arg | Gln | Ala | Pro | Gly |
| 91 | ACC | TTT | GAG | TGG | TAT | TGG | ATG | GGT | TGG | GTC | CGC | CAG | GCT | CCA | GGG |
| | TGG | AAA | CTC | ACC | ATA | ACC | TAC | CCA | ACC | CAG | GCG | GTC | CGA | GGT | CCC |
| | Lys | Gly | Leu | Glu | Trp | Val | Ser | Ala | Ile | Ser | Gly | Ser | Gly | Gly | Ser |
| 136 | AAG | GGT | CTA | GAG | TGG | GTC | TCA | GCT | ATC | AGT | GGT | AGT | GGT | GGT | AGC |
| | TTC | CCA | GAT | CTC | ACC | CAG | AGT | CGA | TAG | TCA | CCA | TCA | CCA | CCA | TCG |
| | Thr | Tyr | Tyr | Ala | Asp | Ser | Val | Lys | Gly | Arg | Phe | Thr | Ile | Ser | Arg |
| 181 | ACA | TAC | TAC | GCA | GAC | TCC | GTG | AAG | GGC | CGG | TTC | ACC | ATC | TCC | CGC |
| | TGT | ATG | ATG | CGT | CTG | AGG | CAC | TTC | CCG | GCC | AAG | TGG | TAG | AGG | GCG |

| | | | | | | | | | | | | | | | |
|---|---|---|---|---|---|---|---|---|---|---|---|---|---|---|---|
| | Asp | Asn | Ser | Lys | Asn | Thr | Leu | Tyr | Leu | Gln | Met | Asn | Ser | Leu | Arg |
| 226 | GAC | AAT | TCC | AAG | AAC | ACG | CTG | TAT | CTG | CAA | ATG | AAC | AGC | CTG | CGT |
| | CTG | TTA | AGG | TTC | TTG | TGC | GAC | ATA | GAC | GTT | TAC | TTG | TCG | GAC | GCA |
| | Ala | Glu | Asp | Ala | Ala | Val | Tyr | Tyr | Cys | Ala | Lys | Val | Lys | Leu | Gly |
| 271 | GCC | GAG | GAC | GCC | GCG | GTA | TAT | TAC | TGT | GCG | AAA | GTT | AAG | TTG | GGG |
| | CGG | CTC | CTG | CGG | CGC | CAT | ATA | ATG | ACA | CGC | TTT | CAA | TTC | AAC | CCC |
| | Gly | Gly | Pro | Asn | Phe | Gly | Tyr | Arg | Gly | Gln | Gly | Thr | Leu | Val | Thr |
| 316 | GGG | GGG | CCT | AAT | TTT | GGC | TAC | CGG | GGC | CAG | GGA | ACC | CTG | GTC | ACC |
| | CCC | CCC | GGA | TTA | AAA | CCG | ATG | GCC | CCG | GTC | CCT | TGG | GAC | CAG | TGG |

*Bam*HI

| | | | | | | | | |
|---|---|---|---|---|---|---|---|---|
| | Val | Ser | Cys | *** | *** | Gly | Ser | (SEQ ID NO: 14) |
| 361 | GTC | TCG | TGC | TAA | TAA | GGA | TCC | (SEQ ID NO: 12) |
| | CAG | AGC | ACG | ATT | ATT | CCT | AGG | (SEQ ID NO: 13) |

Forward primer (SEQ ID NO: 15)

5'-AGTGCGTCGACGGAGGTGCAGCTGTTGGAGTCT-3'

Reverse primer (SEQ ID NO: 16)

5'-AAAGGATCCTTATTAGCACGAGACGGTGACCAGGGTTCCCTG-3'

**Fig. 2.1: DNA sequence of TAR2-10-27 cys and the PCR primers used to amplify the engineered dAb.**

### 2.2 Expression and purification of TAR2-10-27 cys

The expression and purification of DOM1h-10-27 cys was as described in Section 1.2 but with the following modifications. As the dAb is a VH, Streamline Protein A was used to purify the protein from the culture supernatant. Also the protein was eluted from the resin using 100 mM glycine pH 3.0 instead of pH 2.0 buffer.

### 2.3 PEGylation of TAR2-10-27 cys

The monomer PEGylation of TAR2-10-27cys was as outlined in Section 1.3.1. Again VH dAbs may be multimerised using PEG as outlined in Section 1.3.3. The purification of PEGylated TAR2-10-27 cys was done using cation exchange chromatography at pH 4.0 (as outlined in Section 1.3.2), as the pI of the protein is ~8.5.

### 3.0 PEGylation of VH and Vk dAbs using NHS and SPA modified mPEG

As well as using a site specific method of PEGylation, a more random approach was used to covalently modify the protein. This involved using NHS or SPA modified PEGs, which react with solvent exposed surface lysine residues on the dAb [Figure 6-2]. This has the advantage in that the dAb does not require any protein engineering as there are several surface lysines already present on the surface of VH and Vk dAbs. Also any dAb format may be PEGylated without any prior modification, for example TAR1-5-19 disulphide dimer [Figure 7-5,6] or ultra affinity dimers [Figure 8-13, 8-14, 8-15] (see Section 3.3 and 5.0). As with the MAL-PEGs a variety of linear/forked and branched SPA and NHS-PEG formats are available as shown below.

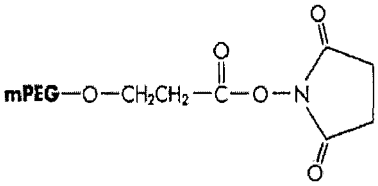

mPEG-SPA

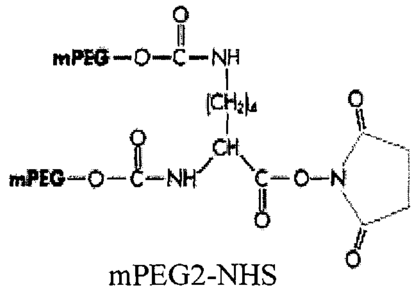

mPEG2-NHS

**Example 3: NHS and SPA-PEGylation of the Vk dAb TAR1-5-19**

**3.1 PCR construction of TAR1-5-19**

TAR1-5-19 will be used as an example of a Vk dAb which has been PEGylated using NHS and SPA-PEGs. TAR1-5-19 was PCR amplified and cloned using the primers below and as outlined in Section 1.1.

*Sal*I

```
     Trp Ser Ala Ser Thr Asp Ile Gln Met Thr Gln Ser Pro Ser Ser Leu Ser Ala Ser Val
1    TGG AGG GCG TCG ACG GAC ATC CAG ATG ACC CAG TCT CCA TCC TCT CTG TCT GCA TCT GTA
     ACC TCG CGC AGC TGC CTG TAG GTC TAC TGG GTC AGA GGT AGG AGA GAC AGA CGT AGA CAT

Gly  Asp Arg Val Thr Ile Thr Cys Arg Ala Ser Gln Ser Ile Asp Ser Tyr Leu His Trp
61   GGA GAC CGT GTC ACC ATC ACT TGC CGG GCA AGT GAG AGC ATT GAT AGT TAT TTA CAT TGG
     CCT CTG GCA CAG TGG TAG TGA ACG GCC CGT TCA GTC TCG TAA CTA TCA ATA AAT GTA ACC
```

| | |
|---|---|
| | Tyr Gln Gln Lys Pro Gly Lys Ala Pro Lys Leu Leu Ile Tyr Ser Ala Ser Glu Leu Gln |
| 121 | TAC CAG CAG AAA CCA GGG AAA GCC CCT AAG CTC CTG ATC TAT AGT GCA TCC GAG TTG CAA |
| | ATG GTC GTC TTT GGT CCC TTT CGG GGA TTC GAG GAC TAG ATA TCA CGT AGG CTC AAC GTT |
| | Ser Gly Val Pro Ser Arg Phe Ser Gly Ser Gly Ser Gly Thr Asp Phe Thr Leu Thr Ile |
| 181 | AGT GGG GTC CCA TCA CGT TTC AGT GGC AGT GGA TCT GGG ACA GAT TTC ACT CTC ACC ATC |
| | TCA CCC CAG GGT AGT GCA AAG TCA CCG TCA CCT AGA CCC TGT CTA AAG TGA GAG TGG TAG |
| | Ser Ser Leu Gln Pro Glu Asp Phe Ala Thr Tyr Tyr Cys Gln Gln Val Val Trp Arg Pro |
| 241 | AGC AGT CTG CAA CCT GAA GAT TTT GCT ACG TAC TAC TGT CAA CAG GTT GTG TGG CGT CCT |
| | TCG TCA GAC GTT GGA CTT CTA AAA CGA TGC ATG ATG ACA GTT GTC CAA CAC ACC GCA GGA |
| | *Bam*HI |
| | Phe Thr Phe Gly Gln Gly Thr Lys Val Glu Ile Lys Arg *** *** Gly Ser Gly |
| 301 | TTT ACG TTC GGC CAA GGG ACC AAG GTG GAA ATC AAA CGG TAA TAA GGA TCC GGC |
| | AAA TGC AAG CCG GTT CCC TGG TTC CAC CTT TAG TTT GCC ATT ATT CCT AGG CCG |

(SEQ ID NO: 19)
(SEQ ID NO: 17)
(SEQ ID NO: 18)

Forward primer (SEQ ID NO: 20)

5'-TGGAGCGCGTCGACGGACATCCAGATGACCCAGTCTCCA-3'

Reverse primer (SEQ ID NO: 21)

5'-AAAGGATCCTTATTACCGTTTGATTTCCACCTTGGTCCC-3'

### 3.2 Expression and purification of TAR1-5-19

The expression and purification of TAR1-5-19 was as outlined in Section 1.2. The only modification was that once purified the protein was dialysed against PBS or buffer exchanged (PD10) to remove the tris/glycine buffer present.

### 3.3 PEGylation of monomeric and TAR1-5-19 disulphide dimer with NHS or SPA-PEG

PEGylation reactions were carried out in either PBS buffer or 50 mM sodium phosphate pH 7.0. 400 μM of TAR1-5-19 monomer was mixed with a 5-10 molar excess of activated PEG (either NHS or SPA dissolved in 100% methanol). The reaction was allowed to proceed at room temperature for 2-4 hours and then stopped by the addition of 1M glycine pH 3.0 to a final concentration of 20 mM. It was found that the TAR1-5-19 disulphide dimer produced during expression (Section 1.2) could also be PEGylated using the above methodology. This allowed the disulphide dimer format to be PEGylated without the need for reduction with DTT followed by modification with the PEG2-(MAL)2. The only modification to the protocol was the addition of 10% glycerol to the dAb dimer to prevent precipitation during any concentration steps.

### 3.4 Purification of the PEGylated Vk dAbs

The monomer and disulphide dimer PEGylated dAb was purified using cation exchange chromatography as outlined in Section 1.3.3.

**Example 4: NHS and SPA-PEGylation of a VH dAb HEL4**

**4.1 PCR construction of HEL4**

HEL4 will be used as an example of a VH dAb which has been PEGylated using NHS and SPA-PEGs. HEL4 was PCR amplified and cloned using the primers below and under the conditions as outlined in Section 1.1, with the only modification being that the template DNA was a plasmid vector containing the HEL4 DNA sequence.

*Sal*I

```
     Ala Ser Thr Glu Val Gln Leu Leu Glu Ser Gly Gly Gly Leu Val Gln Pro Gly Gly Ser
1    GCG TCG ACG GAG GTG CAG CTG TTG GAG TCT GGG GGA GGC TTG GTA CAG CCT GGG GGG TCC
     CGC AGC TGC CTC CAC GTC GAC AAC CTC AGA CCC CCT CCG AAC CAT GTC GGA CCC CCC AGG

     Leu Arg Leu Ser Cys Ala Ala Ser Gly Phe Arg Ile Ser Asp Glu Asp Met Gly Trp Val
61   CTG CGT CTC TCC TGT GCA GCC TCC GGA TTT AGG ATT AGC GAT GAG GAT ATG GGC TGG GTC
     GAC GCA GAG AGG ACA CGT CGG AGG CCT AAA TCC TAA TCG CTA CTC CTA TAC CCG ACC GAG

     Arg Gln Ala Pro Gly Lys Gly Leu Glu Trp Val Ser Ser Ile Tyr Gly Pro Ser Gly Ser
121  CGC CAG GCT CCA GGG AAG GGT CTA GAG TGG GTA TCA AGC ATT TAT GGC CCT AGC GGT AGC
     GCG GTC CGA GGT CCC TTC CCA GAT CTC ACC CAT AGT TCG TAA ATA CCG GGA TCG CCA TCG

     Thr Tyr Tyr Ala Asp Ser Val Lys Gly Arg Phe Thr Ile Ser Arg Asp Asn Ser Lys Asn
181  ACA TAC TAC GCA GAC TCC GTG AAG GGC CGG TTC ACC ATC TCC CGT GAC AAT TCC AAG AAC
     TGT ATG ATG CGT CTG AGG CAC TTC CCG GCC AAG TGG TAG AGG GCA CTG TTA AGG TTC TTG

     Thr Leu Tyr Leu Gln Met Asn Ser Leu Arg Ala Glu Asp Thr Ala Val Tyr Tyr Cys Ala
241  ACG CTG TAT CTG CAA ATG AAC AGC CTG CGT GCC GAG GAC ACC GCG GTA TAT TAT TGC GCG
     TGC GAC ATA GAC GTT TAC TTG TCG GAC GCA CGG CTC CTG TGG CGC CAT ATA ATA ACG CGC

     Ser Ala Leu Glu Pro Leu Ser Glu Pro Leu Gly Phe Trp Gly Gln Gly Thr Leu Val Thr
301  AGT GCT TTG GAG CCG CTT TCG GAG CCC CTG GGC TTT TGG GGT CAG GGA ACC CTG GTC ACC
     TCA CGA AAC CTC GGC GAA AGC CTC GGG GAC CCG AAA ACC CCA GTC CCT TGG GAC CAG TGG
```

*Bam*HI

```
     Val Ser Ser *** *** Gly Ser
361  GTC TCG AGC TAA TAA GGA TCC
     CAG AGC TCG ATT ATT CCT AGG
```

(SEQ ID NO: 24)
(SEQ ID NO: 22)
(SEQ ID NO: 23)

Forward primer (SEQ ID NO: 25)

5'-AGTGCGTCGACGGAGGTGCAGCTGTTGGAGTCT-3'

Reverse primer (SEQ ID NO: 26)

5'-AAAGGATCCTTATTAGCTCGAGACGGTGACCAGGGTTCCCTG-3'

**4.2 Expression and purification of HEL4**

The VH dAb was expressed and purified as outlined in Section 2.2.

**4.3 NHS and SPA PEGylation of HEL4**

The methodology for modification of the surface lysine residues using NHS and SPA activated PEGs was as described in Section 3.3. The only modification to the protocol was that the reaction was terminated by the addition of 1M Tris buffer pH 8.0 to a final concentration of 20 mM.

**4.4 Purification of NHS or SPA PEGylated HEL4**

Purification of the PEGylated protein was carried out using anion exchange chromatography as the pI of the HEL4 is ~4. The column used was a 1ml Resource Q column (Amersham Pharmacia), which had been equilibrated with 50 mM Tris pH 8.0. The pH of the sample was shifted to 8 prior to loading onto the column. A linear gradient from 0 to 500 mM sodium chloride in 50 mM Tris pH 8.0 was used to separate the PEGylated protein from unmodified dAb.

**4.5 Affinity binding of PEGylated HEL4**

A HEL4 affinity based resin (based on the antigen, hen egg white lysozyme) was produced to test the functionality of the PEGylated dAb. Lysozyme was coupled to NETS activated Sepharose 4B resin (Amersham Pharmacia) following the manufacturer's instructions. The PEGylated samples (5 $\mu$g) were then mixed with the affinity resin (100 $\mu$l) and allowed to bind in PBS at room temperature for 30 mins. The resin was then extensively washed with PBS (3x 1ml) to remove unbound protein. The affinity resin was then run on a SDS-PAGE to determine if the PEGylated HEL4 had bound to the matrix (see Section 8.3).

**Example 5: PEGylation of ultra affinity dimers using SPA and NHS activated PEGs**

$V_H$ and $V_k$ dAbs may be multimerised using a $(Gly_4Ser)_n$ linker (n = 0-7) to form a single polypeptide chain (e.g. two dAbs to give an ultra affinity dimer; Figure 8-12). Therefore it is possible to form homodimeric ($VH_1$-$VH_1$ and $VL_1$-$VL_1$) or heterodimeric ($VH_1$-$VH_2$ and $VL_1$-$VL_2$) pairings with dual specificities. As well as forming dimers, additional dAbs may be added to make larger proteins such as trimers [Figure 10-27] or tetramers [Figure 11-32]. Again since a combination of VH and Vk dAbs may be used to make these larger formats, it is possible to make dual specific molecules. For example a dAb trimer with the ability to have an extended serum half-life and engage TNF$\alpha$; one dAb with the ability to bind to serum albumin linked to two TAR1-5-19 dAbs that bind TNF as a dimer. The ultra affinity dimers have been PEGylated using NHS and SPA-PEGs [Figure 8-13, 8-14, and 8-15] as well as being specifically engineered to accept MAL-PEGs at the C-terminus of the protein (example 6a and 6b) [Figure 8-16]. In this example the ultra affinity dimer TAR1-5-19-Dimer 4 was modified with either 20K-SPA or 40K-NHS. The DNA sequence of the ultra affinity dimer TAR1-5-19 Dimer 4 is shown below.

| | |
|---|---|
| | Asp Ile Gln Met Thr Gln Ser Pro Ser Ser Leu Ser Ala Ser Val Gly Asp Arg Val Thr |
| 1 | GAC ATC CAG ATG ACC CAG TCT CCA TCC TCT CTG TCT GCA TCT GTA GGA GAC CGT GTC ACC |
| | CTG TAG GTC TAC TGG GTC AGA GGT AGG AGA GAC AGA CGT AGA CAT CCT CTG GCA CAG TGG |

*Kpn*I

| | |
|---|---|
| | Ile Thr Cys Arg Ala Ser Gln Ser Ile Asp Ser Tyr Leu His Trp Tyr Gln Gln Lys Pro |
| 61 | ATC ACT TGC CGG GCA AGT CAG AGC ATT GAT AGT TAT TTA CAT TGG TAC CAG CAG AAA CCA |
| | TAG TGA ACG GCC CGT TCA GTC TCG TAA CTA TCA ATA AAT GTA ACC ATG GTC GTC TTT GGT |
| | Gly Lys Ala Pro Lys Leu Leu Ile Tyr Ser Ala Ser Glu Leu Gln Ser Gly Val Pro Ser |
| 121 | GGG AAA GCC CCT AAG CTC CTG ATC TAT AGT GCA TCC GAG TTG CAA AGT GGG GTC CCA TCA |
| | CCC TTT CGG GGA TTC GAG GAC TAG ATA TCA CGT AGG CTC AAC GTT TCA CCC CAG GGT AGT |
| | Arg Phe Ser Gly Ser Gly Ser Gly Thr Asp Phe Thr Leu Thr Ile Ser Ser Leu Gln Pro |
| 181 | CGT TTC AGT GGC AGT GGA TCT GGG ACA GAT TTC ACT CTC ACC ATC AGC AGT CTG CAA CCT |
| | GCA AAG TCA CCG TCA CCT AGA CCC TGT CTA AAG TGA GAG TGG TAG TGG TCA GAC GTT GGA |

*Hind*II

Glu Asp Phe Ala Thr Tyr Tyr Cys Gln Gln Val Val Trp Arg Pro Phe Thr Phe Gly Gln
241 GAA GAT TTT GCT ACG TAC TAC TGT CAA CAG GTT GTG TGG CGT CCT TTT ACG TTC GGC CAA
CTT CTA AAA CGA TGC ATG ATG ACA GTT GTC CAA CAC ACC GCA GGA AAA TGC AAG CCG GTT

*Xho*I

Gly Thr Lys Val Glu Ile Lys Arg Ser Ser Gly Gly Gly Gly Ser Gly Gly Gly Gly Ser
301 GGG ACC AAG GTG GAA ATC AAA CGC TCG AGC GGT GGA GGC GGT TCA GGC GGA GGT GGC AGC
CCC TGG TTC CAC CTT TAG TTT GCG AGC TCG CCA CCT CCG CCA AGT CCG CCT CCA CCG TCG

*Sal*I

*Hind*II

Gly Gly Gly Gly Ser Gly Gly Gly Gly Ser Gly Gly Gly Gly Ser Thr Asp Ile Gln Met
361 GGC GGT GGC GGG TCA GGT GGT GGC GGA AGC GGC GGT GGC GGG TCG ACG GAC ATC CAG ATG
CCG CCA CCG CCC AGT CCA CCA CCG CCT TCG CCG CCA CCG CCC AGC TGC CTG TAG GTC TAC

Thr Gln Ser Pro Ser Ser Leu Ser Ala Ser Val Gly Asp Arg Val Thr Ile Thr Cys Arg
421 ACC CAG TCT CCA TCC TCC CTG TCT GCA TCT GTA GGA GAC CGT GTC ACC ATC ACT TGC CGG
TGG GTC AGA GGT AGG AGG GAC AGA CGT AGA CAT CCT CTG GCA CAG TGG TAG TGA ACG GCC

*Kpn*I

Ala Ser Gln Ser Val Lys Glu Phe Leu Trp Trp Tyr Gln Gln Lys Pro Gly Lys Ala Pro
481 GCA AGT CAG AGC GTT AAG GAG TTT TTA TGG TGG TAC CAG CAG AAA CCA GGG AAA GCC CCT
CGT TCA GTC TCG CAA TTC CTC AAA AAT ACC ACC ATG GTC GTC TTT GGT CCC TTT CGG GGA

Lys Leu Leu Ile Tyr Met Ala Ser Asn Leu Gln Ser Gly Val Pro Ser Arg Phe Ser Gly
541 AAG CTC CTG ATC TAT ATG GCA TCC AAT TTG CAA AGT GGG GTC CCA TCA CGT TTC AGT GGC
TTC GAG GAC TAG ATA TAC CGT AGG TTA AAC GTT TCA CCC CAG GGT AGT GCA AAG TCA CCG

Ser Gly Ser Gly Thr Asp Phe Thr Leu Thr Ile Ser Ser Leu Gln Pro Glu Asp Phe Ala
601 AGT GGA TCT GGG ACA GAT TTC ACT CTC ACC ATC AGC AGT CTG CAA CCT GAA GAT TTT GCT
TCA CCT AGA CCC TGT CTA AAG TGA GAG TGG TAG TCG TCA GAC GTT GGA CTT CTA AAA CGA

*Hind*II

Thr Tyr Tyr Cys Gln Gln Lys Phe Lys Leu Pro Arg Thr Phe Gly Gln Gly Thr Lys Val
661 ACG TAC TAC TGT CAA CAG AAG TTT AAG CTG CCT CGT ACG TTC GGC CAA GGG ACC AAG GTG
TGC ATG ATG ACA GTT GTC TTC AAA TTC GAC GGA GCA TGC AAG CCG GTT CCC TGG TTC CAC

*Not*I

Glu Ile Lys Arg Ala Ala Ala His His His His His His Gly Ala Ala Glu Gln Lys Leu
721 GAA ATC AAA CGG GCG GCC GCA CAT CAT CAT CAC CAT CAC GGG GCC GCA GAA CAA AAA CTC
CTT TAG TTT GCC CGC CGG CGT GTA GTA GTA GTG GTA GTG CCC CGG CGT CTT GTT TTT GAG

Ile Ser Glu Glu Asp Leu Asn Gly Ala Ala
781 ATC TCA GAA GAG GAT CTG AAT GGG GCC GCA
TAG AGT CTT CTC CTA GAC TTA CCC CGG CGT
(SEQ ID NO: 29)
(SEQ ID NO:27)
(SEQ ID NO: 28)

**5.1 Expression and purification of ultra affinity dimer TAR1-5-19 Dimer 4**

The expression of the dimer was as described in Section 1.2 with the only modification in the protocol being that the construct was transformed into TOP10 F' cells (Invitrogen) and that carbenicillin was used at a concentration of 100μg/ml.

**5.2 20K SPA and 40K NHS PEGylation and purification of the ultra affinity dimer TAR1-5-19 Dimer 4**

The dimer was PEGylated as outlined in Section 3.3 with either 20K SPA PEG or 40K NHS PEG. Both the 20K SPA and the 40K NHS PEGylated dimers were purified by cation exchange chromatography as described in Section 1.3.3 example 1a.

**Example 6a: Site specific PEGylation of ultra affinity dimers using MAL activated PEGs**

As with dAbs engineered with C-terminal cys, ultra affinity dAb dimers can also be modified in a similar fashion [Figure 8-16]. Again the cys-dimer can be used as the basic building block to create larger multi-dAb PEGylated formats [Figure 8-17, and 11-29, 11-31]. TAR1-5-19 homodimer with a $(Gly_4Ser)_5$ linker cys (see Figure 8-16) will be used as an example. As with the TAR1-5-19 cys monomer, both a monomeric (i.e. dimer; Figure 8-16) and dimeric (i.e. tetramer; Figure 8-17) will be produced in solution during expression. The DNA sequence of the ultra affinity dimer TAR1-5-19 homodimer cys is shown below.

Asp Ile Gln Met Thr Gln Ser Pro Ser Ser Leu Ser Ala Ser Val Gly Asp Arg Val Thr
1 GAC ATC CAG ATG ACC CAG TCT CCA TCC TCT CTG TCT GCA TCT GTA GGA GAC CGT GTC ACC
CTG TAG GTC TAC TGG GTC AGA GGT AGG AGA GAC AGA CGT AGA CAT CCT CTG GCA CAG TGG

*Kpn*I

Ile Thr Cys Arg Ala Ser Gln Ser Ile Asp Ser Tyr Leu His Trp Tyr Gln Gln Lys Pro
61 ATC ACT TGC CGG GCA AGT CAG AGC ATT GAT AGT TAT TTA CAT TGG TAC CAG CAG AAA CCA
TAG TGA ACG GCC CGT TCA GTC TCG TAA CTA TCA ATA AAT GTA ACC ATG GTC GTC TTT GGT

Gly Lys Ala Pro Lys Leu Leu Ile Tyr Ser Ala Ser Glu Leu Gln Ser Gly Val Pro Ser
121 GGG AAA GCC CCT AAG CTC CTG ATC TAT AGT GCA TCC GAG TTG CAA AGT GGG GTC CCA TCA
CCC TTT CGG GGA TTC GAG GAC TAG ATA TCA CGT AGG CTC AAC GTT TCA CCC CAG GGT AGT

Arg Phe Ser Gly Ser Gly Ser Gly Thr Asp Phe Thr Leu Thr Ile Ser Ser Leu Gln Pro
181 CGT TTC AGT GGC AGT GGA TCT GGG ACA GAT TTC ACT CTC ACC ATC AGC AGT CTG CAA CCT
GCA AAG TCA CCG TCA CCT AGA CCC TGT CTA AAG TGA GAG TGG TAG TCG TCA GAC GTT GGA

*Hind*II
Glu Asp Phe Ala Thr Tyr Tyr Cys Gln Gln Val Val Trp Arg Pro Phe Thr Phe Gly Gln
241 GAA GAT TTT GCT ACG TAC TAC TGT CAA CAG GTT GTG TGG CGT CCT TTT ACG TTC GGC CAA
CTT CTA AAA CGA TGC ATG ATG ACA GTT GTC CAA CAC ACC GCA GGA AAA TGC AAG CCG GTT

*Xho*I
Gly Thr Lys Val Glu Ile Lys Arg Ser Ser Gly Gly Gly Gly Ser Gly Gly Gly Gly Ser
301 GGG ACC AAG GTG GAA ATC AAA CGC TCG AGC GGT GGA GGC GGT TCA GGC GGA GGT GGC AGC
CCC TGG TTC CAC CTT TAG TTT GCG AGC TCG CCA CCT CCG CCA AGT CCG CCT CCA CCG TCG

*Sal*I
*Hind*II
Gly Gly Gly Gly Ser Gly Gly Gly Gly Ser Gly Gly Gly Gly Ser Thr Asp Ile Gln Met
361 GGC GGT GGC GGG TCA GGT GGT GGC GGA AGC GGC GGT GGC GGG TCG ACG GAC ATC CAG ATG
CCG CCA CCG CCC AGT CCA CCA CCG CCT TCG CCG CCA CCG CCC AGC TGC CTG TAG GTC TAC

Thr Gln Ser Pro Ser Ser Leu Ser Ala Ser Val Gly Asp Arg Val Thr Ile Thr Cys Arg
421 ACC CAG TCT CCA TCC TCT CTG TCT GCA TCT GTA GGA GAC CGT GTC ACC ATC ACT TGC CGG
TGG GTC AGA GGT AGG AGA GAC AGA CGT AGA CAT CCT CTG GCA CAG TGG TAG TGA ACG GCC

*Kpn*I
Ala Ser Gln Ser Ile Asp Ser Tyr Leu His Trp Tyr Gln Gln Lys Pro Gly Lys Ala Pro
481 GCA AGT CAG AGC ATT GAT AGT TAT TTA CAT TGG TAC CAG CAG AAA CCA GGG AAA GCC CCT
CGT TCA GTC TCG TAA CTA TCA ATA AAT GTA ACC ATG GTC GTC TTT GGT CCC TTT CGG GGA

Lys Leu Leu Ile Tyr Ser Ala Ser Glu Leu Gln Ser Gly Val Pro Ser Arg Phe Ser Gly
541 AAG CTC CTG ATC TAT AGT GCA TCC GAG TTG CAA AGT GGG GTC CCA TCA CGT TTC AGT GGC
TTC GAG GAC TAG ATA TCA CGT AGG CTC AAC GTT TCA CCC CAG GGT AGT GCA AAG TCA CCG

| | |
|---|---|
| | Ser Gly Ser Gly Thr Asp Phe Thr Leu Thr Ile Ser Ser Leu Gln Pro Glu Asp Phe Ala |
| 601 | AGT GGA TCT GGG ACA GAT TTC ACT CTC ACC ATC AGC AGT CTG CAA CCT GAA GAT TTT GCT |
| | TCA CCT AGA CCC TGT CTA AAG TGA GAG TGG TAG TCG TCA GAC GTT GGA CTT CTA AAA CGA |
| | *Hind*II |
| | Thr Tyr Tyr Cys Gln Gln Val Val Trp Arg Pro Phe Thr Phe Gly Gln Gly Thr Lys Val |
| 661 | ACG TAC TAC TGT CAA CAG GTT GTG TGG CGT CCT TTT ACG TTC GGC CAA GGG ACC AAG GTG |
| | TGC ATG ATG ACA GTT GTC CAA CAC ACC GCA GGA AAA TGC AAG CCG GTT CCC TGG TTC CAC |
| | Glu Ile Lys Arg Cys |
| 721 | GAA ATC AAA CGC TGC |
| | CTT TAG TTT GCG ACG |
| | (SEQ ID NO: 32) |
| | (SEQ ID NO: 30) |
| | (SEQ ID NO: 31) |

**6.1 Expression and purification of ultra affinity dimer TAR1-5-19 homodimer cys**

The expression of the dimer was as described in Section 1.2 with the only modification in the protocol being that the construct was transformed into TOP10 F' cells (Invitrogen) and that carbenicillin was used at a concentration of 100μg/ml.

**6.2 Dimerisation and purification of TAR1-5-19 homodimer cys with 40K PEG2-(MAL)2**

TAR1-5-19 homodimer cys was PEGylated with 40K PEG2-(MAL)2 to form the dimer (i.e. 2 x linker dimers with a total of 4 dAbs; Figure 11-29]). The reaction and purification conditions were as described in Section 1.3.1 and modified as outlined in Example 1a.

**Example 6b: Tetramerisation and purification of TAR1-5-19 homodimer cys with 40K PEG2-(MAL)2**

TAR1-5-19 homodimer cys was PEGylated with 4-arm 20K PEG MAL to form the dimer tetramer (i.e. 4 x linker dimers with a total of 8 dAbs; Figure 11-31). The reaction and purification conditions were as described in Section 1.3.1 and modified as outlined in Example 1c.

**Example 7: Multimerisation of a low affinity binding dAb (VH or Vk) to create higher affinity formats**

TAR1-5 will be used as an example of a Vk dAb which has a relatively low binding affinity as a monomer for TNF, but the dAb can be formatted to increase its affinity via multimerisation. Again a C-terminal cysteine was used to multimerised the dAb via 4-arm 20K PEG MAL.

**7.1 PCR construction of TAR1-5 cys**

The following oligonucleotides were used to specifically PCR TAR1-5 with *Sal*I and *Bam*HI sites for cloning and also to introduce a C-terminal cysteine residue. The DNA sequence of TAR1-5 cys and the PCR primers used to amplify the engineered dAb are shown below.

*Sal*I

| | |
|---|---|
| | Trp Ser Ala Ser Thr Asp Ile Gln Met Thr Gln Ser Pro Ser Ser Leu Ser Ala Ser Val |
| 1 | TGG AGC GCG TCG ACG GAC ATC CAG ATG ACC CAG TCT CCA TCC TCC CTG TCT GCA TCT GTA |
| | ACC TCG CGC AGC TGC CTG TAG GTC TAC TGG GTC AGA GGT AGG AGG GAC AGA CGT AGA CAT |

*Kpn*I

| | |
|---|---|
| | Gly Asp Arg Val Thr Ile Thr Cys Arg Ala Ser Gln Ser Ile Phe Met Asn Leu Leu Trp |
| 61 | GGA GAC CGT GTC ACC ATC ACT TGC CGG GCA AGT CAG AGC ATT TTT ATG AAT TTA TTG TGG |
| | CCT CTG GCA CAG TGG TAG TGA ACG GCC CGT TCA GTC TCG TAA AAA TAC TTA AAT AAC ACC |

*Kpn*I

| | |
|---|---|
| | Tyr Gln Gln Lys Pro Gly Lys Ala Pro Lys Leu Leu Ile Tyr Asn Ala Ser Val Leu Gln |
| 121 | TAC CAG CAG AAA CCA GGG AAA GCC CCT AAG CTC CTG ATC TAT AAT GCA TCC GTG TTG CAA |
| | ATG GTC GTC TTT GGT CCC TTT CGG GGA TTC GAG GAC TAG ATA TTA CGT AGG CAC AAC GTT |

| | |
|---|---|
| | Ser Gly Val Pro Ser Arg Phe Ser Gly Ser Gly Ser Gly Thr Asp Phe Thr Leu Thr Ile |
| 181 | AGT GGG GTC CCA TCA CGT TTC AGT GGC AGT GGA TCT GGG ACA GAT TTC ACT CTC ACC ATC |
| | TCA CCC CAG GGT AGT GCA AAG TCA CCG TCA CCT AGA CCC TGT CTA AAG TGA GAG TGG TAG |

*Hind*II

| | |
|---|---|
| | Ser Ser Leu Gln Pro Glu Asp Phe Ala Thr Tyr Tyr Cys Gln Gln Val Val Trp Arg Pro |
| 241 | AGC AGT CTG CAA CCT GAA GAT TTT GCT ACG TAC TAC TGT CAA CAG GTT GTG TGG CGT CCT |
| | TCG TCA GAC GTT GGA CTT CTA AAA CGA TGC ATG ATG ACA GTT GTC CAA CAC ACC GCA GGA |

*Bam*HI

| | |
|---|---|
| | Phe Thr Phe Gly Gln Gly Thr Lys Val Glu Ile Lys Arg Cys *** *** Gly Ser Phe |
| 301 | TTT ACG TTC GGC CAA GGG ACC AAG GTG GAA ATC AAA CGG TGC TAA TAA GGA TCC TTT |
| | AAA TGC AAG CCG GTT CCC TGG TTC CAC CTT TAG TTT GCC ACG ATT ATT CCT AGG AAA |

(SEQ ID NO: 35)
(SEQ ID NO: 33)

(SEQ ID NO: 34)

Forward primer (SEQ ID NO: 36)

5'-TGGAGCGCGTCGACGGACATCCAGATGACCCAGTCTCCA-3'

Reverse primer (SEQ ID NO: 37)

5'-TTAGCAGCCGGATCCTTATTAGCACCGTTTGATTTCCAC-3'

The reaction conditions used were as described in Section 1.1.

### 7.2 Expression and purification of TAR1-5 cys

The expression and purification of the dAb was as outlined in Section 1.2. Again, a 50:50 mix of monomer and TAR1-5 disulphide dimer were formed.

### 7.3 Multimerisation via PEGylation and purification of 4-arm 20K PEG-MAL TAR1-5

The PEGylation and purification conditions were as described in Section 1.3.3 example 1c. The results of cell cytotoxicity assays are shown in Table 1.

### Example 8: *In vitro* functional binding assay: TNF receptor assay and cell assay

### 8.1 Assay data for PEGylated TAR1-5-19 monomer and TAR1-5-19 cys

The affinity of the PEGylated dAb for human TNFα was determined using the TNF receptor binding (RBA) and cell cytotoxicity assay (summarized in Table 1). Figure 1 shows the results from the RBA assay of a few selected PEGylated Vk dAbs, from the graphs the IC 50 was determined and summarized in Table 1. It can be seen that there is only a slight difference between the PEGylated and unmodified forms of the monomer and dimer in the RBA. Even with the larger 40K PEG-MAL the IC 50 is unaffected. A slight decrease is observed with the 40K-NHS, but this may be due to the PEGylation site being closer to the CDRs, whereas with the 40K-MAL is located at the C-terminus of the dAb. This shows that PEGylation has an insignificant effect on antigen binding as long as it is placed away from the CDRs. It can also be seen that the 3 and 4-arm PEGylated formats using the monomeric dAb have an improved affinity for TNF in the RBA. Figure 2 shows the same samples in the cell cytotoxicity assay. Again there is little difference between the PEGylated and unmodified samples, showing the potency of the dAb is maintained. The most significant difference is with the 3 and 4-arm PEG samples, which shows a reduction in the ND 50 to 100 and 30 pM respectively. With TAR1-5 it can be seen that the low affinity binding monomer has an ND 50 of ~10 μM in the cell assay. Upon dimerisation, the affinity is reduced to ~3 μM and when tetramerised using the 4-arm PEG, down to an ND 50 of 200 nM. Therefore even a low affinity dAb may be multimerised using multi-arm PEG to create formats with greater avidity for their antigen.

### 8.2 Assay data for PEGylated ultra affinity dimers TAR1-5-19 Dimer 4 and TAR1-5-19 homodimer cys

The SPA and NHS PEGylation of TAR1-5-19 Dimer 4 again showed that random surface modification only slightly affected the potency of the dimer (see Table 1). With the TAR1-5-19 homodimer cys it was found that dimerising the dimer to form the tetramer maintained the ND 50 of ~3 nM. A significant drop in the ND 50 was seen when the dimer was further multimerised using the 4-arm MAL-PEG to produce the octamer, shifting the ND 50 to ~100 pM.

This reflects the fact that the RBA assay's sensitivity is relatively high and that formats with high affinity will all appear to be ~100 pM.

**Table 1: Summary of the results from the receptor and cell cytotoxicity assays of dAb formats based upon TAR1-5-19**

| dAb format | Receptor assay IC 50 (nM) | Cell cytotoxicity assay ND 50 (nM) |
|---|---|---|
| Unmodified TAR1-5-19 monomer | 30 | 70 |
| 20K SPA TAR1-5-19 monomer | 45 | 70 |
| 40K NHS TAR1-5-19 monomer | 45 | 90 |
| 20K MAL TAR1-5-19 monomer | 30 | 70 |
| 40K MAL TAR1-5-19 monomer | 30 | 80 |
| Unmodified TAR1-5-19 disulphide dimer | 0.8 | 3 |
| 1x20K SPA TAR1-5-19 disulphide dimer | 1 | 3 |
| 1x40K NHS TAR1-5-19 disulphide dimer | 3 | 10 |
| 40K PEG2-(MAL2) TAR1-5-19 dimer | 1 | 3 |
| TAR1-5-19 D4 ultra affinity dimer | -- | 10 |

(continued)

| dAb format | Receptor assay IC 50 (nM) | Cell cytotoxicity assay ND 50 (nM) |
|---|---|---|
| 20K SPA PEG TAR1-5-19 D4 ultra affinity dimer | -- | 30 |
| 40K NHS PEG TAR1-5-19 D4 ultra affinity dimers | -- | 25 |
| TAR1-5-19 homodimer cys ultra affinity dimer | -- | 3 |
| TAR1-5-19 homodimer cys ultra affinity disulphide dimer | -- | 3 |
| 40K PEG2-(MAL)2 TAR1-5-19 homodimer cys ultra affinity | -- | 3 |
| 4-arm PEG-MAL TAR1-5-19 homodimer cys ultra affinity dimer tetramer (5K per arm) | -- | 0.1 |
| 3-arm PEG-MAL TAR1-5-19 trimer (5K per arm) | 0.2 | 0.1 |
| 4-arm PEG-MAL TAR1-5-19 tetramer (5K per arm) | 0.1 | 0.03 |
| 4-arm PEG-MAL TAR1-5-19 tetramer (10K per arm) | -- | 0.02 |
| TAR1-5 monomer | -- | 10,000 |
| TAR1-5 disulphide dimer | -- | 3000 |
| 4-arm PEG-MAL TAR1-5 tetramer (5K per arm) | -- | 200 |

**8.3 Binding of PEGylated HEL4 to lysozyme affinity matrix**

Various PEGylated HEL4 formats were tested to see if they retained the ability to bind antigen once they had been modified. Figure 3 shows the results of the binding assay. Specifically, the SDS PAGE gels showing affinity binding to lysozyme, and the lanes are as follows: 1,4, 7, 10, 13, 16 and 19 show protein remaining in the supernatant after batch binding for 30 mins; lanes 2, 5, 8, 11, 14, 17 and 20 show protein that may have eluted during PBS washing step; and lanes 3, 6, 9, 12, 15, 18 and 21 protein that was bound to the lysozyme affinity resin.

From the gels it can be clearly seen that the PEGylated protein is efficiently removed from the supernatant and remains bound to the resin even after several washes with PBS. Thus showing that the PEGylated dAb maintained specificity for its antigen whether is was specifically PEGylated with THIOL or MAL-PEG, or via surface lysine residues using SPA or NHS-PEGs.

**8.4 PEGylation of TAR2-1O-27**

The binding potency of TAR2-10-27 can be determined using a TNF receptor binding assay. The unmodified monomer had an IC 50 of ~3 nM in the assay, and as can be seen from Figure 4. The 40K PEGylated monomer has an IC 50 of ~20 nM.

**Example 9: Hydrodynamic size correlation to the serum *in vivo* half life of VH and Vk PEGylated dAbs**

PEGylation of proteins has been used to increase their *in vivo* serum half-life. The renal filtration cut-off size is approximately 70 kDa, which means for a protein the size of an unmodified dAb (VH ~13-14 kDa and Vk~12 kDa) the serum half-life will be relatively low ($t_{1/2}$ beta of ~10-30 mins). Work carried out on the PEGylation of dAbs has shown that the serum half-life of a dAb (either VH or Vk) can be modulated by the size and the branched nature of the PEG used. This has important applications for example in drug therapies where a prolonged half-life of tens of hours is desired

(e.g. >30 hrs); whereas a significantly shorter residency time of a few hours (3-6 hrs) is required if the dAb were to be labeled and used as an *in vivo* imaging reagent for diagnostic purposes.

We have shown that two parameters play an important role in determining the serum half-lives of PEGylated dAbs. The first is the nature and size of the PEG attachment, i.e. if the polymer used is simply a linear chain or branched/forked in nature. The second is the location of the dAb on the final format and how many "free" unmodified PEG arms the molecule has. The resultant hydrodynamic size of the PEGylated format, as estimated by size exclusion chromatography, reflects the serum half-life of the molecule. Thus the larger the hydrodynamic size of the PEGylated molecule the greater the serum half-life, as shown in Table 2.

The gel filtration matrices used to determine the hydrodynamic sizes of the various PEGylated proteins was based upon highly cross-linked agarose. The fractionation range of the two columns for globular proteins are; Superose 12 HR 1000-3x105 Mr and Superose 6 HR 5000-5x106 Mr. The globular protein size exclusion limits are ~2x106 for the Superose 12 HR and ~4x107 Mr for the Superose 6HR.

**Table 2**

| Format | Size of PEG Linear/branched | Estimated size (kDa)[a] | Hydrodynamic size (kDa)[b] | *In vivo* serum half-life ($t_{1/2}\beta$ hrs) |
|---|---|---|---|---|
| **Vk dAbs** | | | | |
| TAR1-5-19 monomer | na | 12 | 14 | 0.5 |
| TAR1-5-19 disulphide dimer | na | 24 | 30 | 2.5 |
| TAR1-5-19 disulphide dimer 20K PEG-SPA | 20K linear | 60 | 280[c] | 16 |
| TAR1-5-19 Pc fusion | na | 80 | na | >24 |
| TAR1-5-19 monomer 40K PEG-MAL | 40K branched (2x20K) | 95 | 550[c] | >36 |
| TAR1-5-19 dimer 40K PEG | 40K branched (2x20K) | 110 | 580[c] | ~51.6 |
| TAR1-5-19 trimer PEG-MAL | 15K 3-arm branched (3x5K) | 52 | 130[c] | na |
| TAR1-5-19 tetramer PEG-MAL | 20K 4-arm branched (4x5K) | 90 | 150[c] | ~12.7 |
| **VH dAbs** | | | | |
| HEL4 | na | 14 | 16 | na |
| HEL4 5K PEG-MAL | 5K linear | 24 | 70 | na |
| HEL4 20K PEG-MAL | 20K linear | 55 | 275 | na |
| HEL4 30K PEG-THIOL | 30K linear | 84 | 310 | na |
| HEL4 40K PEG-NHS | 40K branched (2x20K) | 95 | 544 | na |

**Notes: [a]estimated size determined by SDS-PAGE. [b]hydrodynamic sizes as determined by Superose 12HR (Amersham Pharmacia) size exclusion chromatography. [c]estimations on sizes.**

Thus, it can be seen from Table 2 that even though the 4-arm PEG format of TAR1-5-19 consists of 20K of PEG and its molecular mass is greater than that of the 20K SPA TAR1-5-19 disulphide dimer (95 kDa compared to 60

kDa), they have similar half-lives considering the renal cut-off is 70 kDa. This may be due to the fact that in the 4-arm format the dAbs are linked to the end of each PEG molecule and so overall the molecule is more compact (smaller hydrodynamic size); i.e. in this format there is a very high density of PEG at the core of the molecule compared to just having a single linear 20K chain. The advantage in using the 4-arm format is the significant increase in affinity to TNF compared to the dimeric molecules (30 pM compared to 3 nM respectively). Thus in some applications where a high affinity binder is required but with a short serum half-life, the molecule could be tailored to have both desired characteristics by varying the number of dAbs present and the size of PEG used.

**Example 10: Protease stability of PEGylated dAbs**

Another key feature of PEGylated dAbs is their increased stability to the action of proteases. dAbs intrinsically are relatively stable to the action of proteases, depending upon the assay conditions. For example TAR1-5-19 is not degraded by trypsin even in the presence of 5 M urea. This is because the dAb does not unfold in the presence of the denaturant, whereas it is totally degraded by pepsin at pH 2 as the protein is unfolded under the acidic conditions. PEGylation has the advantage in that the polymer chain most likely covers the surface of the dAb, thus preventing the protease from gaining access to the polypeptide backbone and cleaving it. Even when the protein is partially denatured at low pH, the presence of the PEG on the dAb significantly increased its resistance to the action of pepsin (Figure 5).

Figure 5 shows the results of pepsin degradation of TAR1-5-19 and other PEGylated variants. Reactions were carried out at pH 2.0, at 37° C for up to 30 minutes in the presence of 250 μg pepsin. The lane designations are as follows: 1: molecular weight ladder; 2: monomer (0 min); 3: monomer (15 min); 4: monomer (30 min); 5: 40K MAL2 dimer (0 min); 6: 40K MAL2 dimer (15 min); 7: 40K MAL2 dimer (30 min); 8: 20K MAL monomer (0 min); 9: 20K MAL monomer (15 min); 10: 20K MAL monomer. The gel shows that the unmodified dAb is degraded very quickly at low pH. Figure 5 also shows that the type of PEG present on the surface of the dAb also can have a significant effect on its resistance to the action of the protease. The linear 20K PEG does protect the dAb to some degree, but even after 30 mins some degradation is seen (bands at 3 kDa, estimated ~15-20% degradation from the gel). Whereas with the 40K PEG MAL2 dimer, very little degradation is seen even after 30 mins (at most <5% degradation from the gel). This is most likely due to the 2x20K format of PEG, which offers greater protection from the action of pepsin. This would suggest that the PEGylated dAb would also be resistant to the action of a wide range of proteases found in the digestive tract of humans and also may maintain functionality through the low pH environment of the stomach. Therefore a PEGylated dAb could be administered orally as a therapeutic drug without the need of complex formulations to prevent degradation or loss of functionality. A PEGylated dAb offers a clear advantage over other formats such as IgG, scFv or Fabs which may be more susceptible to the action of proteases and denaturation (thus potentially loosing functionality) at the extreme pHs experienced during digestion.

**Example 11: Site specific PEGylation of VH or Vk dAbs using NHS or SPA activated PEGs**

Due to the presence of several lysine residues on the surface of a dAb (VH or Vk), it is relatively inefficient to try to couple a single NHS/SPA-PEG to the protein as only low ratios of PEGs:dAb are used. Although a single PEGylated species can be produced, usually only between ~10-20% of the total protein will be PEGylated. Increasing the ratio of activated PEG invariably results in over-PEGylation. This could potentially cause the loss of antigen binding due to the CDRs being obscured by the PEG. A solution to this problem would be to substitute these surface lysines with amino acids that are also present in other human antibody frameworks. For example, VkI (DPK9) has 3 lysine residues in framework 2. These residues could be changed to those found in VkII (DPK18) (arginine and glutamine residues). Substituting the residues with another already found in a human framework would also reduce the potential effects of immunogenicity. Vk dAbs also have a C-terminal lysine residue, which would be retained for site specific PEGylation. Specific lysine engineering could also be done for VH dAbs. Again the surface lysines can be substituted to residues found in other corresponding human VH frameworks, but in this case the C-terminal serine would also require substituting to a lysine. This would then allow the site specific engineering of a single lysine into the dAb (in an identical manner already done with cysteine). The only requirement is that the lysine would need to be solvent accessible and placed so that upon PEGylation it would not significantly reduce binding to antigen.

**Example 12: Identifying potential PEGylation sites on VH and Vk dAb**

To specifically engineer a cysteine residue into the framework of VH or Vk dAbs for PEGylation, several factors have to be considered. These include;

- solvent accessibility of the introduced residue, which requires that it be placed at the surface of the protein

- the proximity of the introduced site to the CDR's, and how upon PEGylation this will affect antigen binding
- the disruption of native favorable interactions (such as hydrogen bonding) which upon substitution to cysteine may destabilize the protein or affect the folding pathway so that the resultant dAb is unable to be expressed efficiently

Therefore to investigate the most suitable site for the surface cysteine a direct comparison of protein expression, PEGylation and affinity binding assays was carried out. This would determine if a C-terminal positioned cysteine residue (Ser120cys) was indeed better than an internal coupling site for protein expression whilst maintaining antigen affinity.

Swiss PDB Viewer was used to calculate the solvent accessibility of surface amino acid residues, thus identifying potential sites for engineering in a cysteine residue. Also these residues were selected due to their distance from the CDRs. Introducing a large PEG molecule close to the CDRs would certainly have a have an affect on antigen binding. Table 3 shows the most suitable sites identified using the structures of HEL4 and Vk dummy (sequences shown below). It can be seen that the potential sites for PEGylation are clustered together into areas on the surface of the dAb. Therefore only one mutant (except for Group V) in each group was selected for expression (shown in bold). These sites are the most likely to be able to accommodate the engineered cysteine residue without significant loss in antigen binding.

Table 3: The sites were identified using the HEL4 crystal structure coordinates, and the modeled structure of Vk dummy. The amino acid numbering used reflects the primary amino acid sequence of each dAb (shown below)

| **dAb** | **Amino acid residues identified suitable for engineering** |
|---|---|
| VH | |
| Group I | **Gln-13,** Pro-14, Gly-15 |
| Group II | **Pro-41,** Gly-42, Lys-43 |
| Group III | **Asp-62,** Lys-65 |
| Group IV | Arg-87, Ala-88, **Glu-89** |
| Group V | **Gln-112, Leu-115, Thr-117,** Ser-119, **Ser-120** |
| Vk | |
| Group I | Val-15, |
| Group II | Pro-40, Gly-41 |
| Group III | Ser-56, Gly-57, Ser-60 |
| Group IV | Pro-80, Glu-81 |
| Group V | Gln-100, Lys-107, Arg-108 |

The primary amino acid sequence of the VH dAb HEL4 (SEQ ID NO: 5)

```
  1  EVQLLESGGG LVQPGGSLRL SCAASGFRIS DEDMGWVRQA PGKGLEWVSS
 51  IYGPSGSTYY ADSVKGRFTI SRDNSKNTLY LQMNSLRAED TAVYYCASAL
101  EPLSEPLGFW GQGTLVTVSS
```

The primary amino acid sequence of Vk dummy (SEQ ID NO: 6)

```
  1  DIQMTQSPSS LSASVGDRVT ITCRASQSIS SYLNWYQQKP GKAPKLLIYA
 51  ASSLQSGVPS RFSGSGSGTD FTLTISSLQP EDFATYYCQQ SYSTPNTFGQ
101  GTKVEIKR
```

### 12.1 Quick-change mutagenesis of TAR2-10-27 to introduce surface cysteines for site specific PEGylation

Quick-change mutagenesis (Stratagene) was used to substitute a surface residue with a single cysteine for site specific PEGylation. Quick-change mutagenesis was carried out following the manufacturer's protocol, using wild type TAR2-10-27 DNA as the template and the appropriate primer pairs (Table 4). Positive clones were identified by DNA sequencing, and each mutant dAb expressed and purified as described in Section 2.2. PEGylation and purification of the formatted proteins was carried out as described in Section 2.3

Table 4: Oligonucleotide pairs used for Quick-change mutagenesis of TAR2-10-27

| Point mutation | SEQ ID NO | Oligonucleotide sequence 5' to 3' |
|---|---|---|
| Gln13cys<br>Forward primer<br>Reverse primer | <br>38<br>39 | <br>TCTGGGGGAGGCTTGGTATGCCCTGGGGGGTCCCTGCGT<br>ACGCAGGGACCCCCCAGGGCATACCAAGCCTCCCCCAGA |
| Pro41cys<br>Forward primer<br>Reverse primer | <br>40<br>41 | <br>ATGGGTTGGGTCCGCCAGGCTTGCGGGAAGGGTCTAGAGTGG<br>CCACTCTAGACCCTTCCCGCAAGCCTGGCGGACCCAACCCAT |
| Asp62cys<br>Forward primer<br>Reverse primer | <br>42<br>43 | <br>GGTAGCACATACTACGCATGCTCCGTGAAGGGCCGGTTC<br>GAACCGGCCCTTCACGGAGCATGCGTAGTATGTGCTACC |
| Glu89cys<br>Forward primer<br>Reverse primer | <br>44<br>45 | <br>ATGAACAGCCTGCGTGCCTGCGACGCCGCGGTATATTAC<br>GTAATATACCGCGGCGTCGCAGGCACGCAGGCTGTTCAT |
| Gln112cys<br>Forward primer<br>Reverse primer | <br>46<br>47 | <br>CCTAATTTTGGCTACCGGGGCTGCGGAACCCTGGTCACCGTC<br>GACGGTGACCAGGGTTCCGCAGCCCCGGTAGCCAAAATTAGG |
| Leu115cys<br>Forward primer<br>Reverse primer | <br>48<br>49 | <br>GGCTACCGGGGCCAGGGAACCTGCGTCACCGTCTCGAGCTAA<br>TTAGCTCGAGACGGTGACGCAGGTTCCCTGGCCCCGGTAGCC |
| Thr117cys<br>Forward primer<br>Reverse primer | <br>50<br>51 | <br>CGGGGCCAGGGAACCCTGGTCTGCGTCTCGAGCTAATAAGGA<br>TCCTTATTAGCTCGAGACGCAGACCAGGGTTCCCTGGCCCCG |

Each one of the TAR2-10-27 cys mutants was expressed and purified and the final yield of protein shown in Table 5.

Table 5: Final purification yields of surface cys mutants of TAR2-10-27

| Mutant | Final expression yield (mg/L culture) | Expression relative to Ser120cys |
|---|---|---|
| Gln13cys | 1.10 | 2.2 |
| Pro41cys | 0.86 | 1.7 |
| Asp62cys | 0.24 | 0.5 |
| Glu89cys | 0.93 | 1.9 |
| Gln112cys | 0.87 | 1.7 |
| Leu115cys | 2.65 | 5.3 |
| Thr117cys | 1.00 | 2.0 |
| Ser120cys | 0.50 | 1.0 |

It can be seen from Table 5 that the expression levels of each mutant dAb does vary significantly depending on the position of the cysteine on the surface of the protein. For example, the mutant Leu115cys has a ~5.3 times greater expression level than Ser120cys. Therefore the position of the PEGylation site can have a significant effect on the expression levels *in vivo.* To see if the position of the PEGylation site had affected the affinity of the dAb for its antigen,

each mutant was PEGylated with 30K-MAL PEG and purified by ion exchange chromatography. As a control, each mutant had the surface cysteine blocked with N-ethylmaleimide to produce monomeric protein. All proteins were put through a DOM1 receptor binding assay (RBA) as well as a MRC-5 cell assay and the results are shown in Table 6.

Table 6: Summary of the RBA and cell assay data for TAR210-27 cys surface mutants. Blocked proteins were generated using N-ethylmaleimide.

| TAR2-10-27 cys mutant | Receptor assay | MRC5 cell assay |
|---|---|---|
| | IC 50 (nM) | ND 50 (nM) |
| Wild type TAR2-10-27 | 3 | 30 |
| Gln13cys blocked | 2 | 30 |
| Pro41cys blocked | 1 | 45 |
| Asp62cys blocked | 15 | 430 |
| Glu89cys blocked | 3 | 70 |
| Gln112cys blocked | 2 | 15 |
| Leu115cys blocked | 3 | 30 |
| Thr117cys blocked | 3 | 30 |
| Ser120cys blocked | 3 | 30 |
| Gln13cys 30K MAL PEG | 8 | 200 |
| Pro41cys 30K MAL PEG | 8 | 200 |
| Asp62cys 30K MAL PEG | >300 | 1000 |
| Glu89cys 30K MAL PEG | 20 | 100 |
| Gln112cys 30K MAL PEG | 15 | 650 |
| Leu115cys 30K MAL PEG | 10 | 300 |
| Thr117cys 30K MAL PEG | 8 | 700 |
| Ser120cys 30K MAL PEG | 10 | 600 |

It can be clearly seen that the location of the PEGylation site does indeed have a significant effect on the affinity of the dAb for its antigen. The best site for PEGylation appears to be at either position Gln13, Pro41 or Leu115 (ND 50's of ~200-300 nM). All of these constructs seem to maintain a relatively high affinity for the antigen compared to direct C-terminal PEGylation at Ser120cys (ND 50 of 600 nM). Therefore selecting an internal PEGylation site rather than a C-terminal one may be more favourable on the grounds that; (i) protein expression is significantly higher and (ii) antigen binding is less affected.

**Example 13: Hydrodynamic size correlation to the serum *in vivo* half life of VH and Vk PEGylated dAbs**

The native molecular mass of the various PEGylated VH and VL dAbs was determined using gel filtration chromatography. A Superose 6HR column (Amersham Biosciences) was equilibrated with 5 column volumes of PBS using an AKTA 100 system (Amersham Biosciences). The column was calibrated using high and low molecular weight protein calibration kits (Amersham Biosciences), following the manufactures instructions. 100 μl of PEGylated protein (1.5 mg/ml) was applied to the column at a flow rate of 0.5 ml/min. The elution volume of the protein was determined by following the absorption at 280 nm. The process was repeated for each PEGylated dAb to determine their individual elution volumes. A calibration curve for the column was generated by plotting the log of the molecular mass vs the $K_{av}$, where $L_{av}$ is:

$$K_{av} = (V_e - V_0) / (V_t - V_0)$$

$V_e$ = elution volume of protein (ml)
$V_0$ = void volume (blue dextran elution volume [7.7 ml])
$V_t$ = total column volume (acetone elution volume 22 ml)

The native molecular mass of each PEGylated dAb was determined by converting the elution volume to a $K_{av}$. The calibration curve plot of $K_{av}$'s vs log of the molecular weight standards was used to extrapolate the mass of the PEGylated proteins.

From the calibration curve of the gel filtration column, the estimated size of the various PEGylated dAbs was estimated and is shown in Table 7. The *in vivo* serum half-life of all of the formats was also determined in mice.

Table 7: Estimations of the hydrodynamic size and *in vivo* serum half-lives of Vk dAbs. Notes: [a] estimated size determined by SDS-PAGE. [b]hydrodynamic sizes as determined by Superose 6HR (Amersham Biosciences) size exclusion chromatography.

| Format of Vk dAb | Size of PEG Linear/ branched | Estimated size (kDa)[3] | Hydrodynamic size (kDa)[b] | *In vivo* serum half-life ($t_{½}\beta$ hrs) |
|---|---|---|---|---|
| TAR1-5-19 monomer | na | 12 | 19.5 | 0.5 |
| TAR1-5-19 disulphide dimer | na | 24 | 22 | 1.2 |
| TAR1-5-19 Fc fusion | na | 80 | na | >24 |
| TAR1-5-19 monomer 30K PEG-MAL | 30K linear | 70 | 1100 | 38.5 |
| TAR1-5-19 monomer 40K PEG-MAL | 40K branched (2x20K) | 95 | 1700 | >36 |
| TAR1-5-19 dimer 40K PEG | 40K branched (2x20K) | 110 | 1990 | 39.5 |
| TAR1-5-19 tetramer PEG MAL | 20K 4-arm branched (4x5K) | 90 | 200 | 12.7 |
| TAR1-5-19 tetramer PEG MAL | 40K 4-arm branched (4x10K) | >110 | 570 | 26.4 |

What clearly can be seen from Table 7 is that the hydrodynamic size of the dAb can be significantly increased by the addition of PEG. Also, not only the size of the PEG, but the structure of the polymer can greatly affect the hydrodynamic size. For example, the 2x20K and the 4x10K have the same PEG content, but the 2x20K is ~3 times the hydrodynamic size of the 4x10K. This can be attributed to the fact that the PEG in the 4x10K format is more densely packed at the core of the molecule compared to the 2x20K PEG, thus significantly reducing its hydrodynamic size. What is also clear is that there is a relationship between the native hydrodynamic mass of the protein and the in vivo serum half-life (see figure 15). Thus the hydrodynamic size as determined by gel filtration chromatography can be used to give an estimated serum half-life. Irrespective of the format and size of the PEG used to modify the dAb (i.e. linear vs branched), it can be seen that the hydrodynamic size is a better indicator of serum half-life than total mass of polymer used to modify the protein.

Thus the hydrodynamic size as determined by gel filtration chromatography can be used to give an estimated serum half-life using figure 15.

**Example 14: Protease stability of PEGylated dAbs**

The protease stability of unmodified and PEGylated protein was investigated by following antigen binding by ELISA. Due to the lower signal generated by PEGylated protein in ELISA, a significantly higher concentration of dAb had to be used. 0.4 μM (5 μg/ml) monomer and 25 μM (300 μg/ml) 40K PEGylated TAR1-5-19 were digested with 250 μg/ml of protease in a final volume of 100 μl for all proteases. The proteases used were porcine pepsin, bovine crude mucosa peptidase, porcine pancreatic elastase, crude bovine pancreatic protease (type I) and rat intestinal powder (Sigma). All digests were carried out in 50 mM Tris buffer pH 8.0, with the exception of pepsin which was done in 20 mM HCl pH 2. Each digest was incubated at 37 ˚C for 30 mins and the reaction then terminated by the addition of 10 μl of a 10x stock solution of Complete protease inhibitors (Roche). Samples were then kept on ice until required. A Maxisorb plate which had been previously coated overnight at 4 ˚C with 1μg/ml of TNF in PBS, was blocked with 2 % Tween-20 in PBS (2% TPBS) for one hour at room temperature. 20 μl of each protease test sample was diluted to 200 μl using 2% TPBS. The samples were then transferred to the antigen coated plated and incubated at room temperate

for one hour. The plate was then washed with 0.1% TPBS before adding 100 μl per well of Protein L-HRP solution (Sigma) (diluted 1:2000 in 2% TPBS) and incubated for one hour. The plate was again washed with 0.1% TPBS and then PBS solution before developing the plate. 100 μl of TMB solution per well was added and allowed to develop before the addition of 100 μl of 1M HCl to terminated the reaction. The level of bound dAb present was indirectly determined by measuring the absorbance at 450 nm using a Versamax plate-reader (Molecular devices). The percentage of functional protein remaining after protease treatment was determined from the A450 nm measurement relative to the no protease control.

The protease stability of the monomeric and 40K PEG TAR1-5-19 against the various proteases is shown in figure 16. It can be seen that even the unmodified monomeric dAb does show a degree of protease resistance, only showing a ~50-70% loss in antigen binding with peptidase, elastase and rat intestinal powder, 90% loss with CBP and a total loss in activity with pepsin and CBP. The protease resistance may be due to the compact protein fold of the dAb, which in turn may reduce the ability of proteases from gaining access to the peptide backbone. When compared to the unmodified dAb, the 40K PEGylated TAR1-5-19 does show a greater degree of stability to all the proteases except pepsin. The increase stability may be due to the surface modification of the dAb with such a large mobile polymer. The surface PEG may "coat" the protein, preventing the proteases from binding and cleaving the peptide backbone, thus enhancing the dAbs stability to these enzymes.

The reason for the total degradation of both formats to pepsin may be attributed to the low pH denaturing conditions to which the dAbs are exposed to during the assay. The lower pH would cause protein unfolding which would allow greater access to the peptide backbone for cleavage by pepsin.

**Example 15: $k_d$ and $k_a$ rate analysis of PEGylated TAR2-10-27 using Biacore**

The effect of PEGylation on the binding affinity of TAR2-10-27 for its antigen was investigated using Biacore. All the PEGylated formats were generated using TAR2-10-27cys (see Example 2). The following methodology was used to determine the on and off rates of each PEGylated dAb format for the antigen. Biotinylated human TNFRI (approx 1 biotin per molecule) was coated onto a single flow cell of a Biacore streptavidin sensor chip at a density of approximately 400 RU. A concentration series of each dAb or PEGylated dAb (2.6 μM, 770 nM, 260 nM, 77 nM and 2.6 nM) was sequentially injected (45μl injection at a flow rate of 30μl/min) over both the TNFRI coated flow cell and an uncoated flow cell (streptavidin), (a subtractive curve was generated by subtracting the uncoated flow cell curve from the TNFRI flow cell curve). The off-rate was analysed for five minutes after the injection of each sample after which the surface was regenerated by injection of 10mM glycine pH 3.

Data generated from the Biacore was fitted using the curve fitting software (Biaevaluation 3.2) and the association constant ($k_a$) and dissociation constant ($k_d$) determined (Table 8).

Table 8: On and off rate analysis of TAR210-27 in various PEGylated formats. Blocked TAR210-27 was generated using N-ethylmaleimide.

| Format | $k_a$ ($M^{-1} S^{-1}$) | $k_d$ ($S^{-1}$) | $K_D$ (from mean $k_d/k_a$) (M) |
|---|---|---|---|
| TAR210-27 | 1.60E+06 | 8.52E-03 | 5.33E-09 |
| TAR210-27 blocked | 5.77E+05 | 5.96E-03 | 1.03E-08 |
| TAR210-27 5K MAL PEG | 9.00E+04 | 0.011 | 1.22E-07 |
| TAR210-27 2x10K MAL PEG | 1.20E+05 | 9.54E-03 | 7.95E-08 |
| TAR210-27 20K MAL PEG | 7.99E+04 | 0.012 | 1.50E-07 |
| TAR210-27 30K MAL PEG | 8.34E+04 | 7.69E-03 | 9.22E-08 |
| TAR210-27 2x20K MAL PEG | 7.47E+04 | 5.17E-03 | 6.92E-08 |

From Table 8 it can be seen that as the size of the PEG chain increases the $K_D$ decreases. This change in the $K_D$ in mainly due to a decrease in $k_a$, with $k_d$ remaining relatively unchanged as the size of the PEG increases.

**Example 16: Efficacy study of PEGylated TAR1-5-19 in a prophylactic model of arthritis.**

Tg197 mice are transgenic for the human TNF-globin hybrid gene and heterozygotes at 4-7 weeks of age develop a chronic, progressive polyarthritis with histological features in common with rheumatoid arthritis [Keffer, J., Probert, L.,Cazlaris, H., Georgopoulos, S.,Kaslaris, E., Kioussis, D., Kollias, G. (1991). Transgenic mice expressing human tumor necrosis factor: a predictive genetic model of arthritis. EMBO J., Vol. 10, pp. 4025-4031.]

To test the efficacy of a PEGylated dAb (PEG format being 2x20k branched with 2 sites for attachment of the dAb [i.e. 40K mPEG2 MAL2], the dAb being TAR1-5-19cys) in the prevention of arthritis in the Tg197 model, heterozygous transgenic mice were divided into groups of 10 animals with equal numbers of male and females. Treatment commenced at 3 weeks of age with weekly intraperitoneal injections of test items. The expression and PEGylation of TAR1-5-19cys monomer is outlined in Section 1.3.3, example 1. All protein preparations were in phosphate buffered saline and were tested for acceptable levels of endotoxins.

The study was performed blind. Each week the animals were weighed and the macrophenotypic signs of arthritis scored according to the following system: 0 = no arthritis (normal appearance and flexion), 1 = mild arthritis (joint distortion), 2 = moderate arthritis (swelling, joint deformation), 3 = heavy arthritis (severely impaired movement).

The outcome of the study clearly demonstrated that 10mg/kg PEGylated TAR1-5-19 inhibited the development of arthritis with a significant difference between the arthritic scoring of the saline control and treated group. The 1mg/kg dose of PEGylated TAR1-5-19 also produced a statistically significantly lower median arthritic score than saline control group (P<0.05% using normal approximation to the Wilcoxon Test).

**Example 18: Efficacy study of PEGylated TAR1-5-19 in a therapeutic model of arthritis.**

To test the efficacy of a PEGylated dAb in the therapeutic model of arthritis in the Tg197 model, heterozygous transgenic mice were divided into groups of 10 animals with equal numbers of male and females. Treatment commenced at 6 weeks of age when the animals had significant arthritic phenotypes. Treatment was twice weekly with 4.6mg/kg intraperitoneal injections of test items. The sample preparation and disease scoring are as described above in example 17.

The arthritic scoring clearly demonstrated that PEGylated TAR1-5-19 inhibited the progression of arthritis in a therapeutic model. The 4.6mg/kg dose of PEGylated TAR1-5-19 produced a statistically significantly lower median arthritic score than saline control group at week 9 (P<0.01% using normal approximation to the Wilcoxon Test).

**Example 19: dAb Efficacy in a Slow Release Format**

To test the efficacy of a dAb from a slow release format, a dAb with a small PEG molecule (where the PEG is 4x5k with four sites for attachment of a dAb with a C-terminal cys residue, the dAb being TAR1-5-19 [i.e. 20K PEG 4 arm MAL]) was loaded into a 0.2 ml osmotic pump. The pump had a release rate of 0.2 ml over a 4 week period was implanted subcutaneously into mice at week 6 in the therapeutic Tg197 model as described above. The arthritic scores of these animals increased at a clearly slower rate when compared to animals implanted with pumps loaded with saline. This demonstrates that dAbs are efficacious when delivered from a slow release format.

**Example 20: PEGylation of VH and VL dAbs using TCEP as a reducing agent**

Methods outlined in Example 1.3 use dithiothreitol (DTT) to reduce the surface thiol on the dAb prior to MAL PEGylation. This method does require the removal of the reducing agent by gel filtration or dialysis before PEGylation as DTT will react rapidly with maleimide-PEG, even at low pH, preventing the formation of the polymer-dAb conjugate. An alternative method is to use a reducing agent such as TCEP. TCEP can be used at lower concentrations, due to its reduction potential, and also reacts relatively slowly with free maleimide, preferentially reducing thiols. Thus the surface thiol present on the dAb may be reduced with TCEP and the MAL-PEG added directly to the reaction mixture. There is no need to remove the TCEP by gel filtration prior to PEGylation. It is also possible to carry out repeated cycles of TCEP reduction followed by MAL-PEG additions to significantly increase yields of PEGylated dAb.

**Example 21: N terminal PEGylation of VH and VL dAbs**

An alternative method of PEGylation of VH and VL dAbs is via N-terminal PEGylation. This can be achieved in two ways, firstly using PEG-aldehyde, which under the correct reaction conditions can be used to selectively modify the N-terminal amine of a protein. This can be achieved due to the fact that the N-terminus amine has a relatively low $pK_a$ of ~6.5. Secondly, a cysteine residue may be engineered at the N-terminus of the protein for site specific PEGylation using MAL-PEG. Again the protein would be reduced using TCEP or DTT (see example 1.3), prior to coupling to PEG-MAL of the desired size.

**Claims**

1. A PEG-linked polypeptide comprising an antibody single variable domain, wherein the variable domain comprises a framework region and has an antigen binding site, and said variable domain binds antigen as a single antibody variable domain in the polypeptide, and further wherein said PEG is present as one or more molecules linked to the polypeptide at a cysteine or lysine residue in the framework regions of said antibody single variable domain.

2. A PEG-linked polypeptide according to claim 1, having a hydrodynamic size of at least 24kDa, the hydrodynamic size being determined using a Superose 6 HR or 12 HR gel chromatography column, a polypeptide concentration of 1.5 mg/ml, and a flow rate of 0.5ml/min.

3. A PEG-linked polypeptide according to claim 1 or 2, wherein the total PEG size is from 20 to 60 kDa.

4. A PEG-linked polypeptide according to any one of the preceding claims, comprising a multimer of antibody single variable domains.

5. A PEG-linked polypeptide according to any one of the preceding claims, wherein said variable domain comprises a universal framework.

6. A PEG-linked polypeptide according to claim 5, wherein each variable domain comprises a $V_H$ framework selected from the group consisting of DP47, DP45 and DP38; or a $V_L$ framework is DPK9.

7. A PEG-linked polypeptide according to claim 1, wherein said polypeptide has specificity for TNF$\alpha$.

8. A PEG-linked polypeptide according to claim 1, wherein said PEG is linked to said antibody single variable domain at a cysteine or lysine residue present in the variable domain framework region other than at the C-terminus or N-terminus of said antibody single variable domain.

9. A PEG-linked polypeptide according to claim 1, wherein said PEG is linked to said antibody single variable domain at a cysteine or lysine residue at position 22 or 65 or a lysine residue spaced at least two residues away from the C- and/or N-terminus.

10. A PEG-linked polypeptide according to claim 1, wherein said polypeptide comprises a DP47 $V_H$ framework linked to PEG at a cysteine residue at position 22 or 65, or a lysine residue at one or more positions selected from the group consisting of: 43, 65, 76 and 98.

11. A PEG-linked polypeptide according to claim 1, wherein said polypeptide comprises a DPK9 $V_K$ framework linked to PEG at a cysteine residue at position 23 or 88 or a lysine residue at one or more positions selected from the group consisting of: 39, 42, 45, 103 and 107.

12. A PEG-linked polypeptide according to claim 1, comprising a $V_H$ dAb having PEG linked to a cysteine or lysine residue at one or more positions selected from the group consisting of: 13, 14, 15, 41, 42, 43, 62, 65, 87, 88, 89, 112, 115, 117, 119 and 120.

13. A PEG-linked polypeptide according to claim 1, comprising a $V_K$ dAb having PEG linked to a cysteine or lysine residue at one or more positions selected from the group consisting of: 15, 40, 41, 56, 57, 60, 80, 81, 100, 107 and 108.

14. A PEG-linked polypeptide according to claim 1, wherein said PEG is linked to a heavy chain variable domain comprising a cysteine or lysine residue substituted at a position selected from the group consisting of Gln13, Pro41 or Leul15.

15. A PEG-linked polypeptide according to claim 1, having a half life between 1.3 and 170 hours.

16. A PEG-linked multimer according to claim 4, wherein said multimer is a dimer, trimer or tetramer of antibody single variable domains.

17. A PEG-linked polypeptide according to claim 4, comprising a homomultimer of antibody single variable domains.

**18.** A PEG-linked polypeptide according to claim 17, wherein said homomultimer comprises only a first and second antibody single variable domain, wherein said first antibody single variable domain of said homodimer comprises an antibody single variable domain and a heavy chain (CH1) constant region, and wherein said second antibody single variable domain of said homodimer comprises an antibody single variable domain and a light chain (CL) constant region.

**19.** A PEG-linked polypeptide according to claim 4, comprising a heteromultimer of antibody single variable domains.

**20.** A PEG-linked polypeptide according to claim 19, wherein said heteromultimer comprises only a first and second antibody single variable domain, wherein said first antibody single variable domain of said heteromultimer comprises an antibody single variable domain and a heavy chain (CH1) constant region, and wherein said second antibody single variable domain of said heteromultimer comprises an antibody single variable domain and a light chain (CL) constant region.

**21.** A PEG-linked polypeptide according to any one of the preceding claims wherein said PEG moiety is a branched PEG.

**22.** A PEG-linked polypeptide according to any one of the preceding claims, which dissociates from human TNFα with a dissociation constant ($K_d$) of 50nM to 20pM, and a $K_{off}$ rate constant of 5 x $10^{-1}$ to 1 x $10^{-7}$ $s^{-1}$, as determined by surface plasmon resonance.

**23.** A PEG-linked polypeptide according to any one of the preceding claims, wherein said PEG is linked to a solvent-accessible lysine in the form of a PEG linked N-hydroxylsuccinimide active ester.

**24.** A PEG-linked polypeptide according to any one of claims 1-22, wherein said PEG is linked to a solvent-accessible cysteine by a sulfhydryl-selective reagent selected from the group consisting of maleimide, vinyl sulfone, and thiol.

**25.** A PEG-linked polypeptide according to claim 33 or 34, wherein the PEG is provided as a single polymer.

**26.** A PEG-linked polypeptide according to any one of the preceding claims which has binding specificity for one of the following:

ApoE, Apo-SAA, BDNF, Cardiotrophin-1, EGF, EGF receptor, ENA-78, Eotaxin, Eotaxin-2, Exodus-2, FGF-acidic, FGF-basic, fibroblast growth factor-10, FLT3 ligand, Fractalkine (CX3C), GDNF, G-CSF, GM-CSF, GF-β 1, insulin, IFN-γ, IGF-I, IGF-II, IL-1α, IL-1β, IL-2, IL-3, IL-4, IL-5, IL-6, IL-7, IL-8 (72 a.a.), IL-8 (77 a.a.), IL-9, IL-10, IL-11, IL-12, IL-13, IL-15, IL-16, IL-17, IL-18 (IGIF), Inhibin α, Inhibin β, IP-10, Keratinocyte growth factor-2 (KGF-2), KGF, Leptin, LIF, Lymphotactin, Mullerian inhibitory substance, monocyte colony inhibitory factor, monocyte attractant protein, M-CSF, MDC (67 a.a.), MDC (69 a.a.), MCP-1 (MCAF), MCP-2, MCP-3, MCP-4, MDC (67 a.a.), MDC (69 a.a.), MIG, MIP-1a, MIP-1β, MIP-3α, MIP-3β, MIP-4, myeloid progenitor inhibitor factor-1 (MPIF-1), NAP-2, Neurturin, Nerve growth factor, β-NGF, NT-3, NT-4, Oncostatin M, PDGF-AA, PDGF-AB, PDGF-BB, PF-4, RANTES, SDF1α, SDF1β, SCF, SCGF, stem cell factor (SCF), TARC, TACE recognition site, TGF-α, TGF-β, TGF-β2, TGF-β3, tumor necrosis factor (TNF), TNF-α, TNF-β, TNF receptor I (p55), TNF receptor II, TNIL-1, TPO, VEGF, VEGF receptor 1, VEGF receptor 2, VEGF receptor 3, GCP-2, GRO/MGSA, GRO-β, GRO-γ, HCC1, 1-309, HER 1, HER2, HER 3, HER 4, IL-1R, IL-6R, IL-10R and IL-18R.

**27.** A PEG-linked polypeptide according to claim 19, which polypeptide has dual binding specificity and binds a target selected from the group consisting of the following pairs:

TNF/TGF-β, TNF/IL-1, TNF/IL-2, TNF/IL-3, TNF/IL-4, TNF/IL-6, TNF/IL-8, TNF/IL-10, TNF/IL-12, TNF/IFN-γ, IL-I/IL-8, IL-I/IL-10, IL-1/IFN-γ, IL-2/IL-3, IL-2/IL-4, IL-2/IL-5, IL-2/IL-6, IL-2/IL-7, IL-2/IL-10, IL-2/IL-12, IL-2/IL-15, IL-2/IFN-γ, IL-2/IFNα/β, IL-3/IL-4, IL-3/IL-5, IL-3/IL-6, IL-3/IFN-γ, IL-4/IL-5, IL-4/IL-6, IL-4/IL-10, IL-4/IL-12, IL-4/IL-13, IL-4/IFN-γ, IL-4/SCF, IL-5/IL-6, IL-5/IFN-γ, IL-6/IL-10, IL-6/IL-11, IL-6/IFN-γ, IL-10/IL-12, IL-10/IFN-γ, IL-12/IL-18, IL-12/IFN-γ, IL-18/IFN-γ, IL-18/IFN-γ and anti-TNF/anti-CD4.

**28.** A pharmaceutical formulation comprising a PEG-linked polypeptide according to any one of the preceding claims; and a carrier.

**29.** A pharmaceutical formulation according to claim 28, wherein said pharmaceutical formulation is suitable for oral administration or is suitable for parenteral administration via a route selected from the group consisting of intravenous,

intramuscular or intraperitoneal injection, implantation, rectal and transdermal administration.

**30.** A pharmaceutical formulation according to claim 29, wherein said pharmaceutical formulation is an extended release parenteral or oral dosage formulation.

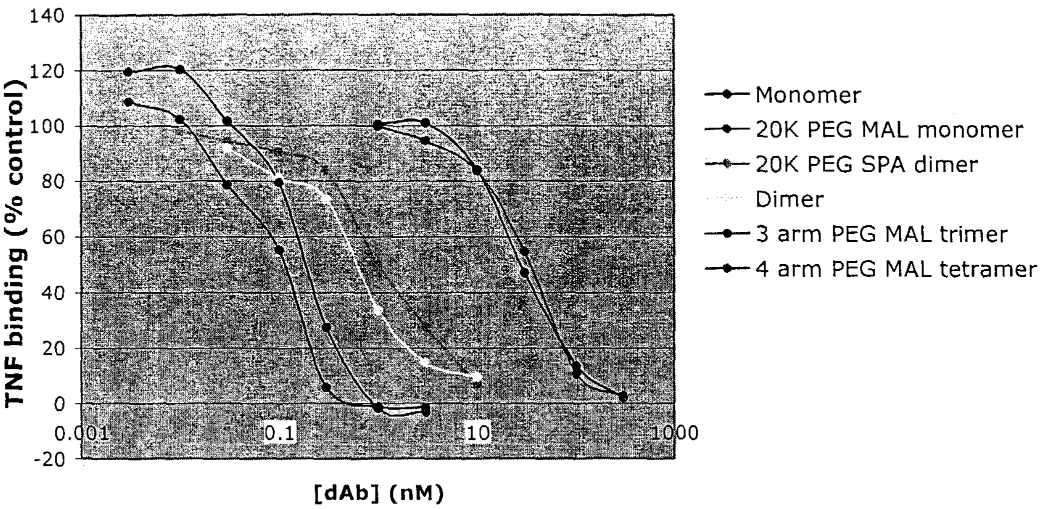


**Figure 1: Receptor binding assay showing the affinity of a range of PEGylated formats of TAR1-5-19**

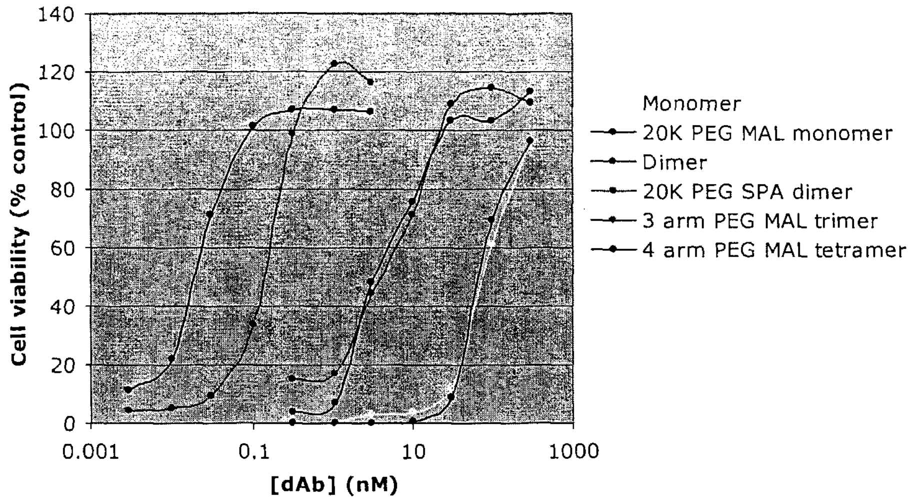


**Figure 2: Cell cytotoxcity assay showing the affinity of a range PEGylated formats of TAR1-5-19**

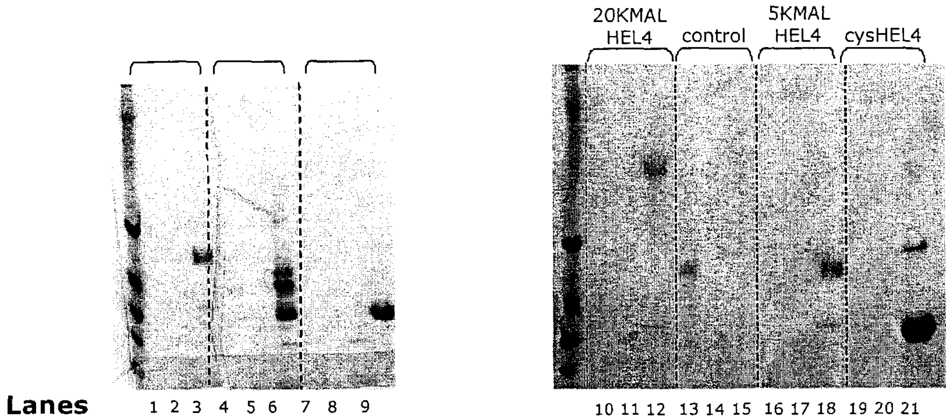


**Figure 3: SDS PAGE gels showing affinity binding to lysozyme. Lanes are as follows: 1, 4, 7, 10, 13, 16 and 19 protein remaining in the supernatant after batch binding for 30 mins; Lanes 2, 5, 8, 11, 14, 17 and 20 protein that may have eluted during PBS washing step; Lanes 3, 6, 9, 12, 15, 18 and 21 protein that was bound to the lysozyme affinity resin**

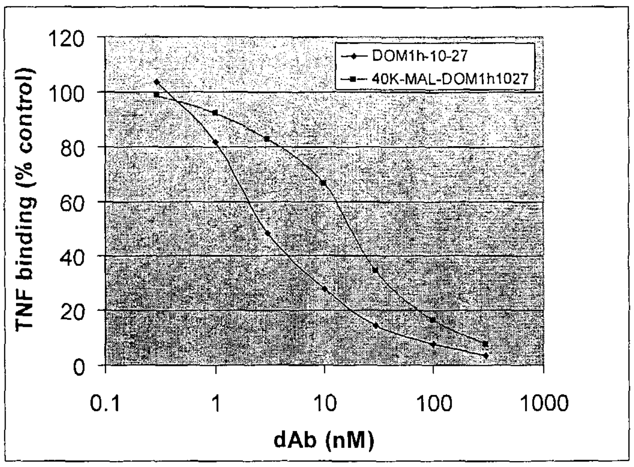


**Figure 4: Receptor binding assay showing the affinity of DOM1h-10-27 and the 40K PEGylated monomer.**

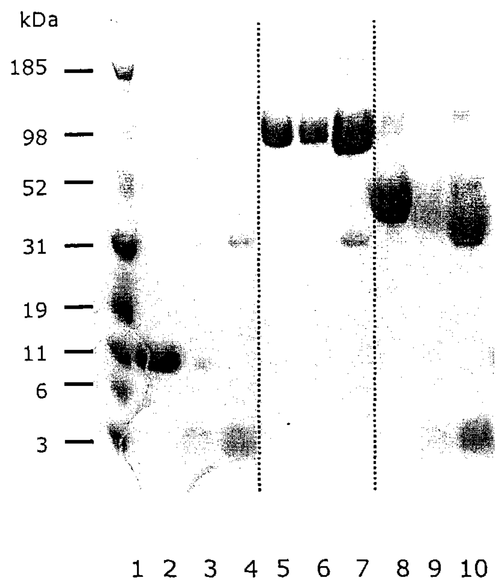


Reaction conditions:
pH 2.0, 250 μg/mL pepsin @ 37 °C

1-Mr
2-Monomer (0 mins)
3-Monomer (15 mins)
4-Monomer (30 mins)

5-40K MAL2 dimer (0 mins)
6-40K MAL2 dimer (15 mins)
7-40K MAL2 dimer (30 mins)

8-20K MAL monomer (0 mins)
9-20K MAL monomer (15 mins)
10-20K MAL monomer (30 mins)

**Figure 5: Gel showing the protease stability of TAR1-5-19 and PEGylated variants to the action of pepsin at pH 2.0.**

Figure 6

Monomer PEGylation of a VH or VL dAb

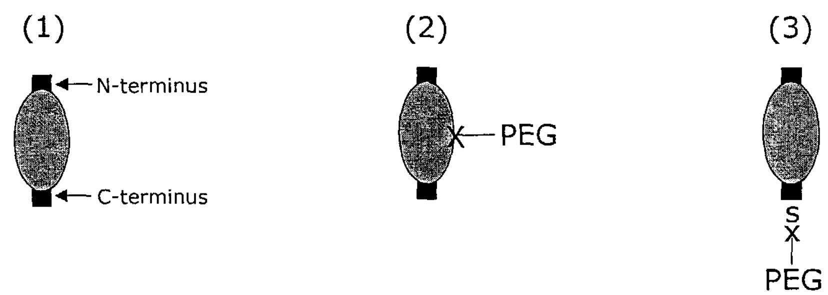


(1) Unmodified VH or VL dAb
(2) VH or VL dAb with surface PEGylation event
(3) VH or VL dAb with a C-terminal cysteine which can be PEGylated

X represents the chemical modification of an amino acid with a chemically activated PEG e.g NHS, SPA, MAL, VS, thiol etc. The amino acid residue that is modified maybe located anywhere on the surface of the dAb. PEG represents a linear/branched/forked or multi arm PEG. S represents the amino acid cysteine

Figure 7

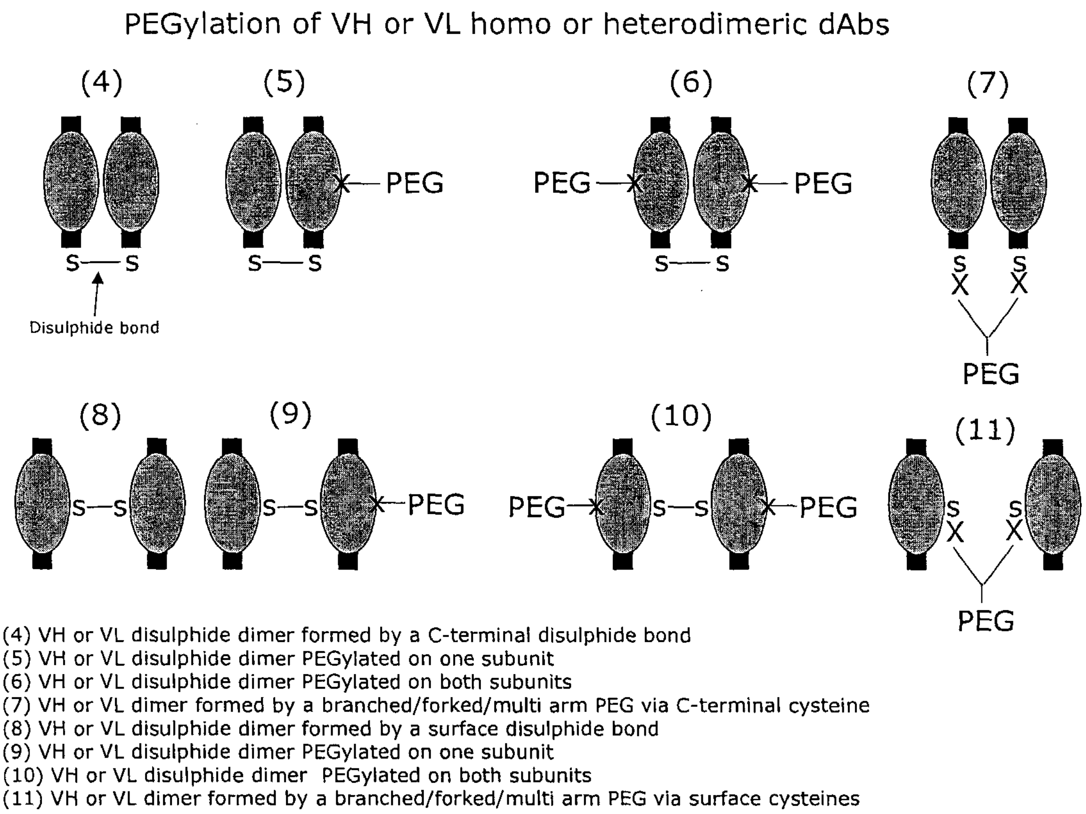


(4) VH or VL disulphide dimer formed by a C-terminal disulphide bond
(5) VH or VL disulphide dimer PEGylated on one subunit
(6) VH or VL disulphide dimer PEGylated on both subunits
(7) VH or VL dimer formed by a branched/forked/multi arm PEG via C-terminal cysteine
(8) VH or VL disulphide dimer formed by a surface disulphide bond
(9) VH or VL disulphide dimer PEGylated on one subunit
(10) VH or VL disulphide dimer PEGylated on both subunits
(11) VH or VL dimer formed by a branched/forked/multi arm PEG via surface cysteines

Figure 8

PEGylation of VH or VL homo or heterodimeric ultra affinity dimers

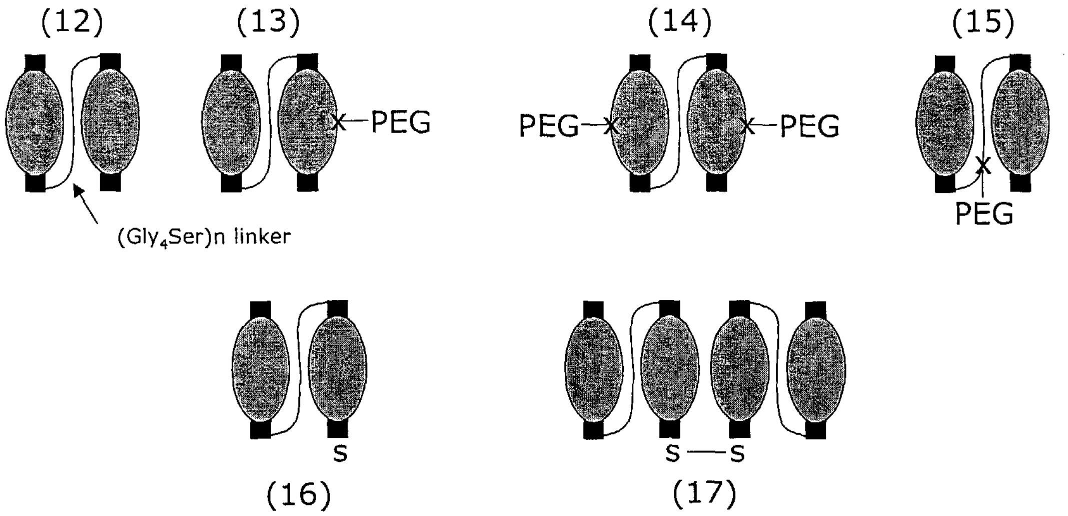


(12) VH or VL linker dimer formed by a $(Gly_4Ser)_n$ linker (n = 0-7)
(13) VH or VL linker dimer PEGylated on one subunit
(14) VH or VL linker dimer PEGylated on both subunits
(15) VH or VL linker dimer PEGylated via the linker
(16) VH or VL linker dimer with a C-terminal cysteine
(17) VH or VL linker disulphide dimer

Figure 9

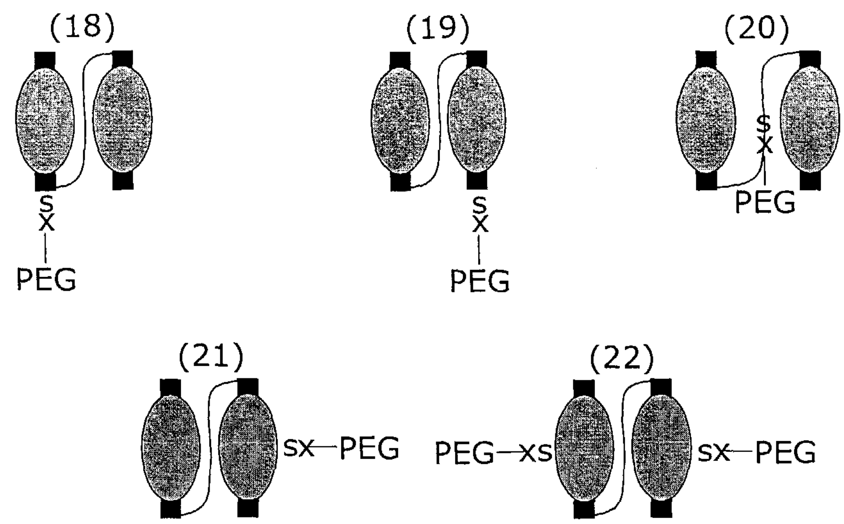


(18, 19) VH or VL linker dimer PEGylated via a C-terminal cysteine one one subunit
(20) VH or VL linker dimer PEGylated via a cysteine present in the linker
(21) VH or VL linker dimer PEGylated via a cysteine present on one subunit
(22) VH or VL linker dimer PEGylated via cysteines present on both subunits

Figure 10

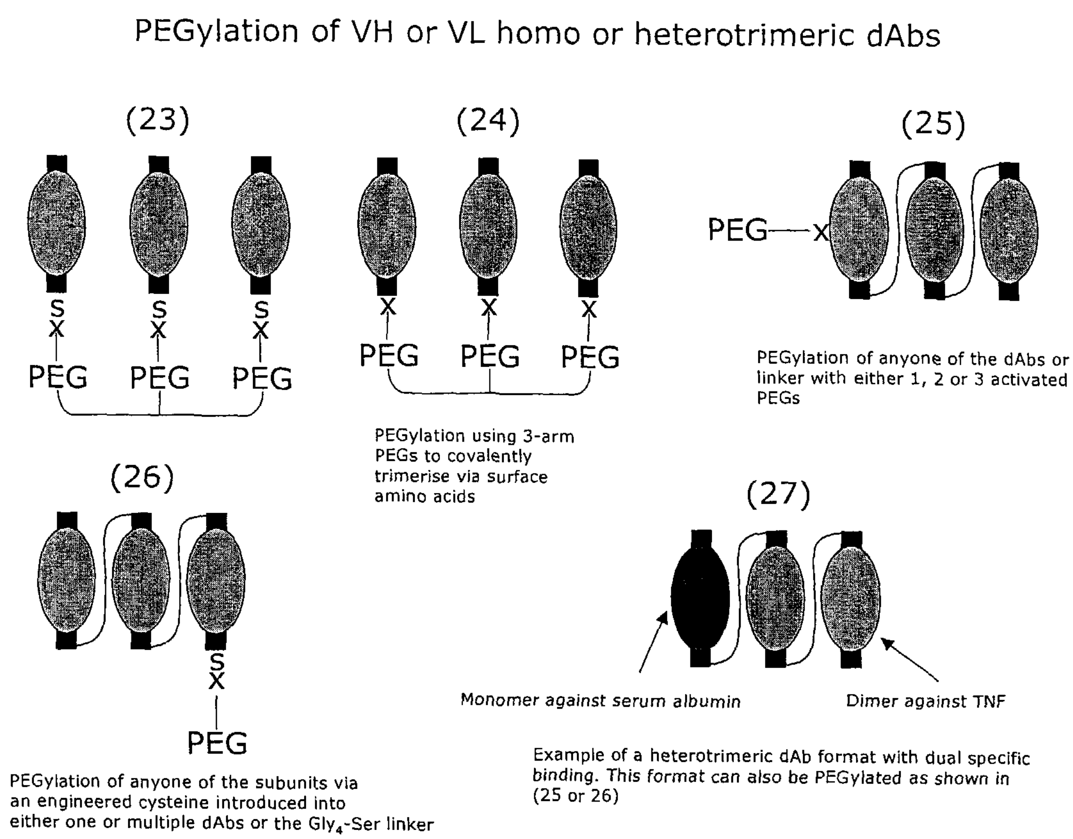
PEGylation of VH or VL homo or heterotrimeric dAbs
(23)
S
X
PEG
S
X
PEG
S
X
PEG
(24)
X
PEG
X
PEG
X
PEG
PEGylation using 3-arm PEGs to covalently trimerise via surface amino acids
(25)
PEG—X
PEGylation of anyone of the dAbs or linker with either 1, 2 or 3 activated PEGs
(26)
S
X
PEG
PEGylation of anyone of the subunits via an engineered cysteine introduced into either one or multiple dAbs or the Gly4-Ser linker
(27)
Monomer against serum albumin
Dimer against TNF
Example of a heterotrimeric dAb format with dual specific binding. This format can also be PEGylated as shown in (25 or 26)

Figure 11

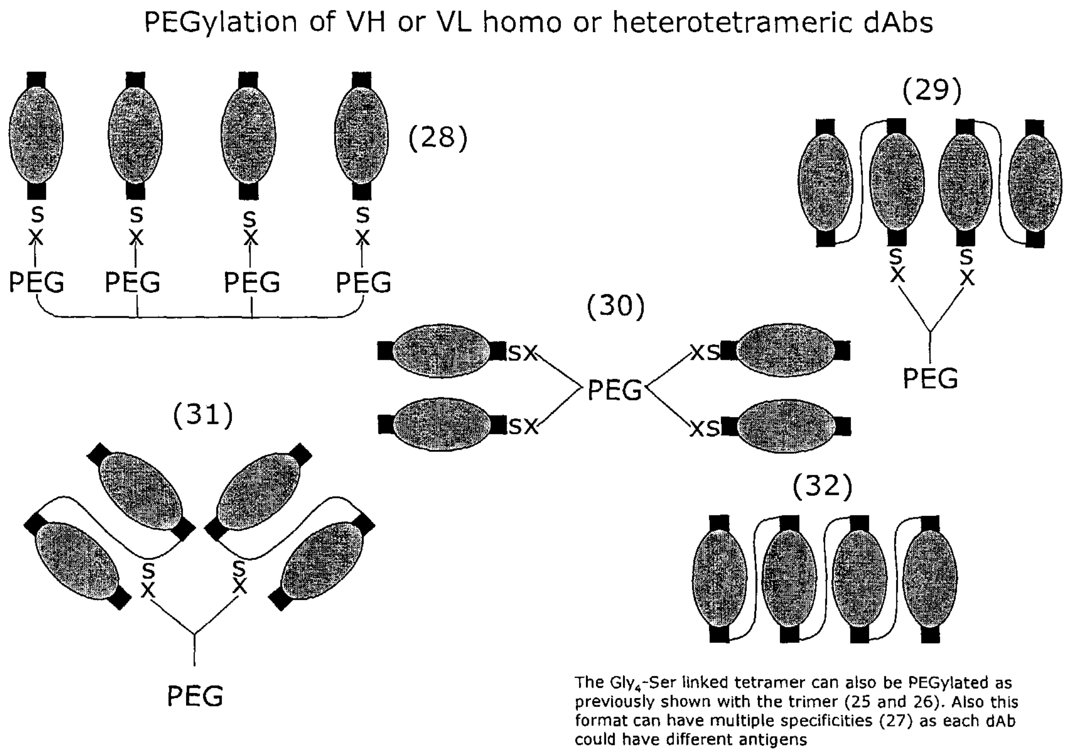


The $Gly_4$-Ser linked tetramer can also be PEGylated as previously shown with the trimer (25 and 26). Also this format can have multiple specificities (27) as each dAb could have different antigens

Using a VH or VL monomer, tetrameric formats can be generated as previously shown for the trimeric formats.

Figure 12

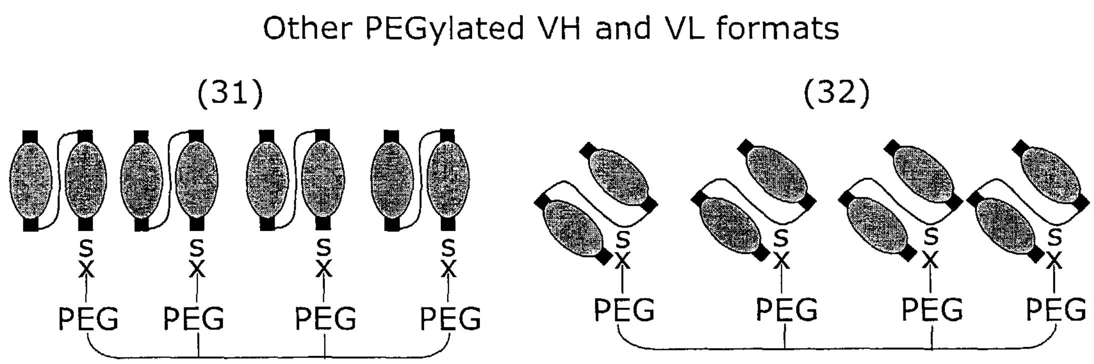


The trimer and tetrameric Gly4-Ser cys linked dAbs can be PEGylated using the multiarm PEG MALs to create multimeric formats

# Figure 13

```
     E   V   Q   L   L   E   S   G   G   G   L   V   Q   P   G   G
1    GAG GTG CAG CTG TTG GAG TCT GGG GGA GGC TTG GTA CAG CCT GGG GGG
     CTC CAC GTC GAC AAC CTC AGA CCC CCT CCG AAC CAT GTC GGA CCC CCC

     S   L   R   L   S   C   A   A   S   G   F   T   F   S   S   Y
49   TCC CTG CGT CTC TCC TGT GCA GCC TCC GGA TTC ACC TTT AGC AGC TAT
     AGG GAC GCA GAG AGG ACA CGT CGG AGG CCT AAG TGG AAA TCG TCG ATA
                                                             HCDR1
     A   M   S   W   V   R   Q   A   P   G   K   G   L   E   W   V
97   GCC ATG AGC TGG GTC CGC CAG GCT CCA GGG AAG GGT CTA GAG TGG GTC
     CGG TAC TCG ACC CAG GCG GTC CGA GGT CCC TTC CCA GAT CTC ACC CAG

     S   A   I   S   G   S   G   G   S   T   Y   Y   A   D   S   V
145  TCA GCT ATT AGT GGT AGT GGT GGT AGC ACA TAC TAC GCA GAC TCC GTG
     AGT CGA TAA TCA CCA TCA CCA CCA TCG TGT ATG ATG CGT CTG AGG CAC
                                     HCDR2
     K   G   R   F   T   I   S   R   D   N   S   K   N   T   L   Y
193  AAG GGC CGG TTC ACC ATC TCC CGT GAC AAT TCC AAG AAC ACG CTG TAT
     TTC CCG GCC AAG TGG TAG AGG GCA CTG TTA AGG TTC TTG TGC GAC ATA

     L   Q   M   N   S   L   R   A   E   D   T   A   V   Y   Y   C
241  CTG CAA ATG AAC AGC CTG CGT GCC GAG GAC ACC GCG GTA TAT TAC TGT
     GAC GTT TAC TTG TCG GAC GCA CGG CTC CTG TGG CGC CAT ATA ATG ACA
```

```
      A   K   S   Y   G   A   F   D   Y   W   G   Q   G   T   L   V
289   GCG AAA AGT TAT GGT GCT TTT GAC TAC TGG GGC CAG GGA ACC CTG GTC
      CGC TTT TCA ATA CCA CGA AAA CTG ATG ACC CCG GTC CCT TGG GAC CAG
                            HCDR3

      T   V   S   S
337   ACC GTC TCG AGC
      TGG CAG AGC TCG
```

# Figure 14

```
        D   I   Q   M   T   Q   S   P   S   S   L   S   A   S   V   G
  1    GAC ATC CAG ATG ACC CAG TCT CCA TCC TCC CTG TCT GCA TCT GTA GGA
       CTG TAG GTC TAC TGG GTC AGA GGT AGG AGG GAC AGA CGT AGA CAT CCT

        D   R   V   T   I   T   C   R   A   S   Q   S   I   S   S   Y
 49    GAC CGT GTC ACC ATC ACT TGC CGG GCA AGT CAG AGC ATT AGC AGC TAT
       CTG GCA CAG TGG TAG TGA ACG GCC CGT TCA GTC TCG TAA TCG TCG ATA
                                   ___________________________________
                                                   LCDR1
        L   N   W   Y   Q   Q   K   P   G   K   A   P   K   L   L   I
 97    TTA AAT TGG TAC CAG CAG AAA CCA GGG AAA GCC CCT AAG CTC CTG ATC
       AAT TTA ACC ATG GTC GTC TTT GGT CCC TTT CGG GGA TTC GAG GAC TAG
       _______

        Y   A   A   S   S   L   Q   S   G   V   P   S   R   F   S   G
145    TAT GCT GCA TCC AGT TTG CAA AGT GGG GTC CCA TCA CGT TTC AGT GGC
       ATA CGA CGT AGG TCA AAC GTT TCA CCC CAG GGT AGT GCA AAG TCA CCG
           ___________________________
                     LCDR2
        S   G   S   G   T   D   F   T   L   T   I   S   S   L   Q   P
193    AGT GGA TCT GGG ACA GAT TTC ACT CTC ACC ATC AGC AGT CTG CAA CCT
       TCA CCT AGA CCC TGT CTA AAG TGA GAG TGG TAG TCG TCA GAC GTT GGA

        E   D   F   A   T   Y   Y   C   Q   Q   S   Y   S   T   P   N
241    GAA GAT TTT GCT ACG TAC TAC TGT CAA CAG AGT TAC AGT ACC CCT AAT
       CTT CTA AAA CGA TGC ATG ATG ACA GTT GTC TCA ATG TCA TGG GGA TTA
                                       _______________________________
                                                      LCDR3
        T   F   G   Q   G   T   K   V   E   I   K   R
289    ACG TTC GGC CAA GGG ACC AAG GTG GAA ATC AAA CGG
       TGC AAG CCG GTT CCC TGG TTC CAC CTT TAG TTT GCC
       ___
```

Figure 15

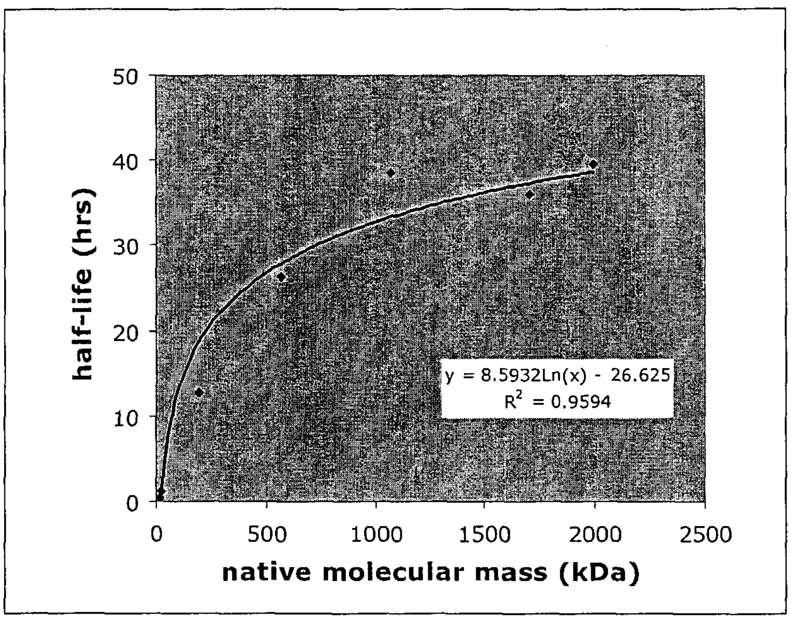
half-life (hrs)
native molecular mass (kDa)
y = 8.5932Ln(x) - 26.625
R2 = 0.9594
0
10
20
30
40
50
0
500
1000
1500
2000
2500

Figure 16

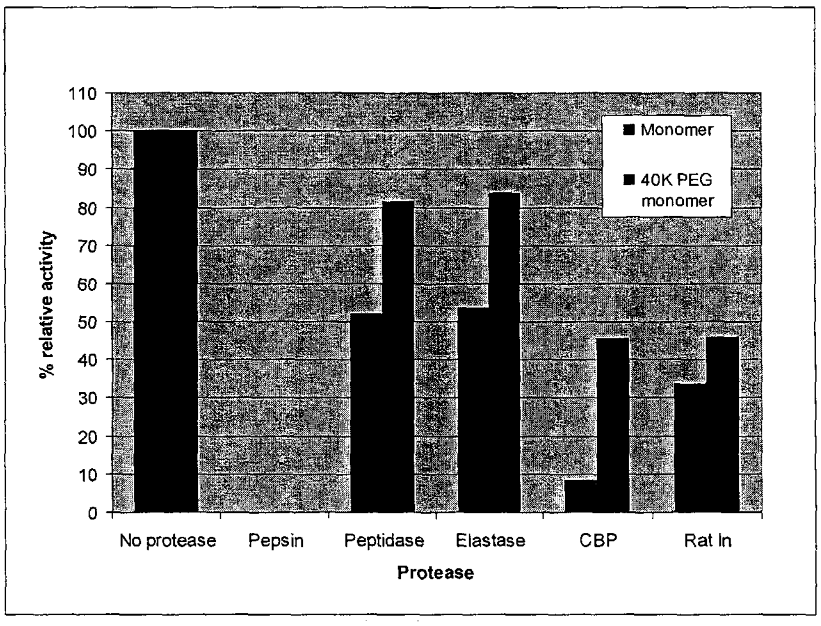
110
100
90
80
70
60
50
40
30
20
10
0
% relative activity
Monomer
40K PEG monomer
No protease
Pepsin
Peptidase
Elastase
CBP
Rat In
Protease

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 03035694 A, Muyldermans
- WO 0029004 A, Plaskin
- WO 9005144 A
- WO 02051870 A, Entwistle
- US 6090382 A, Salfeld
- US 5759808 A
- US 5800988 A
- US 5840526 A
- US 5874541 A
- US 6005079 A
- US 6015695 A
- WO 04041862 A
- WO 960621 A
- WO 9201047 A
- WO 9708320 A
- WO 9920749 A
- WO 03031611 A
- US 4839295 A
- WO 04041867 A
- US 6306406 B
- US 6346274 B
- US 20020182254 A
- US 20020051808 A

### Non-patent literature cited in the description

- **Hamers-Casterman et al.** *Nature,* 1993, vol. 363, 446-448
- **Gahroudi et al.** *FEBS Lett.,* 1997, vol. 414, 521-526
- **Ward et al.** *Nature,* 1989, vol. 341, 544-546
- **Davies ; Riechmann.** *FEBS Lett.,* 1994, vol. 339, 285-290
- **Kabat et al.** Sequences of Immunological Interest. U.S. Dept. Health & Human Services, 1991
- **Davies ; Riechmann.** *Biotechnology N.Y.,* 1995, vol. 13, 475-479
- **Reiter et al.** *J. Mol. Biol.,* 1999, vol. 290, 685-698
- **Tanha et al.** *J. Biol. Chem.,* 2001, vol. 276, 24774-24780
- **Abuchowski et al.** *J. Biol. Chem.,* 1977, vol. 252, 3578
- **Nucci et al.** *Adv. Drug Delivery Reviews,* 1991, vol. 6, 133
- **Francis et al.** Pharmaceutical Biotechnology. vol. 3
- Stability of Protein Pharmaceuticals: in vivo Pathways of Degradation and Strategies for Protein Stabilization. Plenum, 1991, 235-263
- **Zalipsky ; Lee.** Poly(ethylene glycol) Chemistry: Biotechnical and Biomedical Applications. Plenum, 1992, 347-370
- **Goodson ; Katre.** *Bio/Technology,* 1990, vol. 8, 343
- **Hershfield et al.** *PNAS,* 1991, vol. 88, 7185
- **Ling ; Mattiasson.** *Immunol, Methods,* 1983, vol. 59, 327
- **Wilkinson et al.** *Immunol. Letters,* 1987, vol. 15, 17
- **Kitamura et al.** *Cancer Res.,* 1991, vol. 51, 4310
- **Delgado et al.** *Br.J. Cancer,* 1996, vol. 73, 175
- **Pedley et al.** *Br. J. Cancer,* 1994, vol. 70, 1126
- **Ward et al.** *Nature,* 12 October 1989, vol. 341 (6242), 544-6
- **Hentikoff ; Hentikoff.** *Proc. Natl. Acad. Sci. USA,* 1992, vol. 89, 10915-10919
- **Tatusova ; Madden.** *FEMS Microbiol Lett.,* 1999, vol. 174, 247-250
- Current Protocols in Molecular Biology. John Wiley & Sons, 1989, 6.3.1-6.3.6
- **Zalipsky.** *Bioconjug. Chem.,* 1995, vol. 6, 150
- **Greenwald et al.** *Crit. Rev. Ther. Drug Carrier Syst.,* 2000, vol. 17, 101
- **Herman et al.** *Macromol. Chem. Phys.,* 1994, vol. 195, 203
- **Hoogenboom et al.** *Nucleic Acids Res.,* 1991, vol. 19, 4133-4137
- **McCafferty et al.** *Nature,* 1990, vol. 348, 552
- **Kang et al.** *Proc. Natl. Acad. Sci. U.S.A.,* 1991, vol. 88, 4363
- **Clackson et al.** *Nature,* 1991, vol. 352, 624
- **Lowman et al.** *Biochemistry,* 1991, vol. 30, 10832
- **Burton et al.** *Proc. Natl. Acad. Sci U.S.A.,* 1991, vol. 88, 10134
- **Hoogenboom et al.** *Nucleic Acids Res.,* 1991, vol. 19, 4133

- **Chang et al.** *J. Immunol.,* 1991, vol. 147, 3610
- **Breitling et al.** *Gene,* 1991, vol. 104, 147
- **Marks et al.** *J. Mol. Biol.,* 1991, vol. 222, 581
- **Barbas et al.** *Proc. Natl. Acad. Sci. U.S.A.,* 1992, vol. 89, 4457
- **Hawkins ; Winter.** *J. Immunol.,* 1992, vol. 22, 867
- **Marks et al.** *J. Biol. Chem.,* 1992, vol. 267, 16007
- **Lerner et al.** *Science,* 1992, vol. 258, 1313
- **Huston et al.** *Proc. Natl. Acad. Sci U.S.A.,* 1988, vol. 85, 5879-5883
- **Chaudhary et al.** *Proc. Natl. Acad. Sci U.S.A.,* 1990, vol. 87, 1066-1070
- **Vaughan et al.** *Nature Biotech.,* 1996, vol. 14, 309
- **Hoogenboom ; Winter.** *J. Mol. Biol.,* 1992, vol. 227, 381
- **Nissim et al.** *EMBO J.,* 1994, vol. 13, 692
- **Griffiths et al.** *EMBO J.,* 1994, vol. 13, 3245
- **DeKriuf et al.** *J. Mol. Biol.,* 1995, vol. 248, 97
- **Cook ; Tomlinson.** *Immunol Today,* 1995, vol. 16, 237
- **Corbett et al.** *J. Mol. Biol.,* 1997, vol. 268, 69
- **Ravetch et al.** *Cell,* 1981, vol. 27, 583
- **Schäble ; Zachau.** *Biol. Chem. Hoppe-Seyler,* 1993, vol. 374, 1001
- **Williams et al.** *J. Mol. Biol.,* 1996, vol. 264, 220
- **Kawasaki et al.** *Genome Res.,* 1997, vol. 7, 250
- **Hieter et al.** *J. Biol. Chem.,* 1982, vol. 257, 1516
- **Vasicek ; Leder.** *J. Exp. Med.,* 1990, vol. 172, 609
- **Chothia ; Lesk.** *J. Mol. Biol.,* 1987, vol. 196, 901
- **Chothia et al.** *Nature,* 1989, vol. 342, 877
- **Chothia et al.** *J. Mol. Biol.,* 1992, vol. 227, 799
- **Tomlinson et al.** *EMBO J.,* 1995, vol. 14, 4628
- **Martin et al.** *J. Mol. Biol.,* 1996, vol. 263, 800
- **Shirai et al.** *FEBS Letters,* 1996, vol. 399, 1
- **Tomlinson et al.** *J. Mol. Biol.,* 1992, vol. 227, 7768
- **Cox et al.** *Eur. J. Immunol.,* 1994, vol. 24, 827
- **Hawkins et al.** *J. Mol. Biol.,* 1992, vol. 226, 889
- **Sambrook et al.** Molecular Cloning: A Laboratory Manual. Cold Spring Harbor, 1989
- **Hoogenboom et al.** *Nucl. Acids Res.,* 1991, vol. 19, 4133-4137
- **Hood et al.** *Cold Spring Harbor Symp. Quant. Biol.,* 1967, vol. 48, 133
- **Kabat et al.** Sequences of proteins of immunological interest. U.S. Department of Health and Human Services, 1991
- **Deng et al.** *J. Biol. Chem.,* 1994, vol. 269, 9533
- **Low et al.** *J Mol. Biol.,* 1996, vol. 260, 359
- **Barbas et al.** *Proc. Natl. Acad. Sci. USA,* 1992, vol. 89, 4457
- **De Kruif et al.** *J. Mol. Biol.,* 1995, vol. 248, 97
- **Crameri et al.** *Nature Med.,* 1996, vol. 2, 100
- **Riechmann et al.** *Bio/Technology,* 1995, vol. 13, 475
- **Tomlinson et al.** *J. Mol. Biol.,* 1996, vol. 256, 813
- **Kanehisa.** *Nucleic Acids Res.,* 1984, vol. 12, 203
- **Beaucage ; Carruthers.** *Tetrahedron Lett.,* 1981, vol. 22, 1859
- **Matteucci ; Caruthers.** *J. Am. Chem. Soc.,* 1981, vol. 103, 3185
- **Harrison et al.** *Meth. Enzymol.,* 1996, vol. 267, 83-109
- **Marks et al.** *BioTechnology,* 1993, vol. 11, 1145
- **de Kruif et al.** *Proc. Natl. Acad. Sci. U.S.A.,* 1995, vol. 92, 3938
- **Skerra ; Pluckthun.** *Science,* 1988, vol. 240, 1038
- **Better et al.** *Science,* 1988, vol. 240, 1041
- **Bradford.** *Anal. Biochem.,* 1976, vol. 72, 248-254
- **Lowry et al.** *J. Biol. Chem.,* 1951, vol. 193, 265-275
- **Padlan et al.** *Mol. Immunol.,* 1994, vol. 31, 169-217
- **Chothia ; Lesk.** *J. Mol. Biol.,* 1987, vol. 196, 904-917
- **Chothia et al.** *J. Mol. Biol.,* 1985, vol. 186, 651-663
- **Evans, T.** *Molecular Biotechnology,* 2000, vol. 15, 243-248
- **Alceson, L. et al.** *Journal of Biological Chemistry,* 1996, vol. 271, 30517-30523
- Use of functionalized poly(ethylene glycol)s for modification of peptides. **Zalipsky ; Lee.** Poly(Ethylene Glycol Chemistry: Biotechnical and Biomedical Applications. Plenum Press, 1992
- **Kenneth, A et al.** Chemical Stability of Pharmaceuticals: A Handbook for Pharmacists
- **Peters et al.** *Pharmacokinetic analysis: A Practical Approach,* 1996
- **M Gibaldi ; D Perron.** Pharmacokinetics. Marcel Dekker, 1982

- Sustained and Controlled Release Drug Delivery Systems. Marcel Dekker, Inc, 1978
- **Savoie et al.** *Am. J. Respir. Cell Biol.,* 1995, vol. 13, 133-143
- **Suckling et al.** *Lab. Anim.,* 1984, vol. 18, 36-39
- **Feurer et al.** *J. Neuroimmunol.,* 1985, vol. 10, 159-166
- **Brenner et al.** *Isr. J. Med. Sci.,* 1985, vol. 21, 945-949
- **Zamvil et al.** *Nature,* 1985, vol. 317, 355-358
- **Li et al.** *Proc. Natl. Acad. Sci. U.S.A.,* 1994, vol. 91, 11128-11132
- **Okwueze et al.** *Am. J. Physiol.,* 1994, vol. 266, R572-R577
- **Jiao et al.** *Int. J. Obesity,* 1991, vol. 15, 487-495
- **Lee et al.** *Proc. Natl. Acad. Sci. U.S.A.,* 1994, vol. 91, 10878-10882
- **Loweth et al.** *Vet. Pathol.,* 1990, vol. 27, 347-353
- **Chao et al.** *J. Lipid Res.,* 1994, vol. 35, 71-83
- **Yoshida et al.** *Lab. Anim. Sci.,* 1990, vol. 40, 486-489
- **Hara et al.** *Jpn. J. Exp. Med.,* 1990, vol. 60, 315-318
- **Ogura et al.** *Lab. Anim.,* 1989, vol. 23, 169-174
- **Dietrich et al.** *Lab. Anim.,* 1989, vol. 23, 345-352
- **Wu et al.** *Proc. Natl. Acad. Sci. U.S.A.,* 1994, vol. 91, 742-746
- **Engstrand et al.** *Infect. Immunity,* 1990, vol. 58, 1763-1768
- **Hyun et al.** *Lab. Anim. Sci.,* 1991, vol. 41, 442-446
- **Ermel et al.** *Lab. Anim. Sci.,* 1997, vol. 47, 40-49
- **Knippels et al.** *Clin. Exp. Allergy,* 1998, vol. 28, 368-375
- **Adel-Patient et al.** *J. Immunol. Meth.,* 2000, vol. 235, 21-32
- **Kitagawa et al.** *Am. J. Med. Sci.,* 1995, vol. 310, 183-187
- **Panush et al.** *J. Rheumatol.,* 1990, vol. 17, 285-290
- **Mauri et al.** *J. Immunol.,* 1997, vol. 159, 5032-5041
- **Saegusa et al.** *J. Vet. Med. Sci.,* 1997, vol. 59, 897-903
- **Takeshita et al.** *Exp. Anim.,* 1997, vol. 46, 165-169
- **Rothschild et al.** *Clin. Exp. Rheumatol.,* 1997, vol. 15, 45-51
- **Matyas et al.** *Arthritis Rheum.,* 1995, vol. 38, 420-425
- **Walker et al.** *Vet. Immunol. Immunopathol.,* 1983, vol. 15, 97-104
- **Walker et al.** *J. Lab. Clin. Med.,* 1978, vol. 92, 932-943
- **Dieleman et al.** *Scand. J. Gastroenterol.,* 1997, vol. 223, 99-104
- **Anthony et al.** *Int. J. Exp. Pathol.,* 1995, vol. 76, 215-224
- **Osborne et al.** *Br. J. Surg.,* 1993, vol. 80, 226-229
- **Keffer, J. ; Probert, L. ; Cazlaris, H. ; Georgopoulos, S. ; Kaslaris, E. ; Kioussis, D. ; Kollias, G.** Transgenic mice expressing human tumor necrosis factor: a predictive genetic model of arthritis. *EMBO J.,* 1991, vol. 10, 4025-4031